# EUROPÄISCHE PATENTSCHRIFT

(11) **EP 2 976 322 B1**
(45) Veröffentlichungstag und Bekanntmachung des Hinweises auf die Patenterteilung: **20.11.2019**
(21) Anmeldenummer: 14706786.2
(22) Anmeldetag: 24.02.2014
(51) Int. Cl.: C07C 255/51

(54) **SYNTHESEBAUSTEINE FÜR DIE HERSTELLUNG VON MATERIALIEN FÜR ORGANISCHE ELEKTROLUMINESZENZVORRICHTUNGEN**
SYNTHETIC BUILDING BLOCKS FOR THE PRODUCTION OF MATERIALS FOR ORGANIC ELECTROLUMINESCENCE DEVICES
BLOCS DE SYNTHÈSE POUR LA PRODUCTION DE MATÉRIAUX DESTINÉS À DES DISPOSITIFS ÉLECTROLUMINESCENTS ORGANIQUES

(30) Priorität: 22.03.2013 EP 13001485
(43) Veröffentlichungstag der Anmeldung: 27.01.2016
(73) Patentinhaber: Merck Patent GmbH, 64293 Darmstadt (DE)
(72) Erfinder: STOESSEL, Philipp, 60389 Frankfurt am Main (DE); PARHAM, Amir Hossain, 60486 Frankfurt am Main (DE); JATSCH, Anja, 60489 Frankfurt am Main (DE)
(86) Internationale Anmeldenummer: PCT/EP2014/000481
(87) Internationale Veröffentlichungsnummer: WO 2014/146750

(56) Entgegenhaltungen:
- JP-A- 62 240 655
- US-A1- 2010 152 236
- US-A1- 2012 114 974
- HUANG CHAO ET AL: "Synthesis and antimicrobial activity of polyhalo isophthalonitrile derivatives", BIOORGANIC & MEDICINAL CHEMISTRY LETTERS, PERGAMON, AMSTERDAM, NL, Bd. 23, Nr. 8, 16. Februar 2013 (2013-02-16), Seiten 2399-2403, XP028525195, ISSN: 0960-894X, DOI: 10.1016/J.BMCL.2013.02.033
- WALLENFELS K ET AL: "2,4-Dicyan- und 2,4,6-tricyan-fluorbenzol", TETRAHEDRON, ELSEVIER SCIENCE PUBLISHERS, AMSTERDAM, NL, Bd. 23, Nr. 3, 1. Januar 1967 (1967-01-01) , Seiten 1353-1358, XP028087039, ISSN: 0040-4020, DOI: 10.1016/0040-4020(67)85088-9 [gefunden am 1967-01-01]
- RAHUL S NANDURDIKAR ET AL: "Structural modifications modulate stability of glutathione-activated arylated diazeniumdiolate prodrugs", BIOORGANIC & MEDICINAL CHEMISTRY, PERGAMON, GB, Bd. 20, Nr. 9, 20. Februar 2012 (2012-02-20), Seiten 3094-3099, XP028412838, ISSN: 0968-0896, DOI: 10.1016/J.BMC.2012.02.045 [gefunden am 2012-03-09]
- RAYMOND J. PATCH ET AL: "Identification of Diaryl Ether-Based Ligands for Estrogen-Related Receptor [alpha] as Potential Antidiabetic Agents", JOURNAL OF MEDICINAL CHEMISTRY, Bd. 54, Nr. 3, 10. Februar 2011 (2011-02-10), Seiten 788-808, XP055125939, ISSN: 0022-2623, DOI: 10.1021/jm101063h

## Beschreibung

Die vorliegende Erfindung betrifft Verbindungen, die als Synthesebausteine für die Herstellung elektronisch aktiver Materialien, insbesondere für organische Elektrolumineszenzvorrichtungen, Anwendung finden können.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Hierfür werden derzeit insbesondere auch Donor-Akzeptor-substituierte Benzolderivate eingesetzt, insbesondere solche, in denen die Akzeptorgruppen Cyanogruppen sind, die sich in meta-Stellung zueinander befinden, und die Donorgruppen Carbazolderivate. Beispiele für solche Verbindungen sind in C. Adachi et al., Nature 2012, 492, 234-238 beschrieben. Allerdings ist hier nur die Synthese symmetrisch substituierter Verbindungen offenbart. Weiterhin war die chromatographische Reinigung der Produkte erforderlich, was insbesondere die Aufreinigung im technischen Maßstab erschwert. Außerdem sind Dicvanofluorbenzol-Derivate mit Thiophenylgruppen in Chao et al., Bioorganic & Medicinal Chemistry Letters 2013, 23(8), 2399 offenbart. In US 2012/114974, JP 62-240655, US 2010/0152236 sowie in Wallenfels et al., Tetrahedron 1967, 23, 1353, in Nandurdikar et al., Bioorganic & Medicinal Chemistry, Pergamon, 2012, 20(9), 3094 und in Patch et al., Journal of Medicinal Chemistry, 2011, 54(3), 788 werden auch Cyanofluorbenzol- Derivate offenbart. Es ist wünschenswert, Verbindungen zur Verfügung zu haben, aus denen sich die gewünschten, elektronisch aktiven Verbindungen in hoher Ausbeute und Reinheit synthetisieren lassen.

Aufgabe der vorliegenden Erfindung ist daher die Bereitstellung von Verbindungen, welche sich als Synthesevorstufen für die Herstellung elektronisch aktiver Komponenten für die Verwendung in organischen Elektrolumineszenzvorrichtungen eignen, um so einerseits die Materialien in guter Ausbeute und Reinheit herstellen zu können und andererseits dem Fachmann eine größere Wahlmöglichkeit an Materialien für die Herstellung von OLEDs zu ermöglichen.

Überraschend wurde gefunden, dass bestimmte, unten näher beschriebene Verbindungen diese Aufgabe lösen, sich sehr gut für die Herstellung Donor-Akzeptor-substituierter Materialien für die Verwendung in OLEDs eignen. In diesen Verbindungen lässt sich der Fluorsubstituent selektiv und in hoher Ausbeute unter milden Reaktionsbedingungen in einer nukleophilen aromatischen Substitution durch Nukleophile ersetzen. Diese Verbindungen sind daher der Gegenstand der vorliegenden Erfindung.

Gegenstand der vorliegenden Erfindung ist eine Verbindung gemäß Formel (1), Formel (2) oder Formel (3), 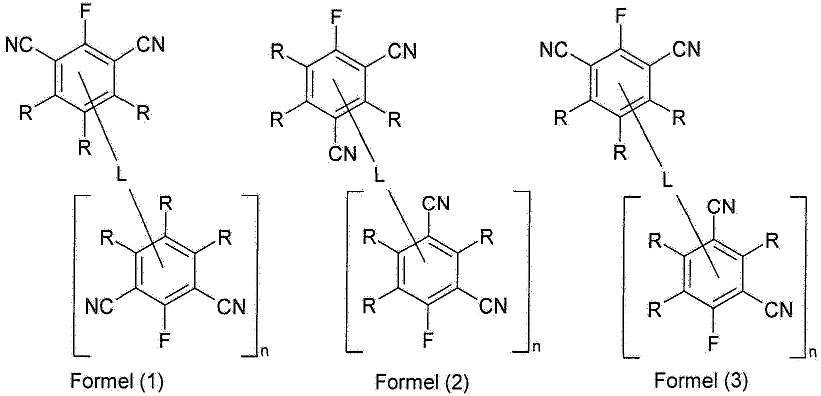 wobei für die verwendeten Symbole und Indizes gilt:
- L: ist eine Einfachbindung oder eine bi-, tri-, tetra-, penta- oder hexavalente Gruppe;
- R: ist bei jedem Auftreten gleich oder verschieden ausgewählt aus der Gruppe bestehend aus H, D, Cl, Br, I, NAr₂, N(R¹)₂, wobei R¹ ungleich H ist, C(=O)Ar, C(=O)R¹, BR¹, P(=O)Ar₂, PAr₂, OAr, S(=O)Ar, S(=O)₂Ar, Si(R¹)₃, einer geradkettigen Alkyl- oder Alkoxygruppe mit 1 bis 40 C-Atomen oder einer verzweigten oder cyclischen Alkyl- oder Alkoxygruppe mit 3 bis 40 C-Atomen oder einer Alkenyl- oder Alkinyl-gruppe mit 2 bis 40 C-Atomen, die jeweils mit einem oder mehreren Resten R¹ substituiert sein kann, wobei jeweils eine oder mehrere nicht-benachbarte CH₂-Gruppen durch R¹C=CR¹, C≡C, Si(R¹)₂, C=NR¹, P(=O)(R¹), NR¹, O, S oder CONR¹ ersetzt sein können und wobei ein oder mehrere H-Atome durch D, F, Cl, Br, I oder CN ersetzt sein können, oder einem aromatischen oder heteroaromatischen Ring-system mit 5 bis 60 aromatischen Ringatomen, das jeweils mit einem oder mehreren Resten R¹ substituiert sein kann; dabei können optional zwei oder mehr benachbarte Substituenten R ein monocyclisches oder polycyclisches, aliphatisches, aromatisches oder heteroaromatisches Ringsystem bilden, das mit einem oder mehreren Resten R¹ substituiert sein kann;
- Ar: ist bei jedem Auftreten gleich oder verschieden ein aromatisches oder heteroaromatisches Ringsystem mit 5-30 aromatischen Ringatomen, das mit einem oder mehreren Resten R¹ substituiert sein kann; dabei können zwei Reste Ar, welche an dasselbe N-Atom oder P-Atom binden, auch durch eine Einfachbindung oder eine Brücke, ausgewählt aus N(R¹), C(R¹)₂, O, S oder BR¹, miteinander verbrückt sein;
- R¹: ist bei jedem Auftreten gleich oder verschieden ausgewählt aus der Gruppe bestehend aus H, D, F, Cl, Br, I, CN, NO₂, N(R²)₂, P(=O)(R²)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C≡C, Si(R²)₂, C=NR², P(=O)(R²), SO, SO₂, NR², O, S oder CONR² ersetzt sein können und wobei ein oder mehrere H-Atome durch D, F, Cl, Br, I oder CN ersetzt sein können, einem aromatischen oder heteroaromatischen Ringsystem mit 5 bis 60 aromatischen Ringatomen, das jeweils mit einem oder mehreren Resten R² substituiert sein kann; dabei können optional zwei oder mehr benachbarte Substituenten R¹ ein monocyclisches oder polycyclisches, aliphatisches Ringsystem bilden, das mit einem oder mehreren Resten R² substituiert sein kann;
- R²: ist bei jedem Auftreten gleich oder verschieden ausgewählt aus der Gruppe bestehend aus H, D, F, CN, einem aliphatischen Kohlenwasserstoffrest mit 1 bis 20 C-Atomen, einem aromatischen oder heteroaromatischen Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R² miteinander ein mono- oder polycyclisches, aliphatisches Ringsystem bilden können;
- n: ist 1, 2, 3, 4 oder 5, mit der Maßgabe, dass L jeweils anstelle eines Restes R an den Benzolgrundkörper bindet und die entsprechende Gruppe R somit nicht vorhanden ist.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

In einer Ausführungsform der Erfindung sind Verbindungen der Formel (1), (2) und (3) von der Erfindung ausgenommen, in denen R gleich oder verschieden für Carbazol oder für ein substituiertes Carbazol, das jeweils über das Stickstoffatom an das Grundgerüst gebunden ist, steht.

In einer bevorzugten Ausführungsform der Erfindung ist n = 1, 2 oder 3, besonders bevorzugt 1 oder 2, ganz besonders bevorzugt 1. Dabei ist für n = 1 L eine Einfachbindung oder eine bivalente Gruppe, für n = 2 eine trivalente Gruppe, etc..

In einer weiteren bevorzugten Ausführungsform der Erfindung steht L für eine Einfachbindung, NR, BR, P(=O)R, eine geradkettige Alkylen- oder Alkylidengruppe mit 1 bis 10 C-Atomen oder eine verzweigte oder cyclische Alkylen- oder Alkylidengruppe mit 3 bis 10 C-Atomen, die mit jeweils einem oder mehreren Resten R substituiert sein kann, wobei eine oder mehrere nicht benachbarte CH₂-Gruppen durch -RC=CR-, -C≡C-, Si(R)₂, C=O, -O-, -S- oder -CONR- ersetzt sein können und wobei ein oder mehrere H-Atome durch D oder F ersetzt sein können, oder ein aromatisches oder heteroaromatisches Ringsystem mit 5 bis 24 aromatischen Ringatomen, welches mit einem oder mehreren Resten R substituiert sein kann. Besonders bevorzugt steht L für eine Einfachbindung, NR, eine geradkettige Alkylen- oder Alkylidengruppe mit 1 bis 6 C-Atomen oder eine verzweigte oder cyclische Alkylen- oder Alkylidengruppe mit 3 bis 6 C-Atomen, wobei eine oder mehrere nicht benachbarte CH₂-Gruppen durch -O- ersetzt sein können, oder ein aromatisches oder heteroaromatisches Ringsystem mit 5 bis 18 aromatischen Ringatomen, welches mit einem oder mehreren Resten R substituiert sein kann. Dabei ist, wie oben beschrieben, die Gruppe L für n = 0 nicht vorhanden, und für n ≥ 1 bindet die Gruppe L jeweils statt einem der Reste R an den Benzolgrundkörper. Für n = 2 können bevorzugte Gruppen L weiterhin auch gewählt sein aus N, B oder P=O.

Bevorzugte Ausführungsformen der Verbindungen der Formel (1) sind die Verbindungen der folgenden Formeln (1f) bis (1h). Bevorzugte Ausführungsformen der Verbindungen der Formel (2) sind die Verbindungen der folgenden Formeln (2h) bis (2l). Bevorzugte Ausführungsformen der Verbindungen der Formel (3) sind die Verbindungen der folgenden Formeln (3a) bis (3f). 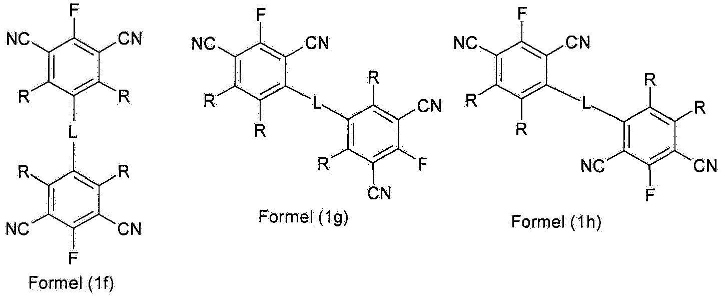 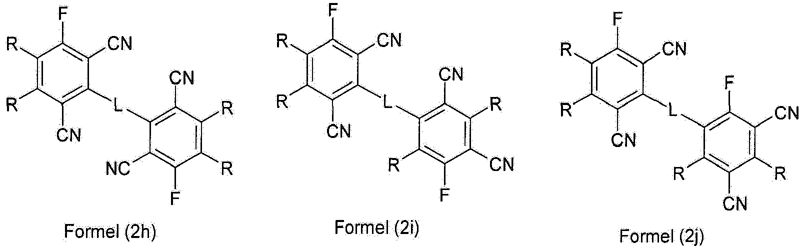 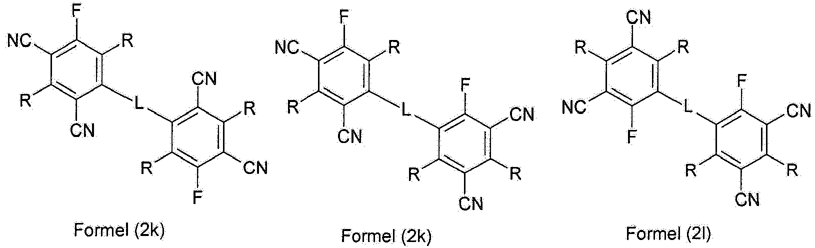 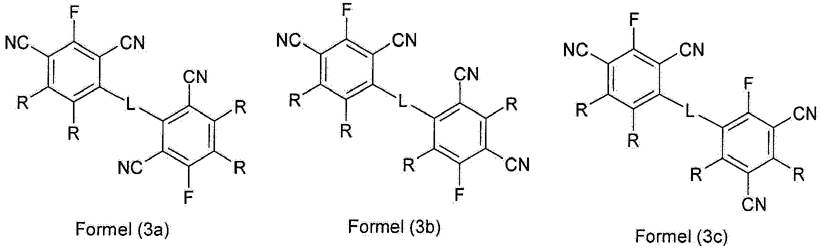 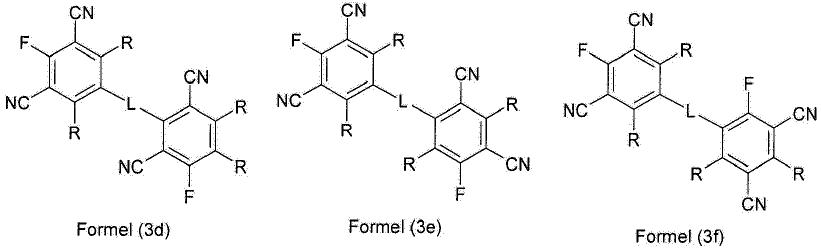 wobei R und L die oben genannten Bedeutungen aufweisen.

Wie oben erwähnt, können mehrere benachbarte Reste R auch miteinander einen Ring bilden, so dass sich beispielsweise durch Bildung einer ankondensierten Benzogruppe insgesamt ein Naphthalin bildet. Bevorzugt ist auch die Bildung eines ankondensierten aliphatischen Rings, wobei der Cyclus bevorzugt insgesamt 5 bis 7 Ringatome aufweist. Bevorzugte ankondensierte Ringe, die sich durch Ringbildung benachbarter Reste R bilden, sind weiterhin die Strukturen der folgenden Formel (4), 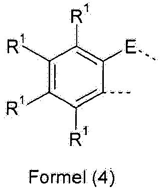 wobei R¹ die oben genannten Bedeutungen aufweist, die gestrichelten Bindungen die Verknüpfung der Gruppe mit dem Benzolgrundkörper andeuten und weiterhin gilt:
- E: ist ausgewählt aus der Gruppe bestehend aus C(R¹)₂, NR¹, O, S, BR¹ oder Si(R¹)₂, bevorzugt NR¹, O oder S.

Bevorzugte Ausführungsformen der Formel (1), die eine Gruppe der Formel (4) aufweisen, sind die Verbindungen der folgenden Formeln (5a) und (5b), und bevorzugte Ausführungsformen der Formel (2), die eine Gruppe der Formel (4) aufweisen, sind die Verbindungen der folgenden Formeln (6a) und (6b), 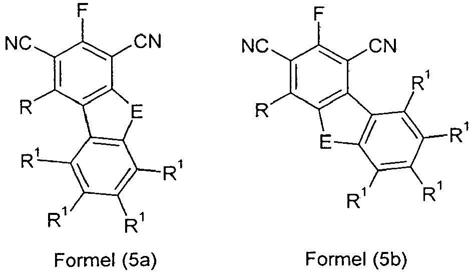 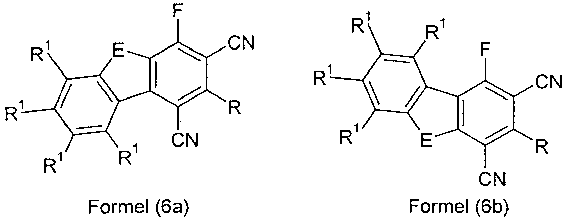 wobei die verwendeten Symbole die oben genannten Bedeutungen aufweisen.

Dabei sind jeweils nur Strukturen mit n = 0 abgebildet. Ganz entsprechend können auch Strukturen mit n ≥ 1 Einheiten gemäß Formel (4) enthalten.

Bevorzugte Reste R sind bei jedem Auftreten gleich oder verschieden ausgewählt aus der Gruppe bestehend aus H, NAr₂, C(=O)Ar, P(=O)Ar₂, PAr₂, Si(R¹)₃, einer geradkettigen Alkylgruppe mit 1 bis 10 C-Atomen oder einer verzweigten oder cyclischen Alkylgruppe mit 3 bis 10 C-Atomen oder einer Alkenylgruppe mit 2 bis 10 C-Atomen, die jeweils mit einem oder mehreren Resten R¹ substituiert sein kann, wobei jeweils eine oder mehrere nicht-benachbarte CH₂-Gruppen durch R¹C=CR¹ oder O ersetzt sein können und wobei ein oder mehrere H-Atome durch F ersetzt sein können, einem aromatischen oder heteroaromatischen Ringsystem mit 5 bis 40 aromatischen Ringatomen, das jeweils mit einem oder mehreren Resten R¹ substituiert sein kann; dabei können optional zwei oder mehr benachbarte Substituenten R ein monocyclisches oder polycyclisches, aliphatisches, aromatisches oder heteroaromatisches Ringsystem bilden, das mit einem oder mehreren Resten R¹ substituiert sein kann.

Besonders bevorzugte Reste R sind bei jedem Auftreten gleich oder verschieden ausgewählt aus der Gruppe bestehend aus H, NAr₂, einer geradkettigen Alkylgruppe mit 1 bis 6 C-Atomen oder einer verzweigten oder cyclischen Alkylgruppe mit 3 bis 8 C-Atomen, die jeweils mit einem oder mehreren Resten R¹ substituiert sein kann, einem aromatischen oder heteroaromatischen Ringsystem mit 5 bis 24 aromatischen Ringatomen, das jeweils mit einem oder mehreren Resten R¹ substituiert sein kann; dabei können optional zwei oder mehr benachbarte Substituenten R ein monocyclisches oder polycyclisches, aliphatisches, aromatisches oder heteroaromatisches Ringsystem bilden, das mit einem oder mehreren Resten R¹ substituiert sein kann.

Bevorzugte Gruppen Ar sind ausgewählt aus aromatischen oder heteroaromatischen Ringsystemen mit 5 bis 24 aromatischen Ringatomen, besonders bevorzugt mit 5 bis 18 aromatischen Ringatomen, welche jeweils durch einen oder mehrere Reste R¹ substituiert sein können. Dabei enthält Ar bevorzugt keine kondensierten Aryl- bzw. Heteroarylgruppen, in denen mehr als zwei Sechsringe direkt aneinander ankondensiert sind. Insbesondere ist es bevorzugt, wenn Ar keine kondensierte Aryl- bzw. Heteroarylgruppe enthalten, in der zwei oder mehr Sechsringe direkt aneinander ankondensiert sind.

Wenn R für eine Alkylgruppe steht, so ist es bevorzugt, wenn diese Alkylgruppe keine benzylischen Protonen aufweist, wenn also kein Wasserstoffatom an das Kohlenstoffatom gebunden ist, welches direkt an den Benzolring bindet. Dies wird beispielsweise durch die Verwendung von tertiären Alkylgruppen erreicht, wie z. B. tert-Butyl.

Wenn R für eine Gruppe Si(R¹)₃ steht, steht R¹ in dieser Gruppe bevorzugt gleich oder verschieden bei jedem Auftreten für eine Alkylgruppe mit 1 bis 10 C-Atomen, besonders bevorzugt mit 1 bis 4 C-Atomen.

In einer bevorzugten Ausführungsform der Erfindung ist mindestens einer der Reste R ein aromatisches oder heteroaromatisches Ringsystem oder eine Diarylaminogruppe NAr₂.

Wenn R für ein aromatisches oder heteroaromatisches Ringsystem steht, weist dieses, wie oben beschrieben, bevorzugt 5 bis 24 aromatische Ringatome auf und kann durch einen oder mehrere Reste R¹ substituiert sein. Bevorzugte aromatische bzw. heteroaromatische Gruppen R sind ausgewählt aus Benzol, Biphenyl, insbesondere ortho-, meta- oder para-Biphenyl, Terphenyl, insbesondere ortho-, meta-, para- oder verzweigtes Terphenyl, Quaterphenyl, insbesondere ortho-, meta- para- oder verzweigtes Quaterphenyl, 1-, 2-, 3- oder 4-Spirobifluoren, 1-, 2-, 3- oder 4-Fluoren, 1- oder 2-Naphthyl, Pyrrol, Furan, Thiophen, Indol, Benzofuran, Benzothiophen, Carbazol, Azacarbazol, Dibenzofuran, Dibenzothiophen, Pyridin, Pyrimidin, Pyrazin, Pyridazin, Triazin, Imidazol, Benzimidazol, Pyrazol, Thiazol, Oxazol, Oxadiazol, Triazol, Phenanthren, Triphenylen oder Kombinationen aus zwei oder drei dieser Gruppen, welche jeweils mit einem oder mehreren Resten R¹ substituiert sein können.

Wenn es sich bei dem Rest R um ein aromatisches bzw. heteroaromatisches Ringsystem handelt, so enthält die Verbindung bevorzugt keine kondensierten Aryl- bzw. Heteroarylgruppen, in denen mehr als zwei Sechsringe direkt aneinander ankondensiert sind. Insbesondere ist es bevorzugt, wenn R keine kondensierte Aryl- bzw. Heteroarylgruppe enthalten, in der zwei oder mehr Sechsringe direkt aneinander ankondensiert sind. Besonders bevorzugt enhält die Verbindung der Formel (1), Formel (2), Formel (3) bzw. die bevorzugten Ausführungsformen in keinem der Reste R, R¹ oder R² kondensierte Aryl- oder Heteroarylgruppen, in denen Sechsringe direkt aneinander ankondensiert sind.

Geeignete und bevorzugte aromatische Ringsysteme R sind die Gruppen der folgenden Formeln (7) bis (14), 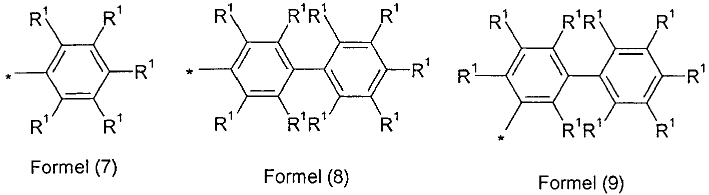 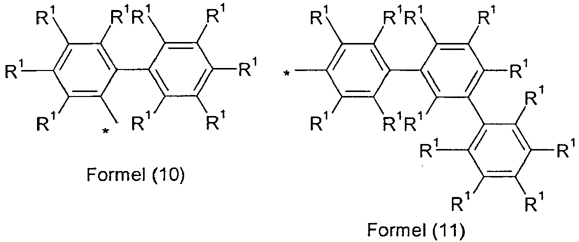 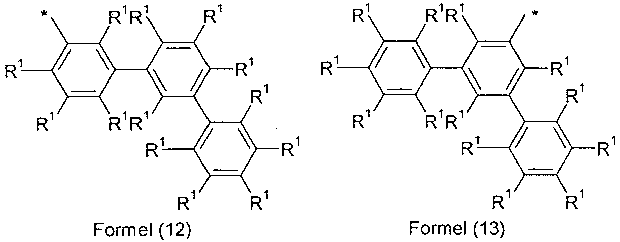 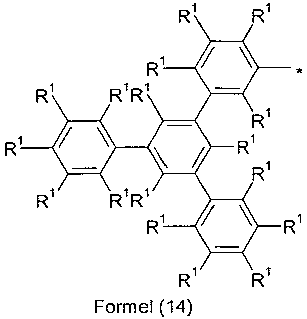 wobei die verwendeten Symbole die oben genannten Bedeutungen aufweisen und * die Position der Bindung der Gruppe gemäß Formel (7) bis (14) andeutet.

Geeignete elektronenarme Gruppen R sind die Gruppen der folgenden Formeln (15) bis (18), 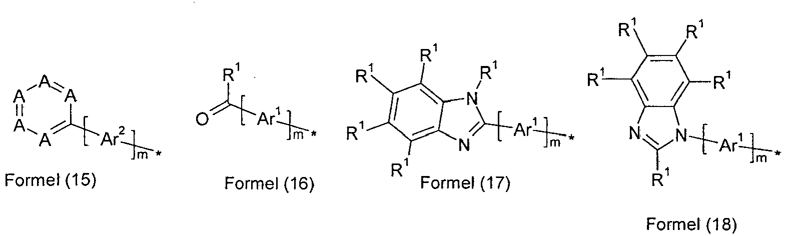 wobei R¹ die oben genannte Bedeutung hat, * die Position der Bindung der Gruppe gemäß Formel (15) bis (18) andeutet und weiterhin gilt:
- A: ist bei jedem Auftreten gleich oder verschieden CR¹ oder N, mit der Maßgabe, dass eine, zwei oder drei Gruppen A für N stehen;
- Ar¹: ist gleich oder verschieden bei jedem Auftreten ein bivalentes aromatisches oder heteroaromatisches Ringsystem mit 5 bis 16 aromatischen Ringatomen, welches durch einen oder mehrere Reste R¹ substituiert sein kann;
- m: ist 0 oder 1.

Bevorzugte Gruppen der Formel (15) sind die Gruppen der folgenden Formeln (15a) bis (15g), 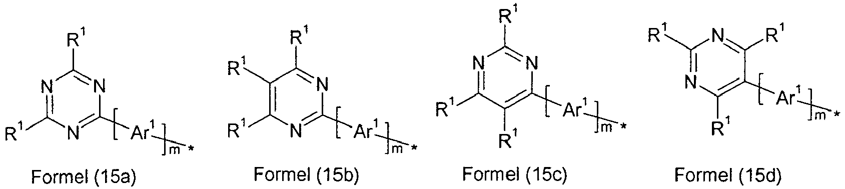 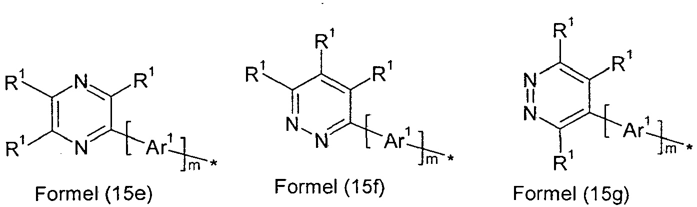 wobei die verwendeten Symbole und Indizes die oben genannten Bedeutungen aufweisen.

Der Rest R¹ in Formel (15a) steht bevorzugt gleich oder verschieden bei jedem Auftreten für ein aromatisches oder heteroaromatisches Ringsystem mit 5 bis 24 aromatischen Ringatomen, welches durch einen oder mehrere Reste R² substituiert sein kann, insbesondere für Phenyl, ortho-, meta- oder para-Biphenyl, ortho-, meta-, para- oder verzweigtes Terphenyl oder ortho-, meta-, para- oder verzweigtes Quaterphenyl. Diese haben bevorzugt dieselben Strukturen, wie oben in den Formeln (7) bis (14) gezeigt, wobei statt Resten R¹ Reste R² gebunden sind.

Der Rest R¹ in den Formeln (15b) bis (15g) steht bevorzugt gleich oder verschieden bei jedem Auftreten für H, D oder ein aromatisches oder heteroaromatisches Ringsystem mit 5 bis 24 aromatischen Ringatomen, welches durch einen oder mehrere Reste R² substituiert sein kann, insbesondere für H oder Phenyl, ortho-, meta- oder para-Biphenyl, ortho-, meta-, para- oder verzweigtes Terphenyl oder ortho-, meta-, para- oder verzweigtes Quaterphenyl. Diese haben bevorzugt dieselben Strukturen, wie oben in den Formeln (7) bis (14) gezeigt, wobei statt Resten R¹ Reste R² gebunden sind.

Weiterhin bevorzugt ist mindestens ein Rest R in Verbindungen der Formel (1), (2) bzw. (3) ausgewählt aus der Gruppe bestehend aus Triarylaminderivaten, Carbazolderivaten, Indenocarbazolderivaten, Indolocarbazolderivaten, Azacarbazolderivaten, Indolderivaten, Furanderivaten, Benzofuranderivaten, Dibenzofuranderivaten, Thiophenderivaten, Benzothiophenderivaten oder Dibenzothiophenderivaten, welche jeweils durch einen oder mehrere Reste R¹ substituiert sein können, oder mindestens ein Substituent R steht für -NAr₂, wobei die beiden Gruppen Ar auch durch eine Gruppe ausgewählt aus NR¹, O, S, C(R¹)₂, Si(R¹)₂ oder BR¹ miteinander verbrückt sein können. Diese Gruppen sind bevorzugt ausgewählt aus den Gruppen der folgenden Formeln (19) bis (33), 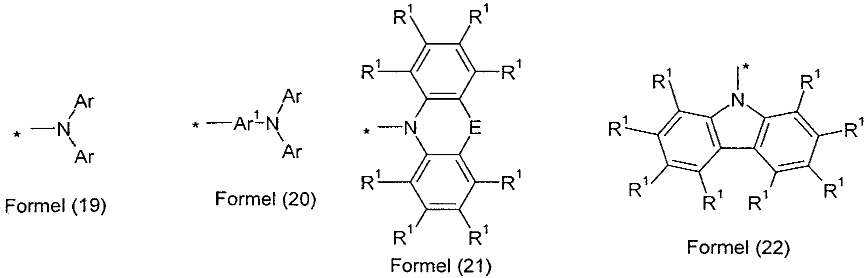 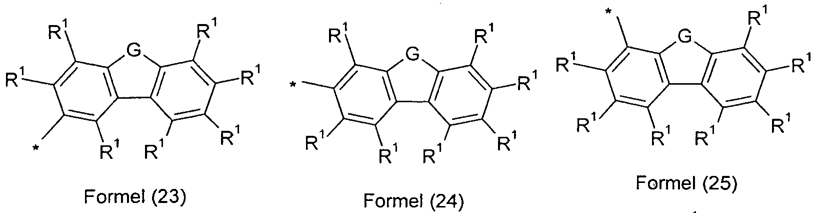 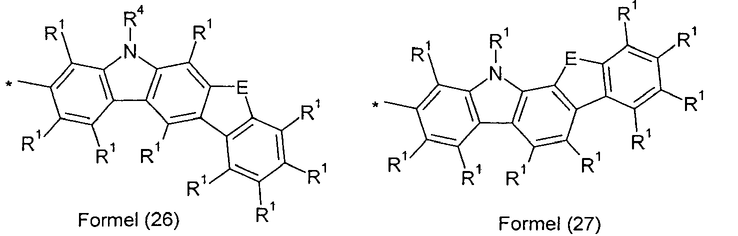 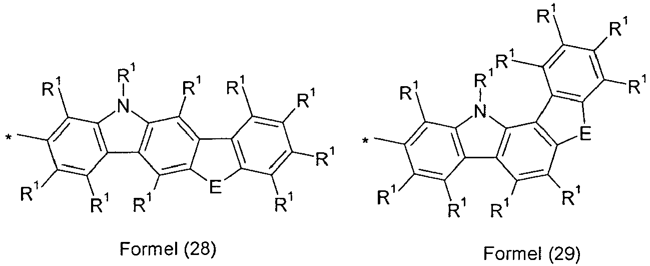 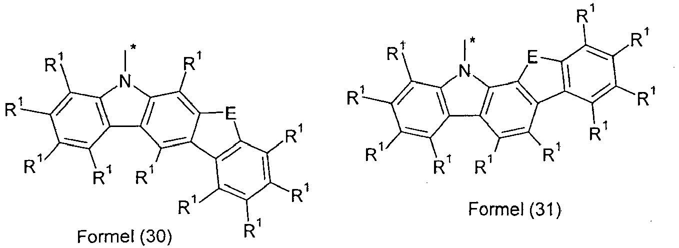 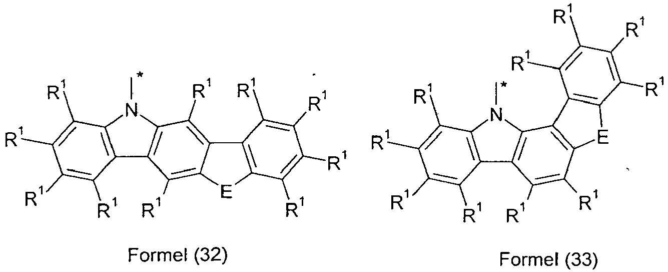 wobei die verwendeten Symbole die oben genannten Bedeutungen aufweisen und weiterhin gilt:
- G: ist ausgewählt aus der Gruppe bestehend aus NR¹, O oder S.

Weiterhin bevorzugt können in den oben genannten Strukturen eine oder zwei Gruppen CR¹ durch N ersetzt sein.

Bevorzugte Substituenten R¹ an den oben genannten Strukturen sind H, CN, Alkylgruppen mit 1 bis 10 C-Atomen, die mit einem oder mehreren Resten R² substituiert sind, oder aromatische oder heteroaromatische Ringsysteme mit 5 bis 24 aromatischen Ringatomen, die mit einem oder mehreren Resten R² substituiert sind.

Wenn E für BR¹ steht, ist R¹ bevorzugt eine Aryl- oder Heteroarylgruppe mit 5 bis 10 aromatischen Ringatomen, die mit einem oder mehreren Resten R² substituiert sein kann. Besonders bevorzugt ist an der Aryl- bzw Heteroarylgruppe in beiden Positionen ortho zur Verknüpfung mit dem Boratom ein Substituent R² ungleich H gebunden, beispielsweise eine Alkylgruppe, CN oder eine Arylgruppe.

Wenn E für C(R¹)₂ oder Si(R¹)₂ steht, ist R¹ bevorzugt gleich oder verschieden bei jedem Auftreten eine Alkylgruppe, eine Arylgruppe oder eine Heteroarylgruppe, die jeweils mit einem oder mehreren Resten R² substituiert sein kann; dabei können die beiden Gruppen R¹ auch miteinander ein Ringsystem bilden.

Wenn E für NR¹ steht, ist R¹ bevorzugt gleich oder verschieden bei jedem Auftreten eine Alkylgruppe, eine Arylgruppe oder eine Heteroarylgruppe, insbesondere eine Arylgruppe, die jeweils mit einem oder mehreren Resten R² substituiert sein können; dabei können die beiden Gruppen R¹ auch miteinander ein Ringsystem bilden.

Die oben genannten bevorzugten Ausführungsformen können beliebig miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die Verbindungen der folgenden Strukturen 40-63, 84-87, 235-246.

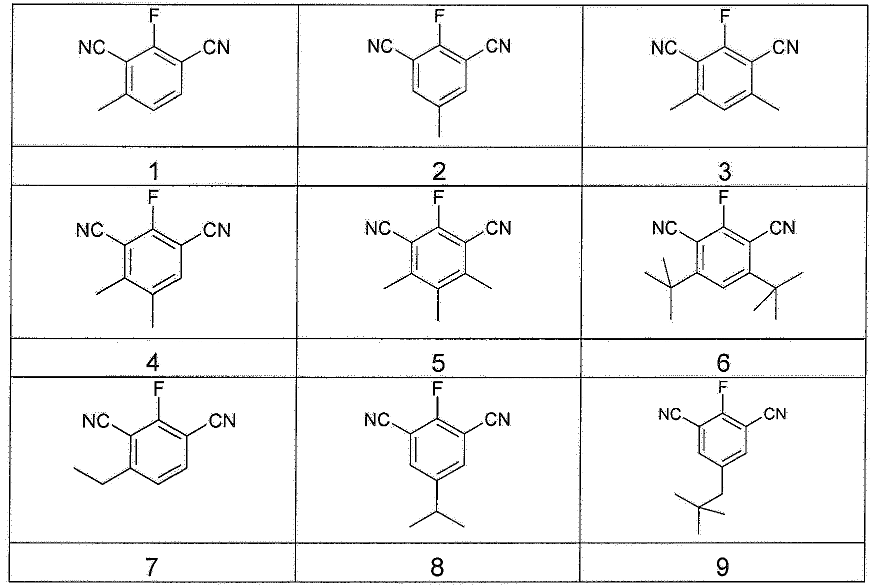

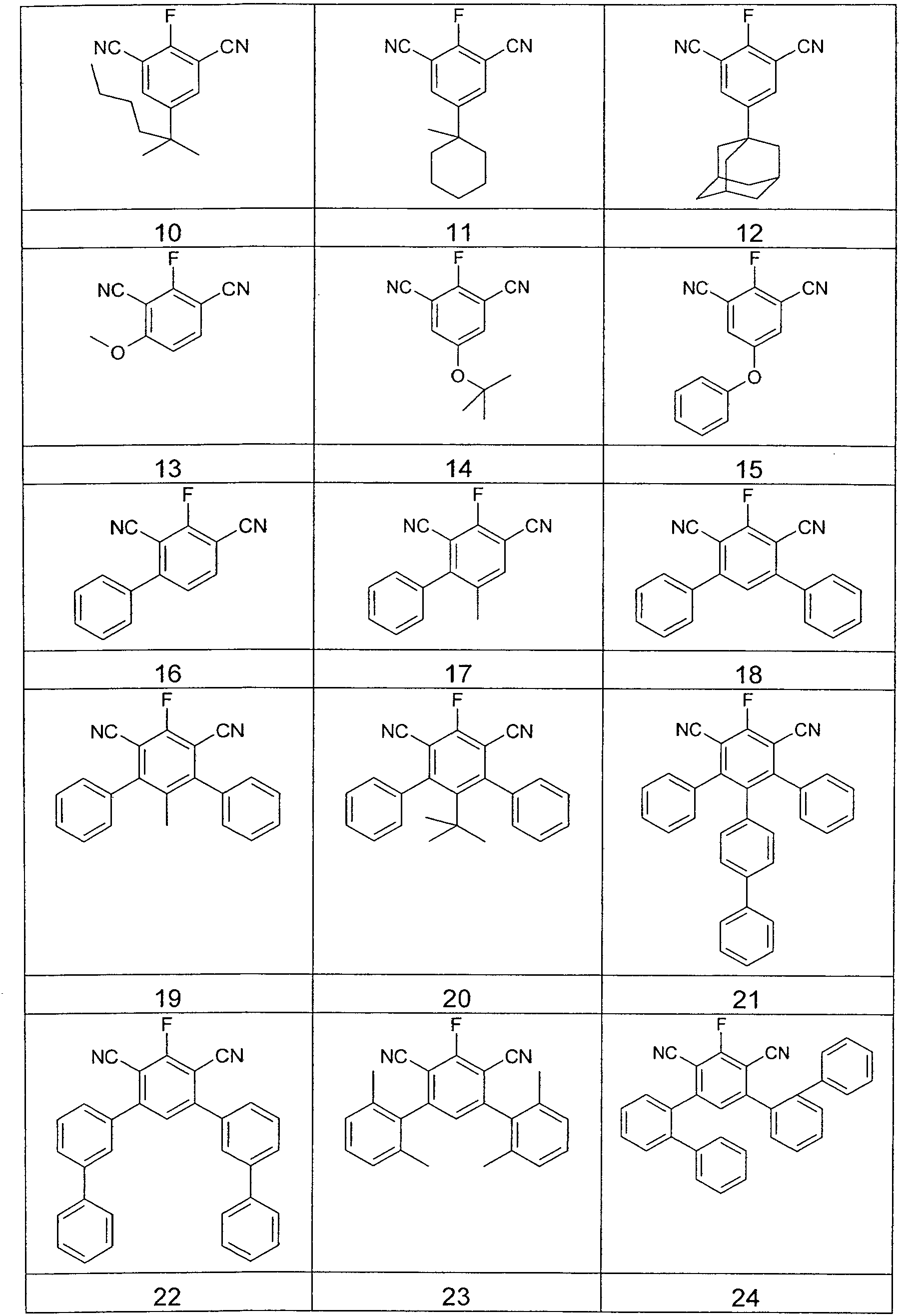

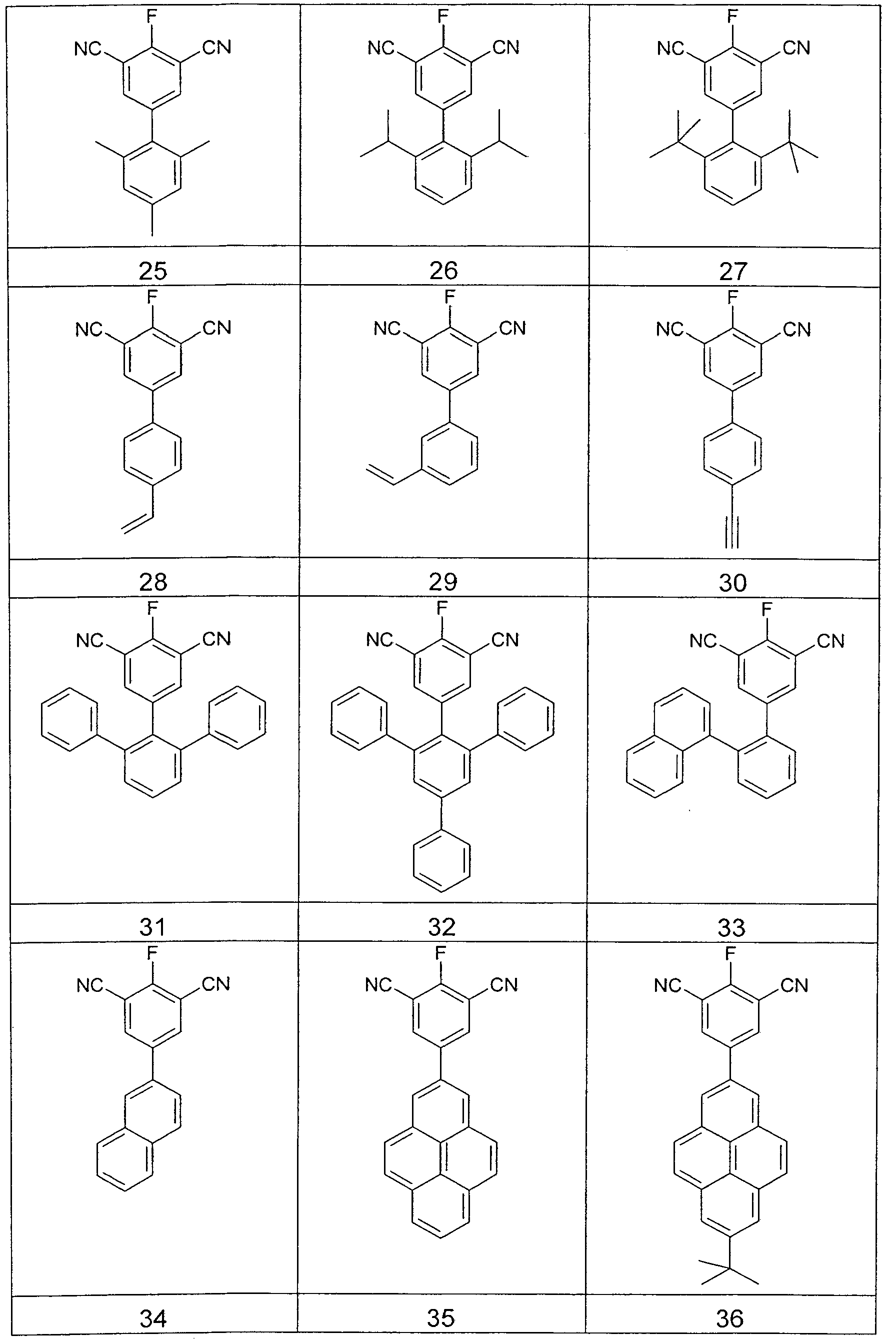

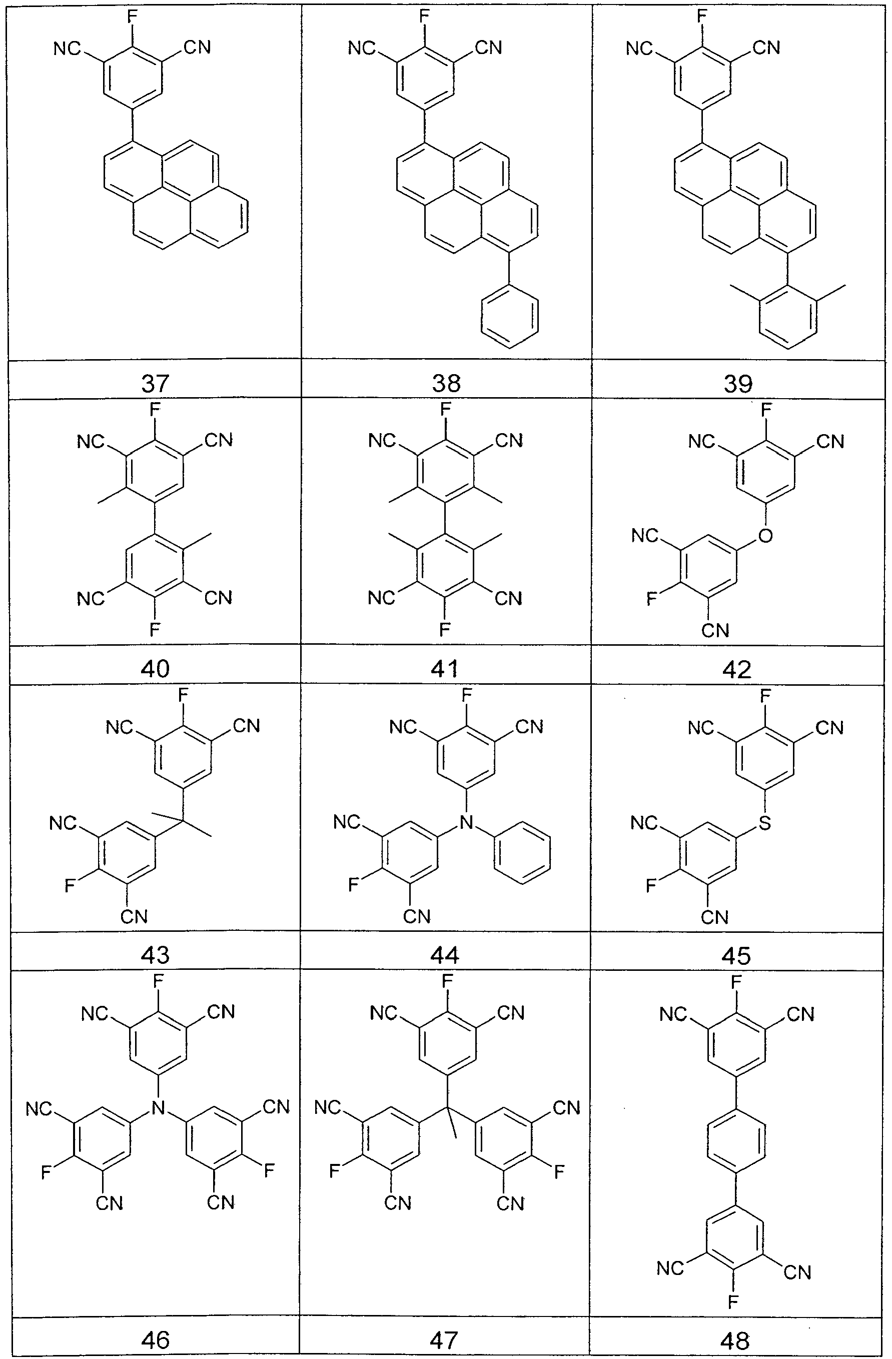

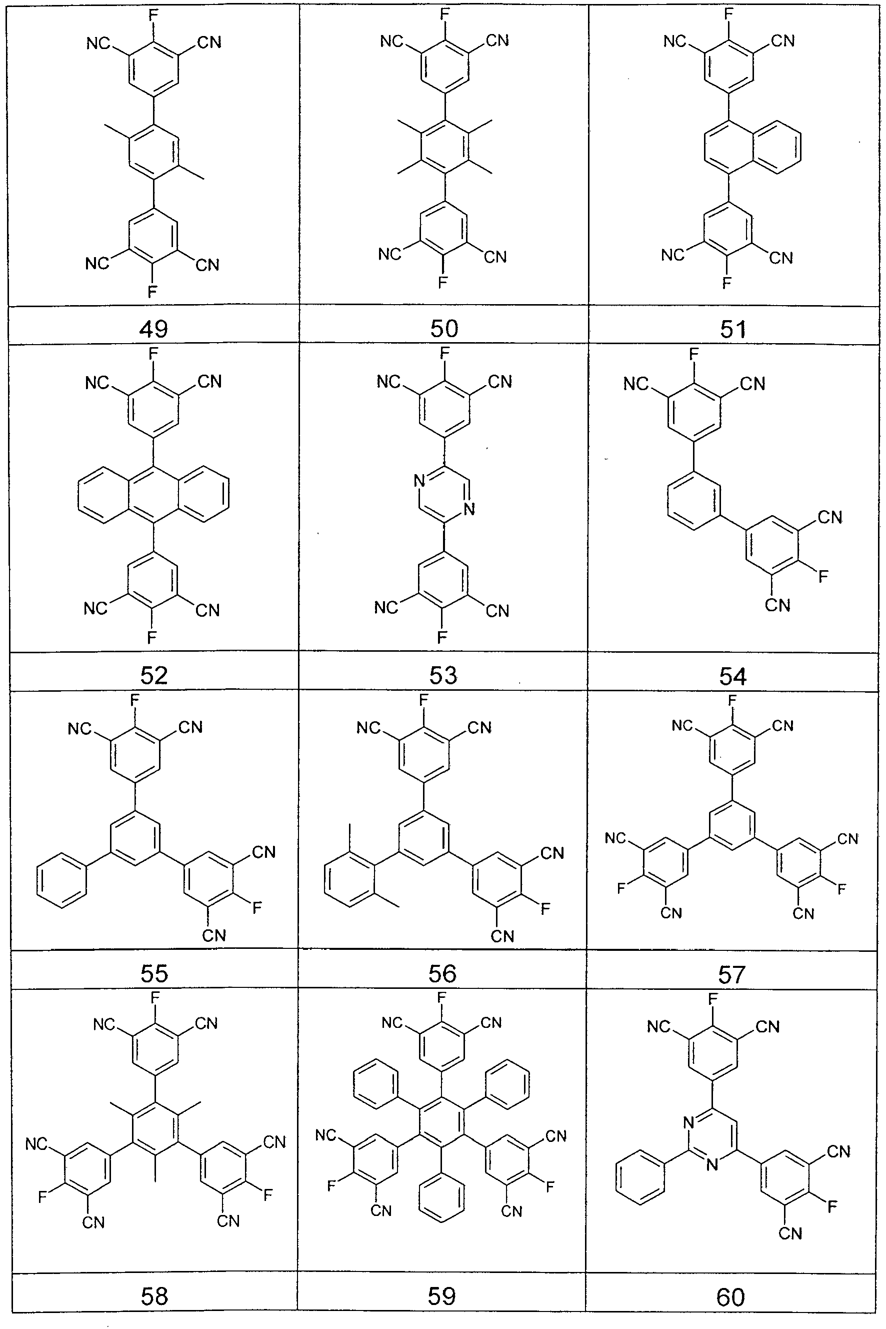

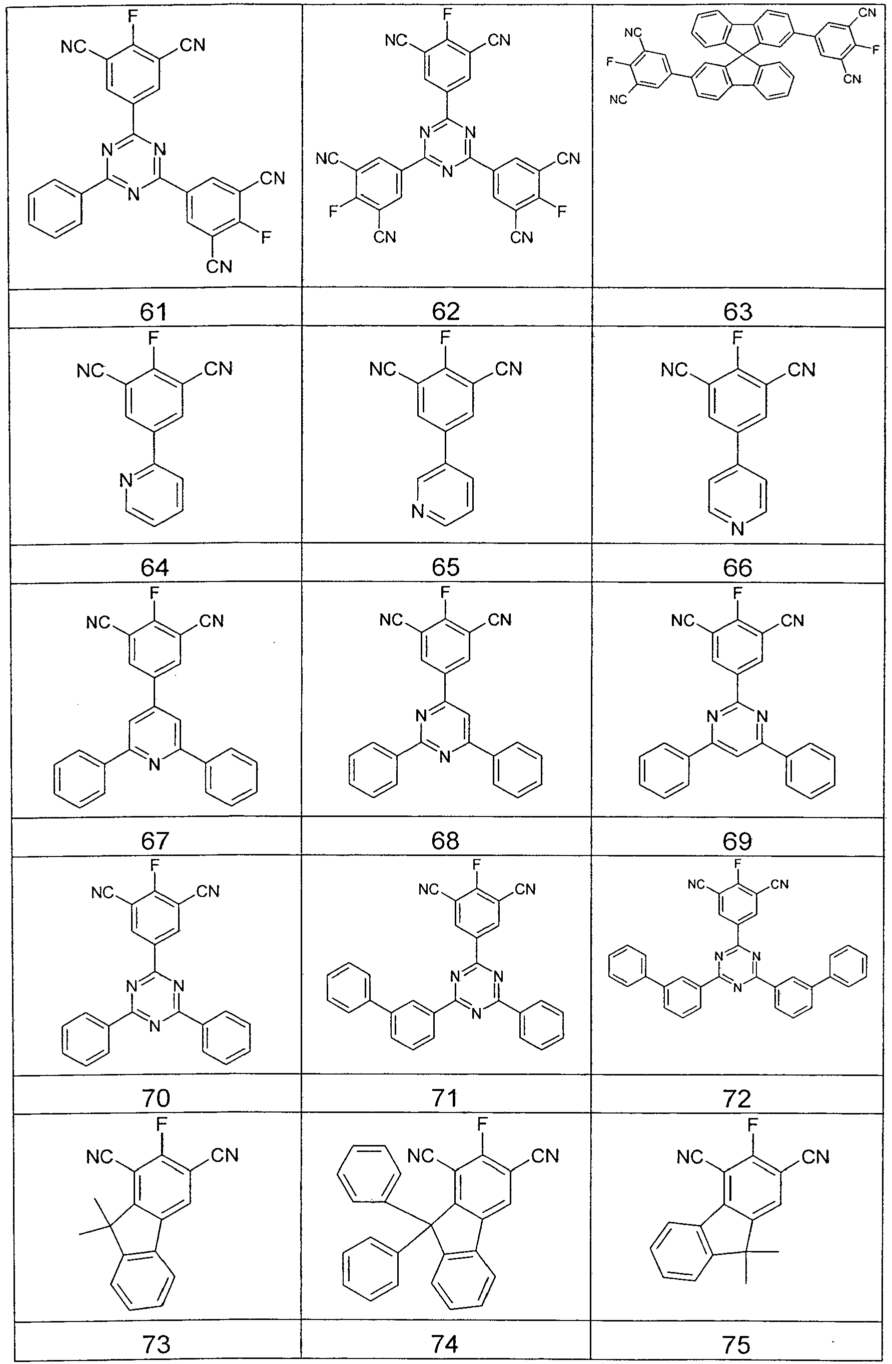

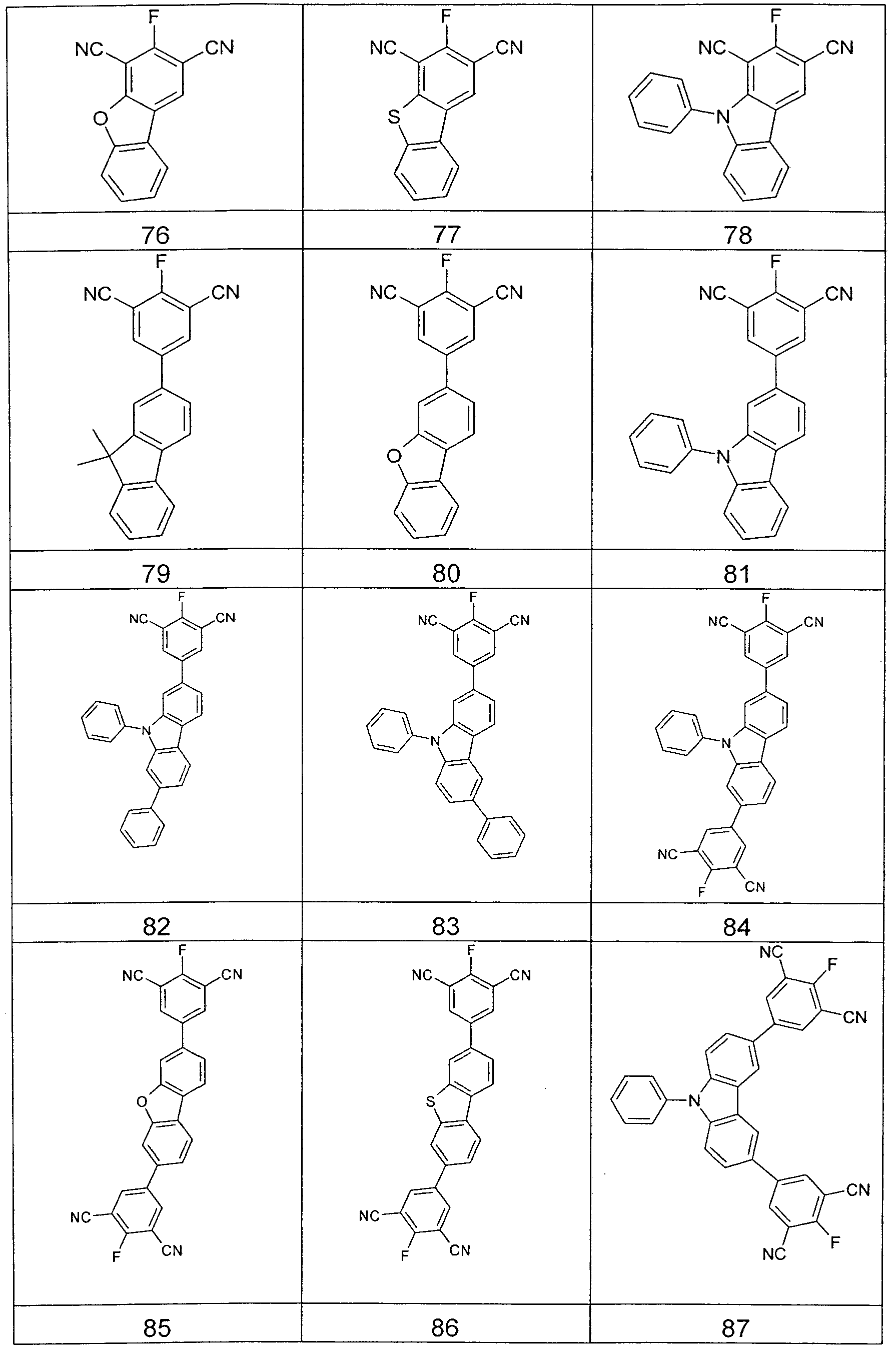

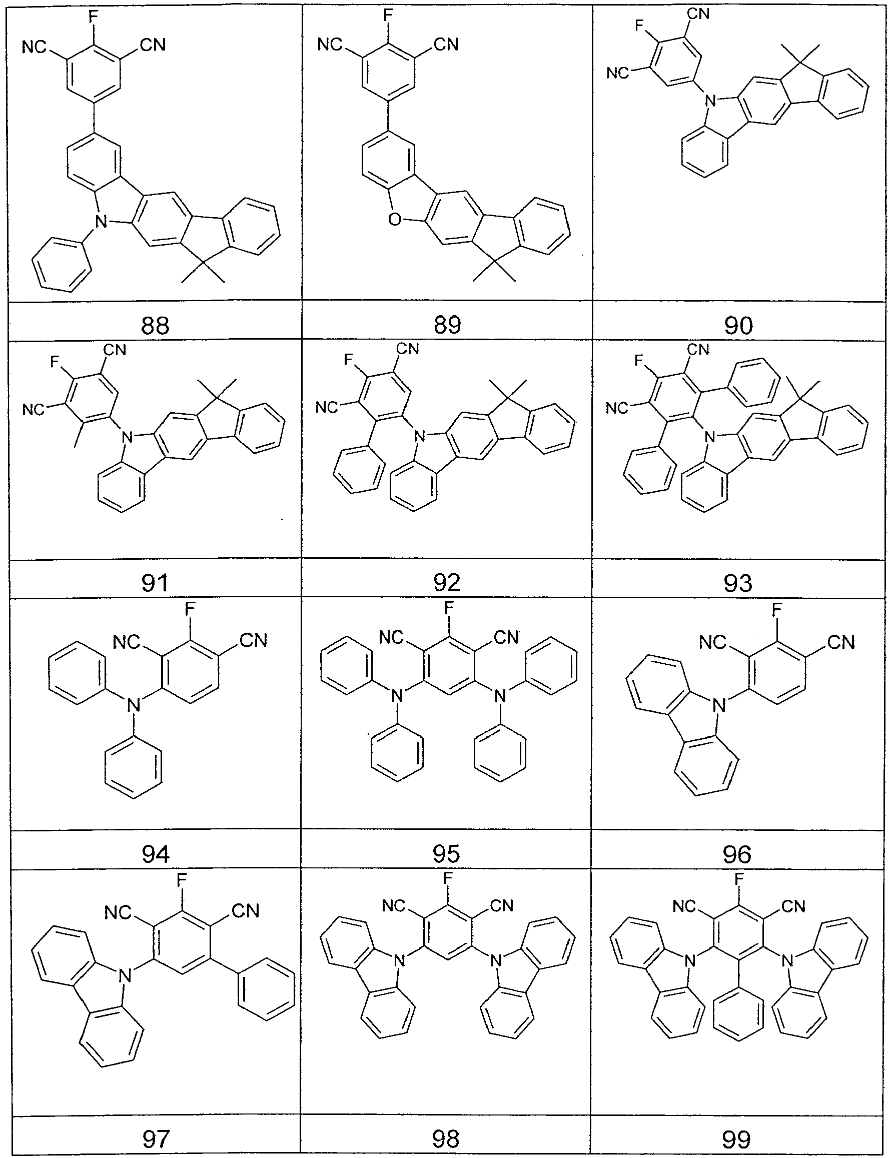

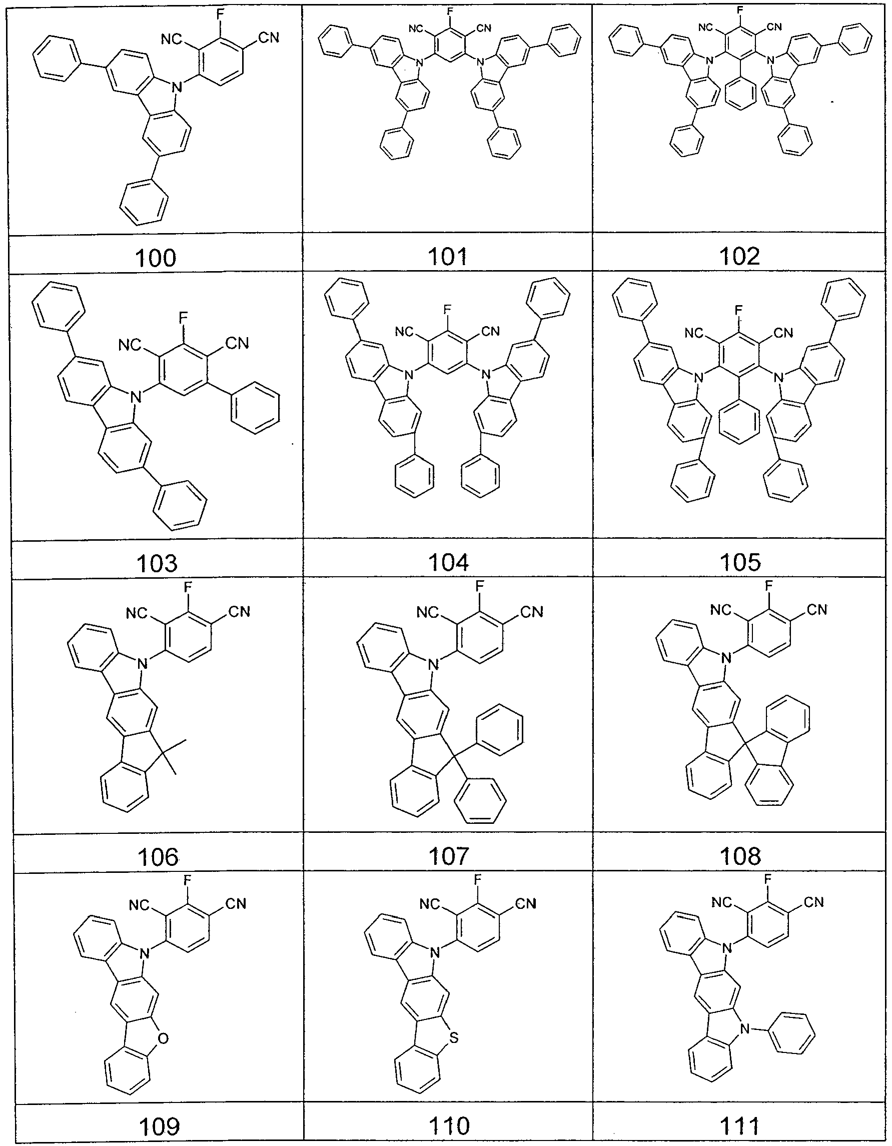

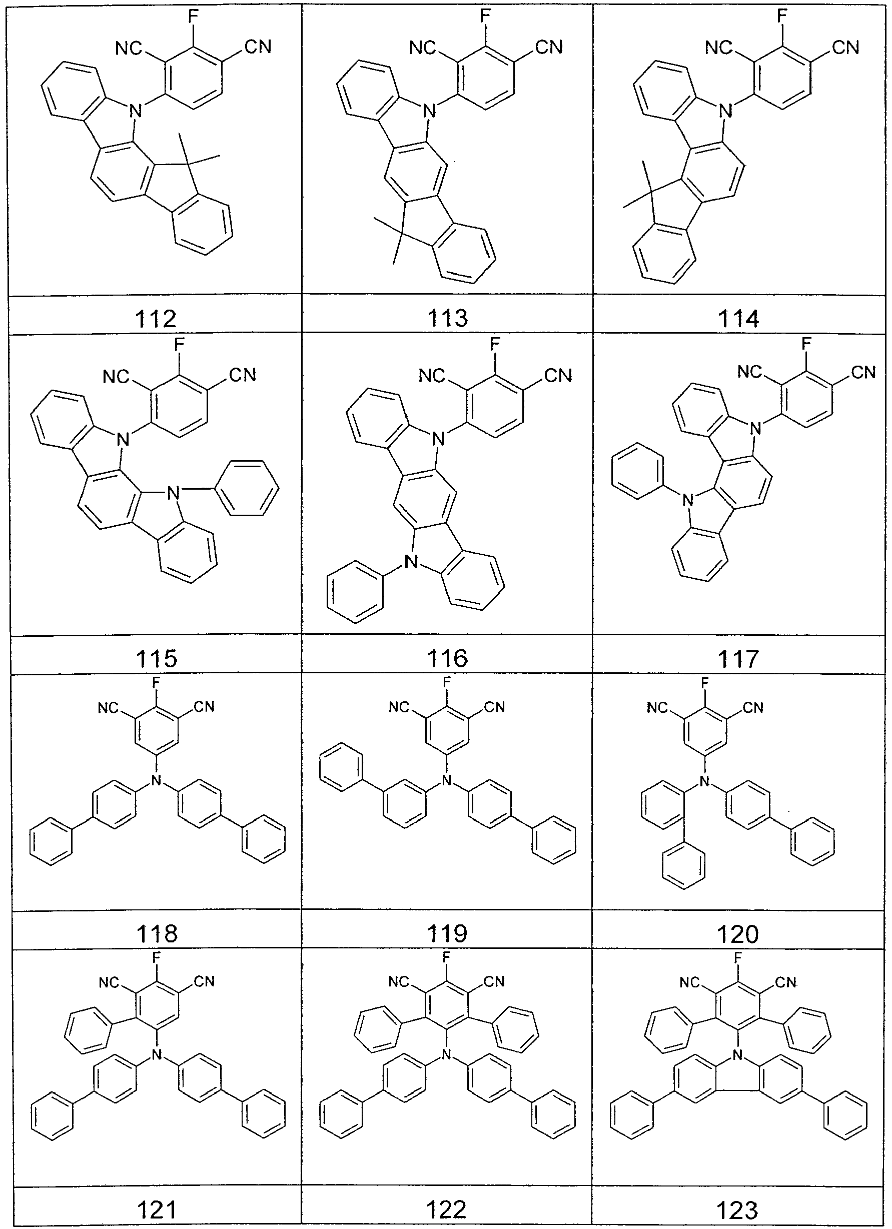

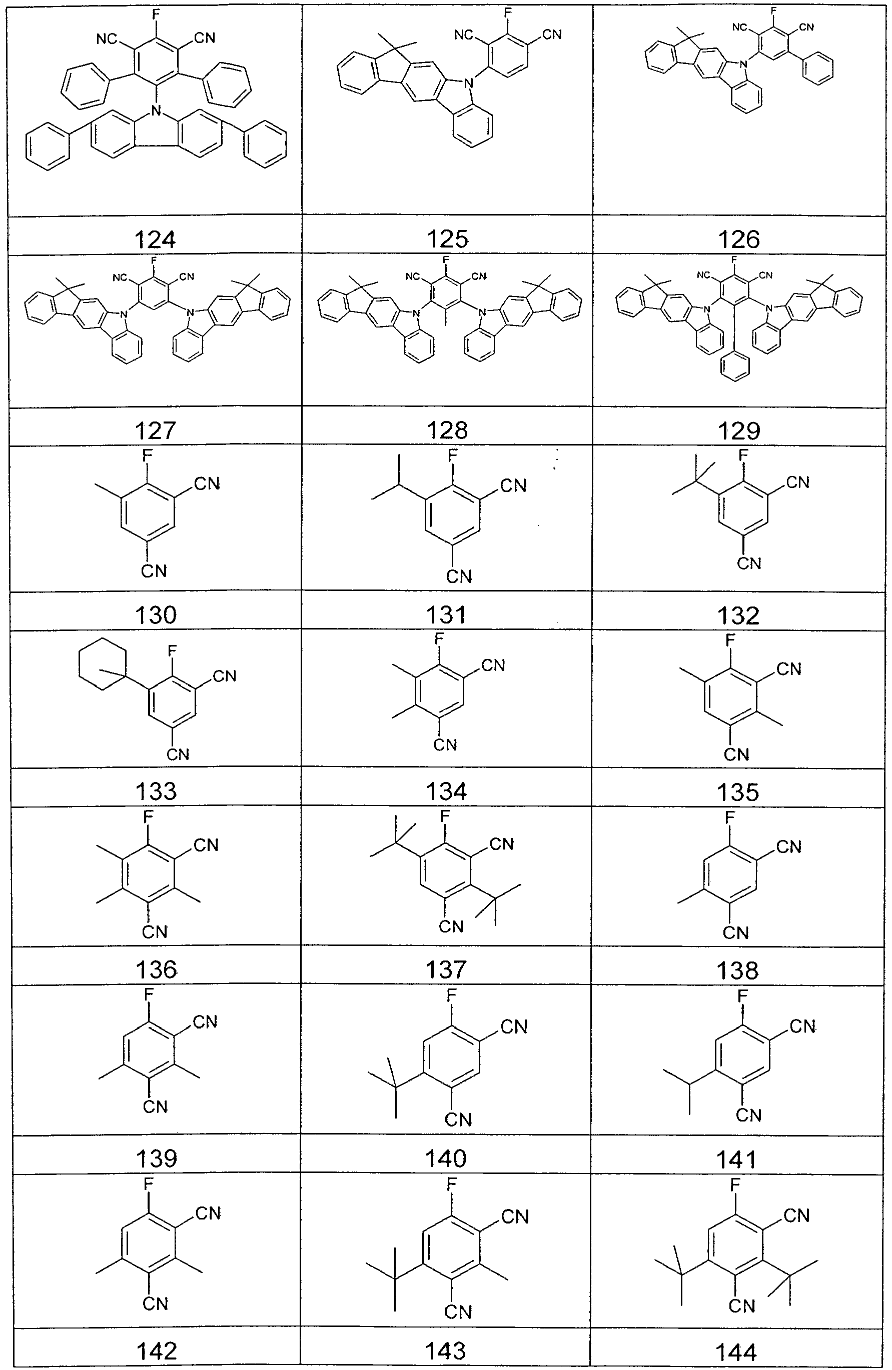

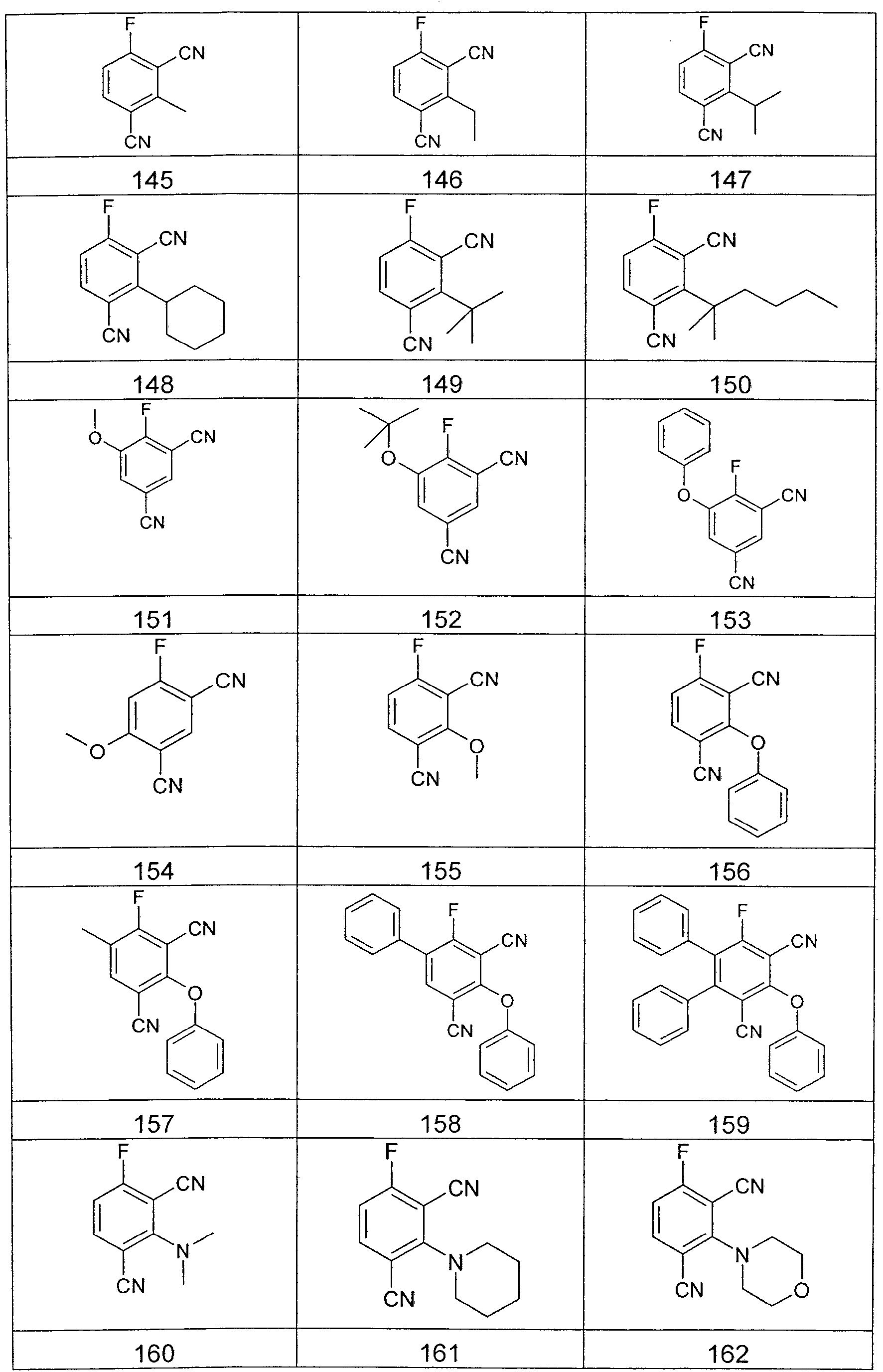

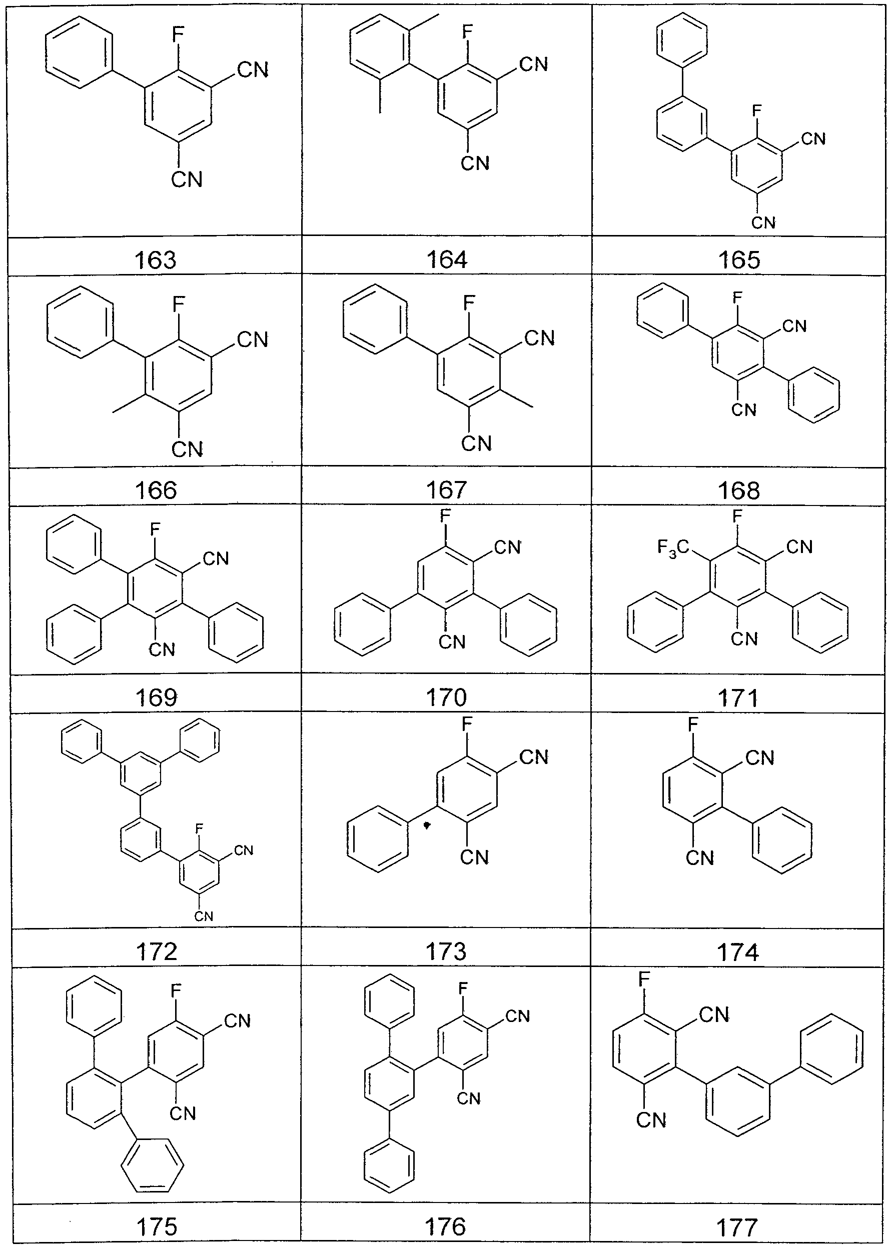

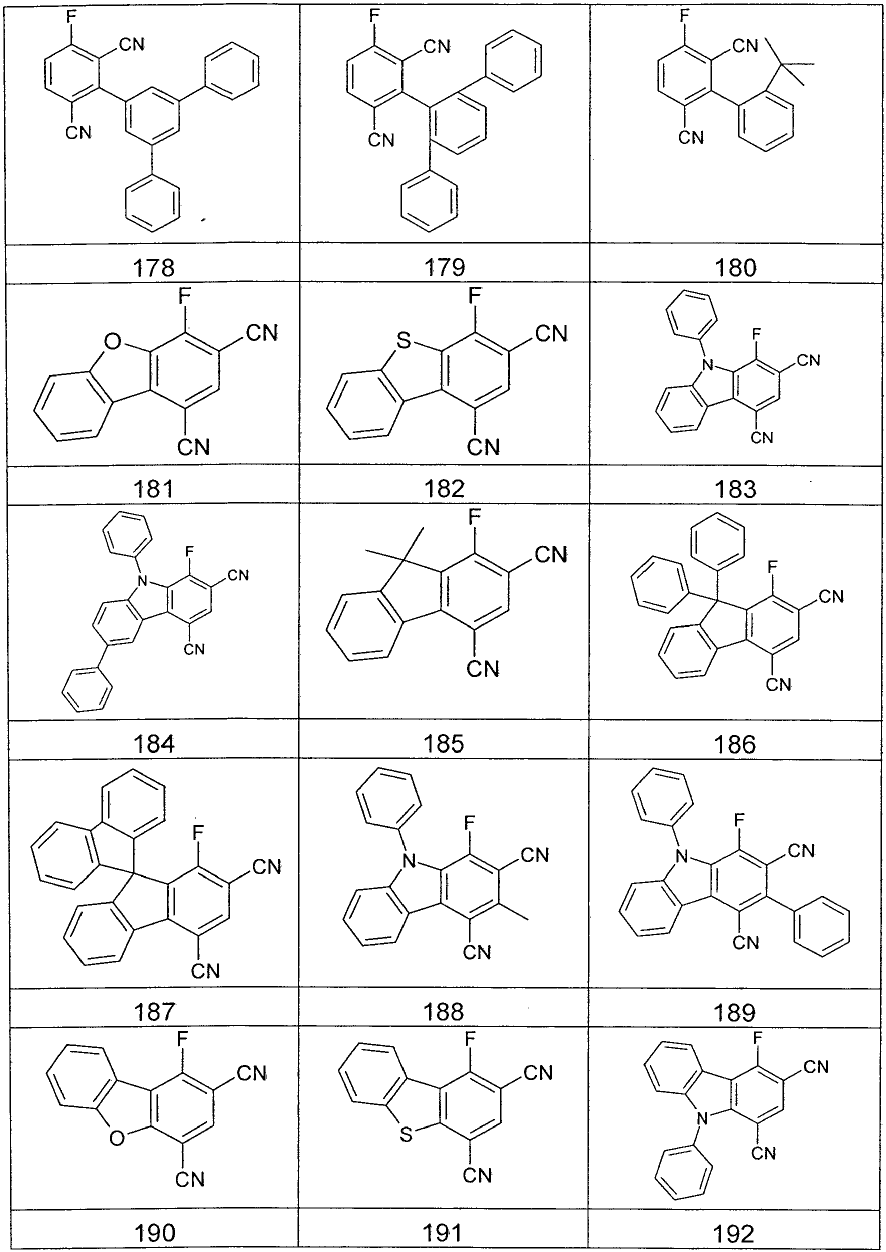

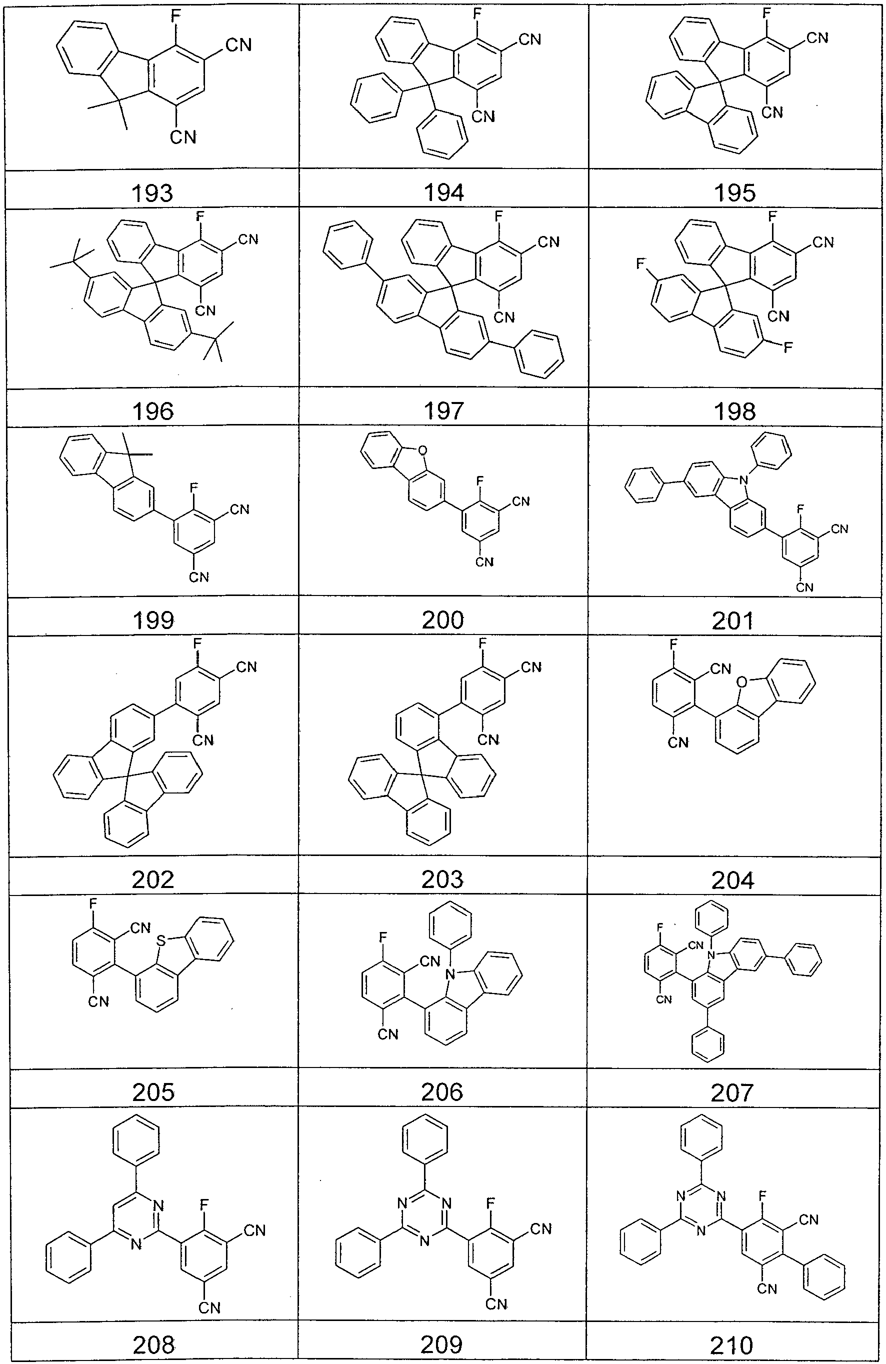

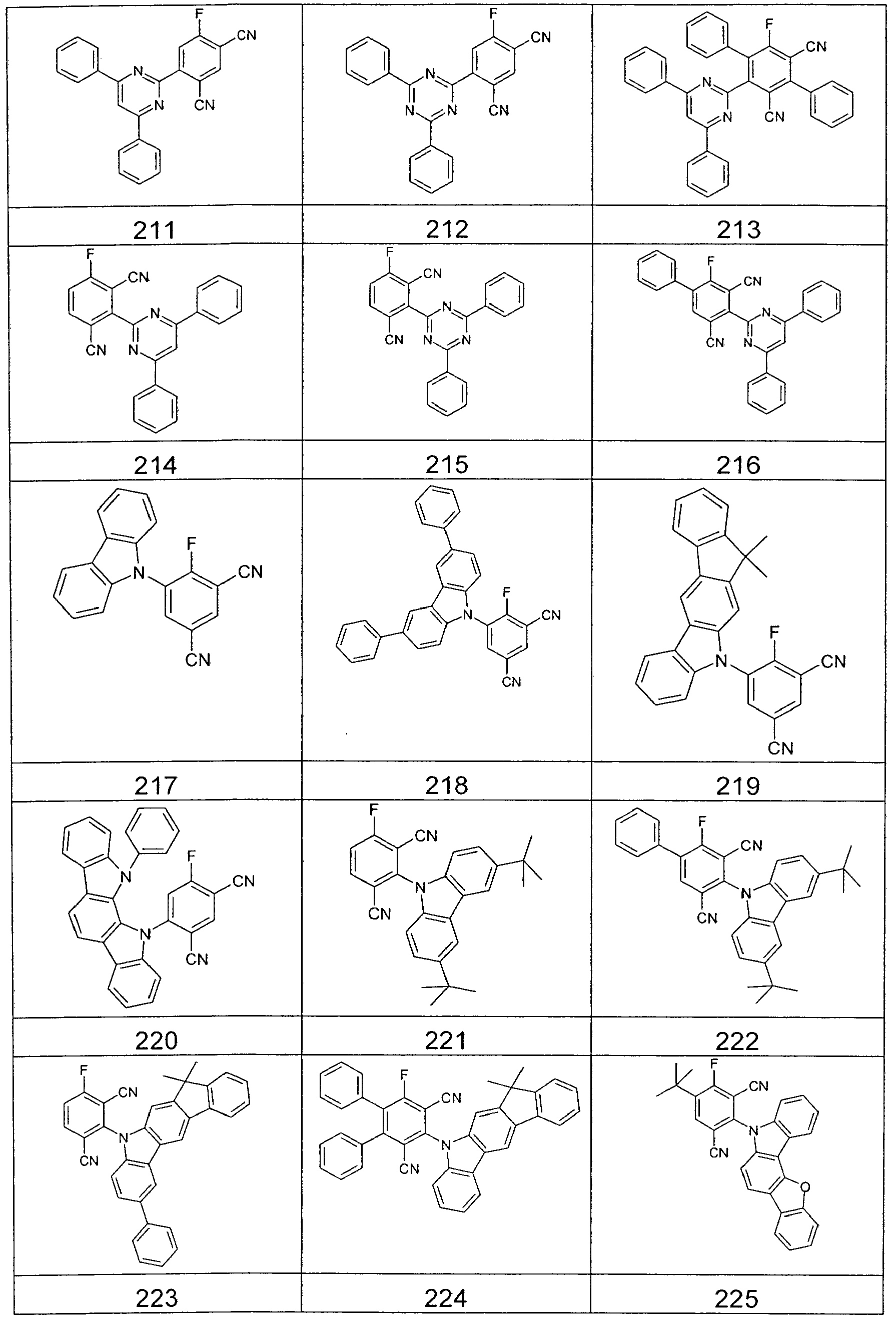

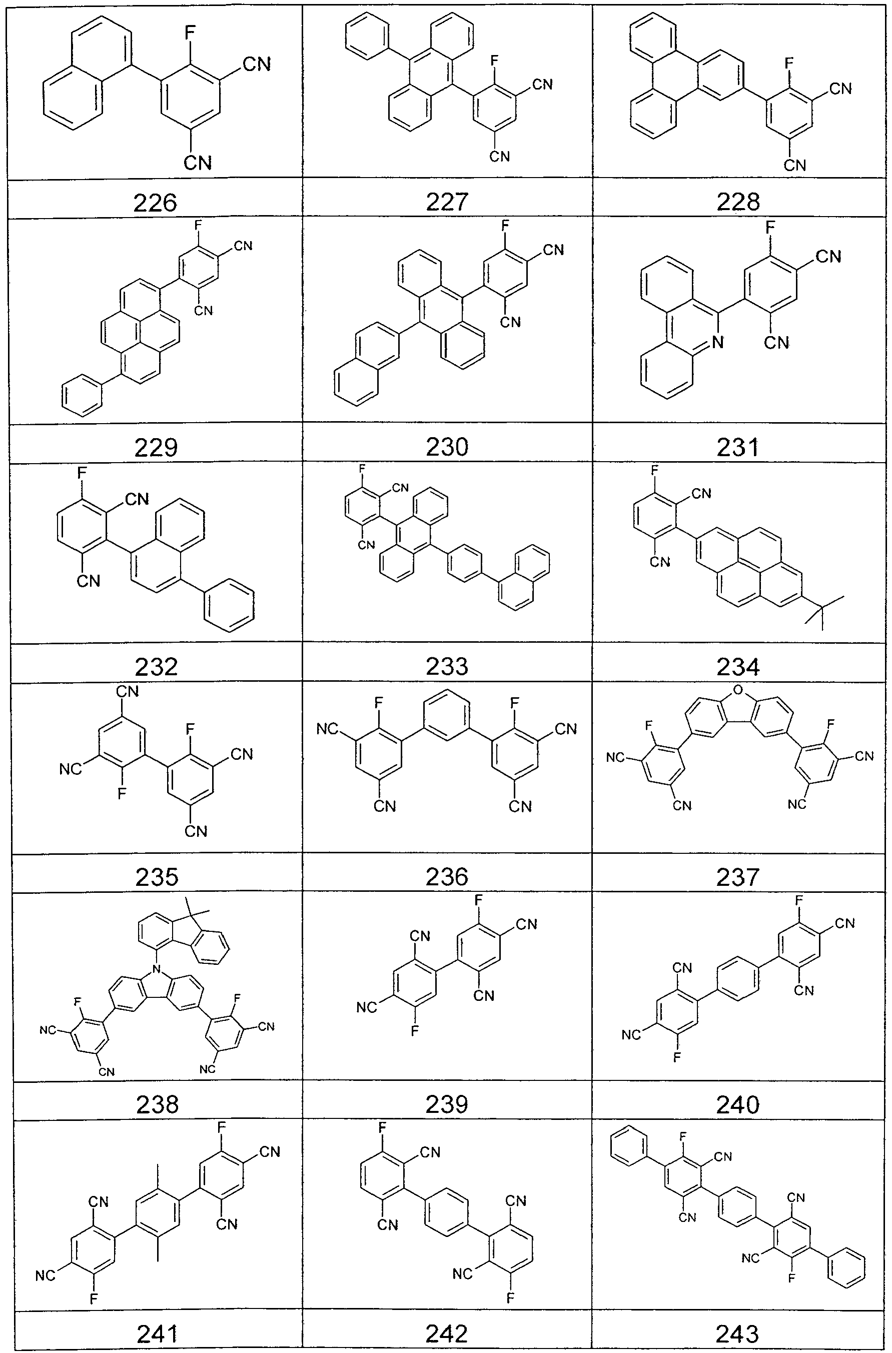

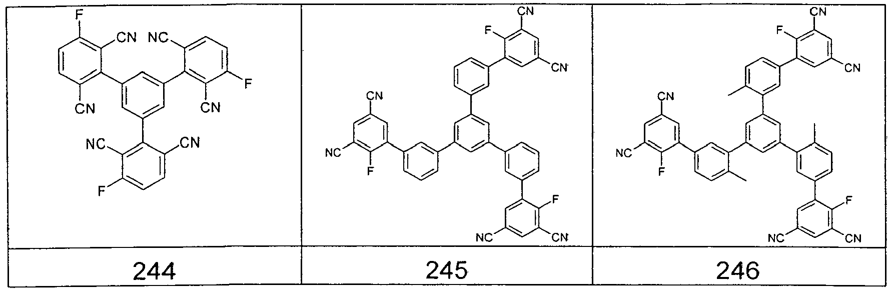

Die erfindungsgemäßen Verbindungen können beispielsweise nach den in Schema 1 und 2 skizzierten Wegen dargestellt werden.

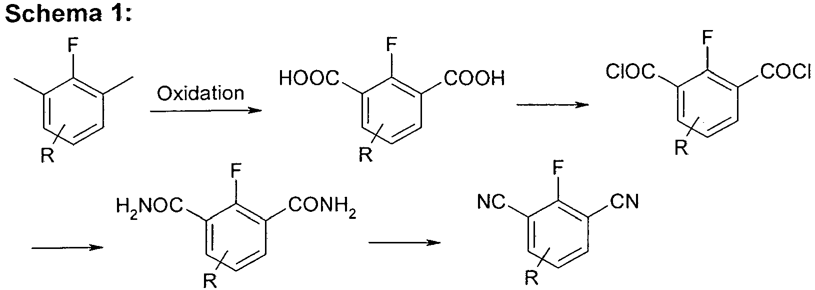

Die in Schema 1 dargestellte Synthese geht aus von 2-Fluor-meta-Xylol-Derivaten, wobei der Rest R wie oben definiert ist. Je nach genauer Struktur der Substituenten R kann hier auch eine Schutzgruppe erforderlich sein. Geeignete Schutzgruppen sind dem Fachmann der organischen Synthese bekannt. Diese werden in einem ersten Schritt zu den entsprechenden Isophthalsäuren oxidiert. Als Oxidationsmittel kommen beispielsweise anorganische Oxidationsmittel wir Permanganate, Chromate, Peroxodisulfate, Persulfate (Oxon), Hypochlorite, Chlorite, Wasserstoffperoxid oder Sauerstoff oder organische Oxidationsmittel wie Peroxide oder Percarbonsäuren in Frage. Im nächsten Schritt werden die Carbonsäuregruppen in die entsprechenden Carbonsäurechloride überführt, z. B. mittels Einwirkung von anorganischen Säurechloriden wie Thionylchlorid, Phosphorylchlorid oder Oxalylchlorid, gegebenenfalls in Gegenwart eines Aktivators wie DMF. Im nächsten Schritt wird das Carbonsäurechlorid durch durch Einwirkung von Ammoniak zum Carbonsäureamid umgesetzt. Dieses wird durch Einwirkung wasserentziehender Mittel, wie z. B. anorganischen Säurechloriden wie Thionylchlorid, Phosphorylchlorid oder Oxalylchlorid, gegebenenfalls in Gegenwart eines Aktivators wie DMF, zum Nitril entwässert.

Die analoge Reaktionssequenz ist auch ausgehend von 6-Fluor-metaxylol-Derivaten durchführbar.

Steht R für Cl, Br oder Iod, lassen sich die Verbindungen durch dem Fachmann bekannte C-C- bzw. C-N-Kupplungsreaktionen wie Suzuki-, Negishi-, Yamamoto-, Grignard-Cross-, Heck-, Sonogashira-, Buchwald-, etc. Kupplung weiter funktionalisieren. Es ist auch möglich, die Halogenfunktionalität zunächst zu einem Boronsäurederivat umzusetzen. Diese Halogen- oder Boronsäure-substituierten Verbindungen eignen sich auch als Ausgangsstufe für die entsprechenden dimeren, trimeren, etc. Strukturen, also Strukturen der Formeln (1), (2) und (3), in denen n ≥ 1 ist, da diese oben genannten Kupplungsreaktionen auch mit einer entsprechenden disubstituierten, trisubstituierten, etc. Gruppe L durchgeführt werden können.

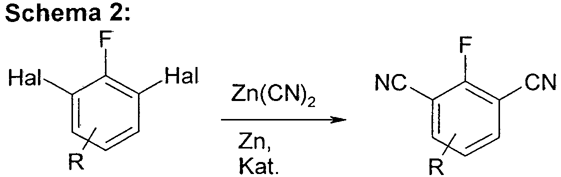

Die in Schema 2 dargestellte Synthese geht aus von 2-Fluor-1,3-dihalogenbenzol-Derivaten, wobei Hal für Cl, Br oder I steht und der Rest R wie oben definiert ist. Je nach genauer Struktur der Substituenten R kann hier auch eine Schutzgruppe erforderlich sein. Geeignete Schutzgruppen sind dem Fachmann der organischen Synthese bekannt. Diese werden mit Zn(CN)₂ in Gegenwart von Zink und einem Katalysator zu den entsprechenden Cyanoverbindungen umgesetzt. Als Katalysator eignen sich Palladiumverbindungen mit Phosphinliganden, beispielsweise Pd(OAc)₂ mit XPhos.

Die analoge Reaktionssequenz ist auch ausgehend von 6-Fluor-1,3-dihalogenbenzol-Derivaten durchführbar.

Ein weiterer Gegenstand der vorliegenden Erfindung ist daher ein Verfahren zur Herstellung einer Verbindung gemäß Formel (1) bzw. (2) bzw. (3), umfassend die Reaktionsschritte:
a) Oxidation eines Fluor-meta-Xylolderivats zur Carbonsäure; und
b) Umsetzung der Carbonsäurefunktionalitäten zu Nitrilgruppen.

Ein nochmals weiterer Gegenstand der vorliegenden Erfindung ist ein Verfahren zur Herstellung einer Verbindung gemäß Formel (1) bzw. (2) bzw. (3) durch Umsetzung eines Fluor-meta-dihalogenbenzol-Derivats mit Zn(CN)₂ und Zn in Gegenwart eines Katalysators.

Die erfindungsgemäßen Verbindungen eignen sich als Synthesevorstufe für die Synthese von Materialien, die in organischen Elektrolumineszenzvorrichtungen eingesetzt werden können. Dabei ist der Fluorsubstituent dieser Verbindungen eine reaktive Abgangsgruppe, die in einer nukleophilen aromatischen Substitution (S_{N}2 aromatisch) selektiv durch ein Nukleophil ersetzt werden kann.

Ein weiterer Gegenstand der vorliegenden Erfindung ist daher die Verwendung einer erfindungsgemäßen Verbindung als Edukt in einer nukleophilen aromatischen Substitutionsreaktion.

Nochmals ein weiterer Gegenstand der vorliegenden Erfindung ist ein Verfahren zur Herstellung einer Verbindung gemäß Formel (34) bzw. Formel (35) bzw. Formel (36), 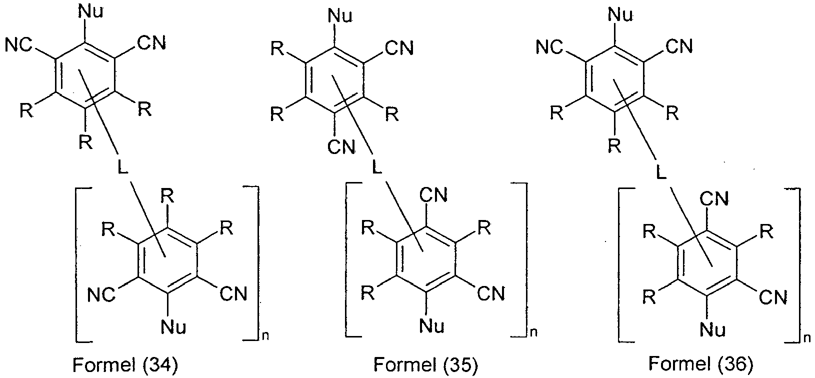 wobei Nu für eine nukleophile Gruppe steht und die anderen verwendeten Symbole die oben genannten Bedeutungen aufweisen,
durch Umsetzung einer Verbindung gemäß Formel (1) bzw. Formel (2) bzw. Formel (3) mit einem Nukleophil.

Dabei handelt es sich bei dieser Umsetzung um eine nukleophile aromatische Substitution. Dem Fachmann ist dieser Reaktionstyp bekannt und er weiß auch, bei welchen Strukturen es sich um Nukleophile handelt.

Der Verbindungen der Formel (34) bzw. (35) bzw. (36), die das Reaktionsprodukt des erfindungsgemäßen Verfahrens sind, eignen sich für den Einsatz in einer elektronischen Vorrichtung, insbesondere in einer organischen Elektrolumineszenzvorrichtung. Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die erfindungsgemäßen Verbindungen zeichnen sich durch einen oder mehrere der folgenden überraschenden Vorteile gegenüber dem Stand der Technik aus:
1. Die erfindungsgemäßen Verbindungen lassen sich in hoher Ausbeute, mit hoher Selektivität und unter milden Reaktionsbedingungen in einer nukleophilen aromatischen Substitutionsreaktion umsetzen. Dabei entstehen die Reaktionsprodukte in sehr hoher Reinheit, wodurch eine aufwändige Reinigung, die immer auch mit Materialverlusten verbunden ist, entfallen kann oder zumindest nur in geringem Ausmaß erforderlich ist. Insbesondere ist eine chromatographische Reinigung der Materialien nicht erforderlich.
2. Die erfindungsgemäßen Verbindungen sind wertvolle Vorstufen zur Synthese von Materialien, welche in organischen Elektrolumineszenzvorrichtungen eingesetzt werden können.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten nicht kommerziell erhältlichen Verbindungen.

### A: Synthese:

### Beispiel S1: 5-Brom-2-fluor-isophthalsäurenitril, S1

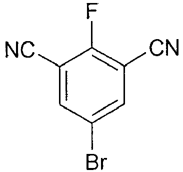

### a) S1a: 5-Brom-2-fluor-isophthalsäure, S1a

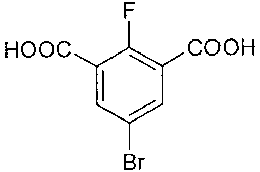

Durchführung analog H. G. Menzella, J. Med. Chem., 2009, 52 (6), 1518. Ein auf 90 °C erwärmtes Gemisch aus 203.1 g (1 mol) 5-Brom-2-fluor-m-xylol [99725-44-7] und 2000 ml Wasser wird unter gutem Rühren löffelweise mit 663.8 g (4.2 mol) Kaliumpermanganat versetzt (Achtung: leicht exotherme Reaktion). Nach vollendeter Zugabe wird die Reaktionsmischung weitere 12 h unter Rückfluss gerührt. Man lässt auf 70 °C erkalten, saugt den gebildeten Braunstein über eine Celiteschicht ab, wäscht zweimal mit je 300 ml warmem Wasser nach und stellt unter -Rühren mit konz. 300 ml HCl sauer. Man lässt die Wasserphase erkalten und saugt dann vom ausgefallenen Produkt ab, wäscht dieses dreimal mit je 300 ml Wasser nach und trocknet dann im Vakuum. Ausbeute: 205.7 g (782 mmol), 78 %. Reinheit: ca. 98 % ig n. ¹H-NMR.

Analog werden folgende Verbindungen dargestellt:

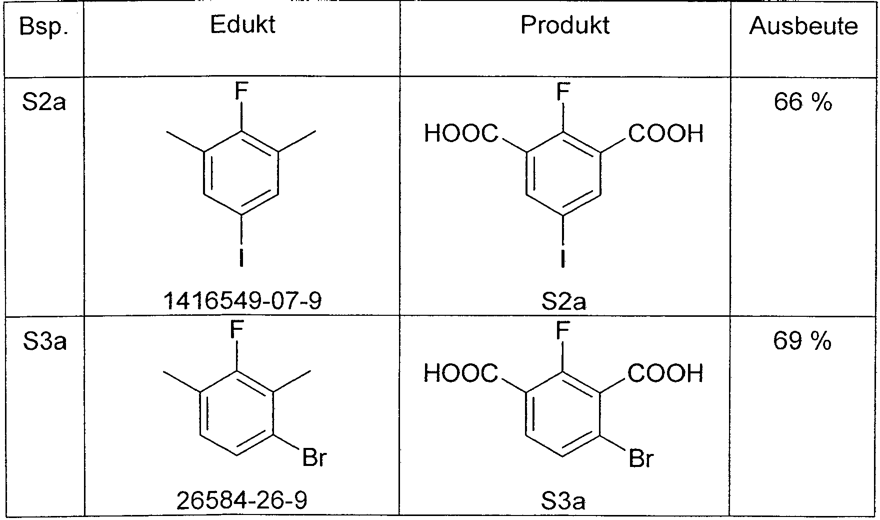

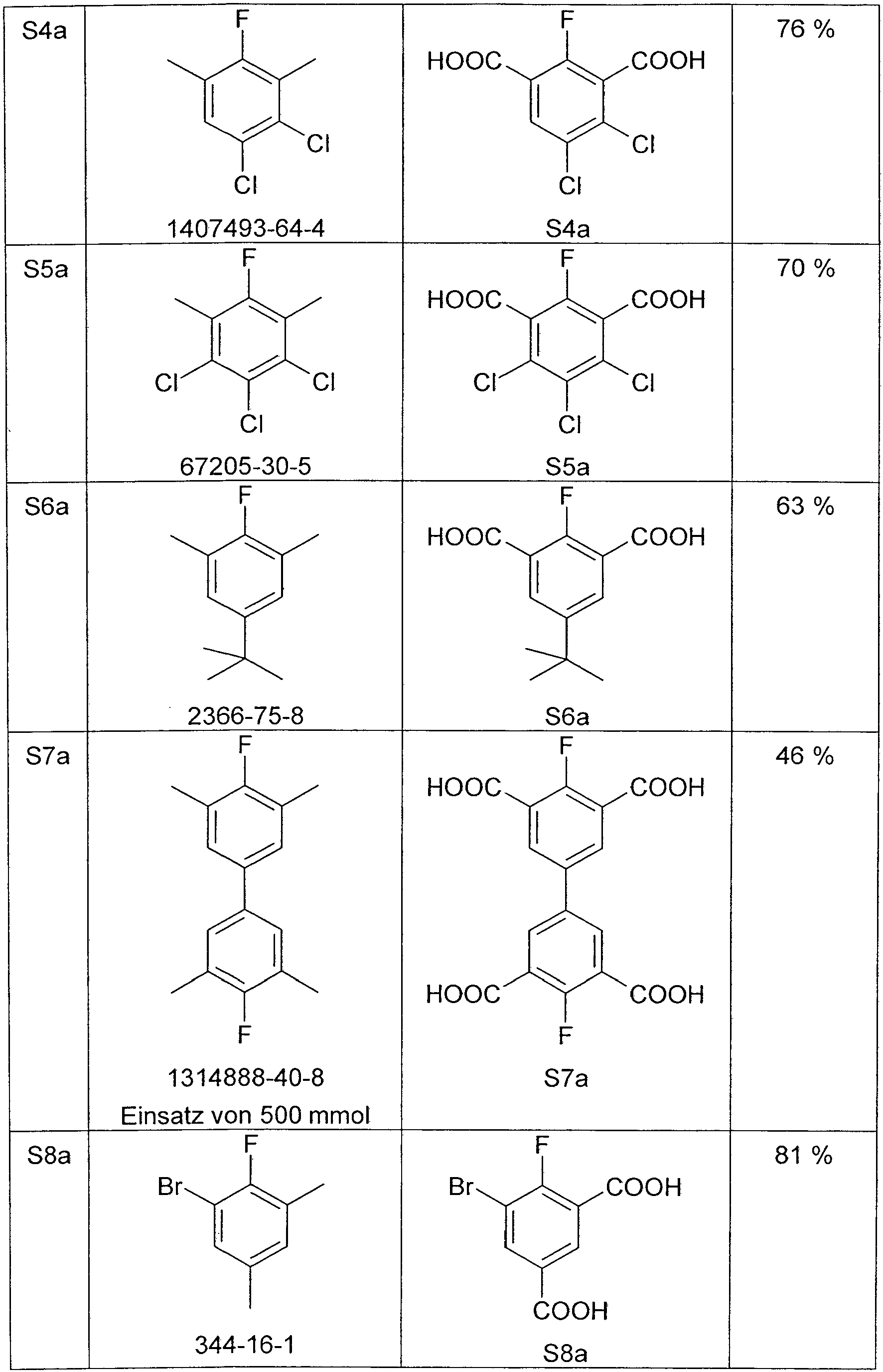

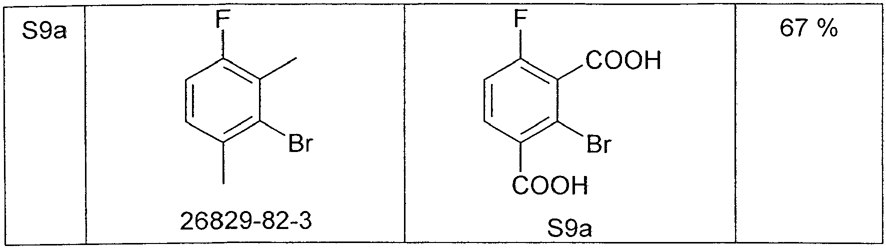

### b) S1b: 5-Brom-2-fluor-isophthalsäurechlorid, S1b

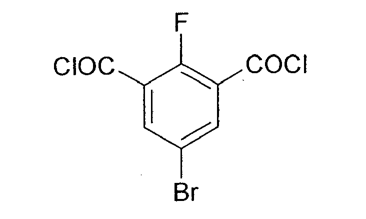

Durchführung analog W. P. Heilmann, J. Med. Chem., 1978, 21 (9), 906. Ein Gemisch aus 131.5 g (500 mmol) 5-Brom-2-fluor-isophthalsäure, S1a und 500 ml Thionylchlorid wird unter Rühren mit 20 Tropfen DMF versetzt und dann langsam auf 70 °C erwärmt. Nach beendeter HCl-Entwicklung rührt man noch 4 h nach und destilliert dann das überschüssige Thionylchlorid ab, bis der Rückstand zu einer fahlgelben kristallinen Masse erstarrt. Das Säurechlorid wird ohne weitere Reinigung verwendet. Ausbeute: quantitativ. Reinheit: ca. 98 % ig nach ¹H-NMR.

Analog werden folgende Verbindungen dargestellt:

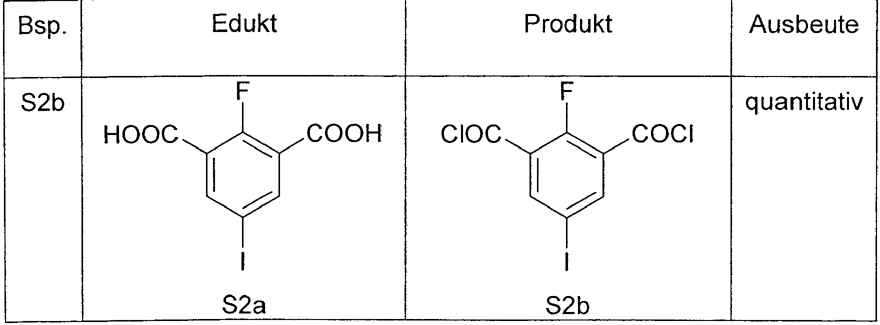

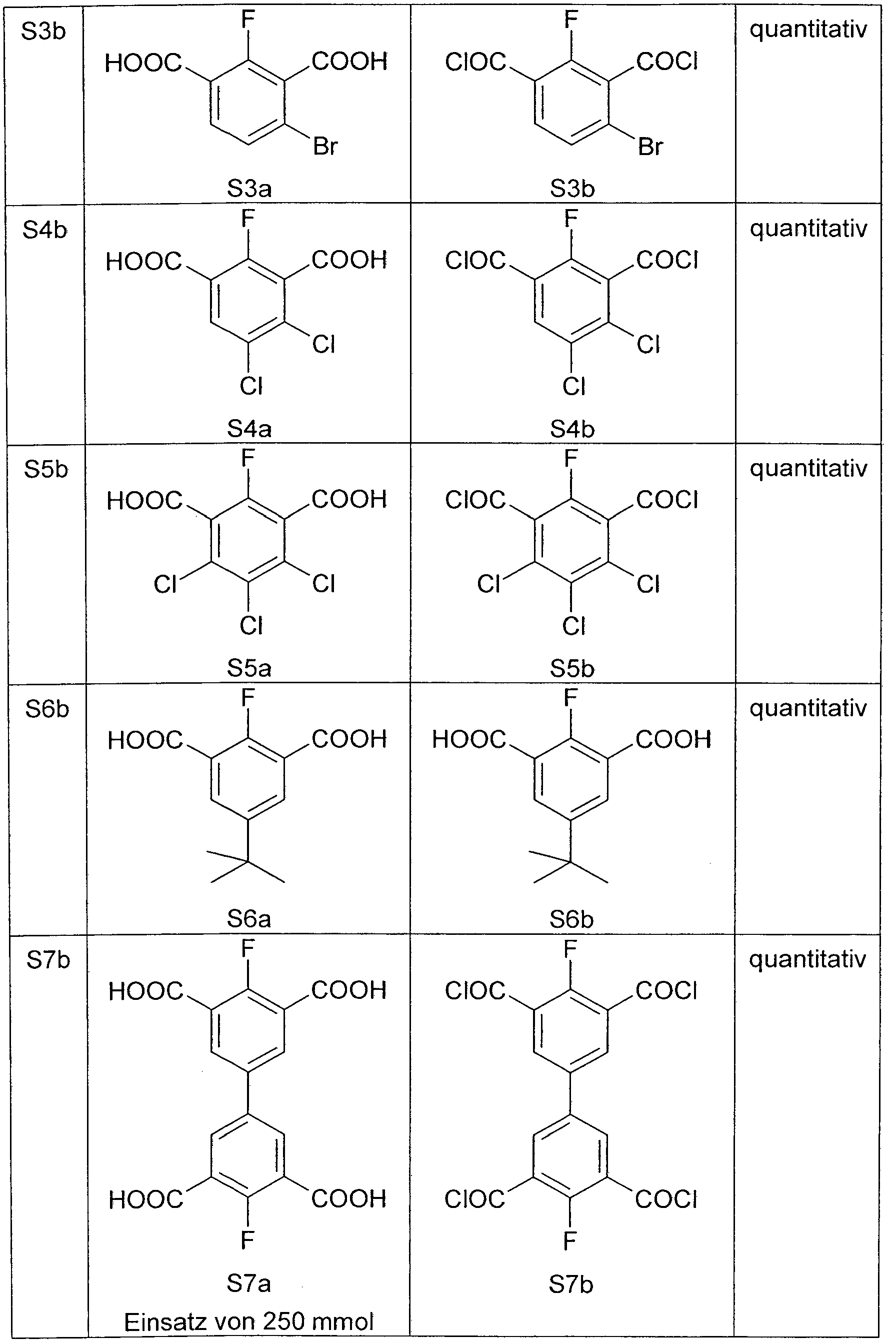

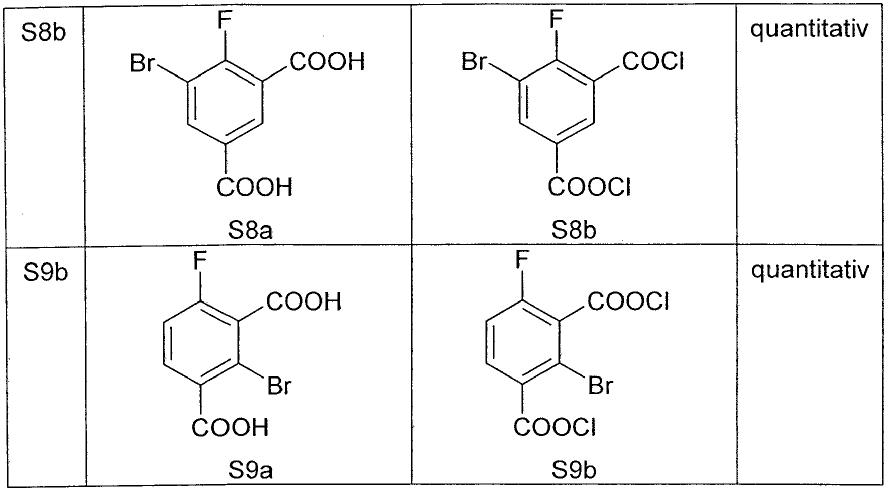

### c) S1c: 5-Brom-2-fluor-isophthalsäureamid, S1c

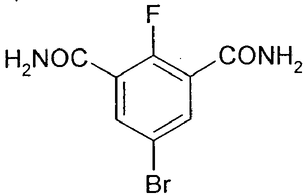

Durchführung analog W. P. Heilmann, J. Med. Chem., 1978, 21 (9), 906. Ein gut gerührtes Gemisch aus 300 ml konz. Ammoniumhydroxid und 800 ml Dioxan wird unter Eiskühlung tropfenweise so mit einer Lösung von 150.0 g (500 mmol) 5-Brom-2-fluor-isophthalsäurechlorid, S1b in 300 ml Dioxan versetzt, dass die Temperatur 50 °C nicht überschreitet. Nach beendeter exothermer Reaktion wird das Kältebad entfernt, die Reaktionsmischung wird nachgerührt, bis eine Innentemperatur von 25 °C erreicht ist, der ausgefallene Feststoff wird abgesaugt, dreimal mit 200 ml Wasser gewaschen und dann im Vakuum getrocknet. Ausbeute: 87.5 g (335 mmol) 67 %. Reinheit: ca. 98 % ig nach ¹H-NMR

Analog werden folgende Verbindungen dargestellt:

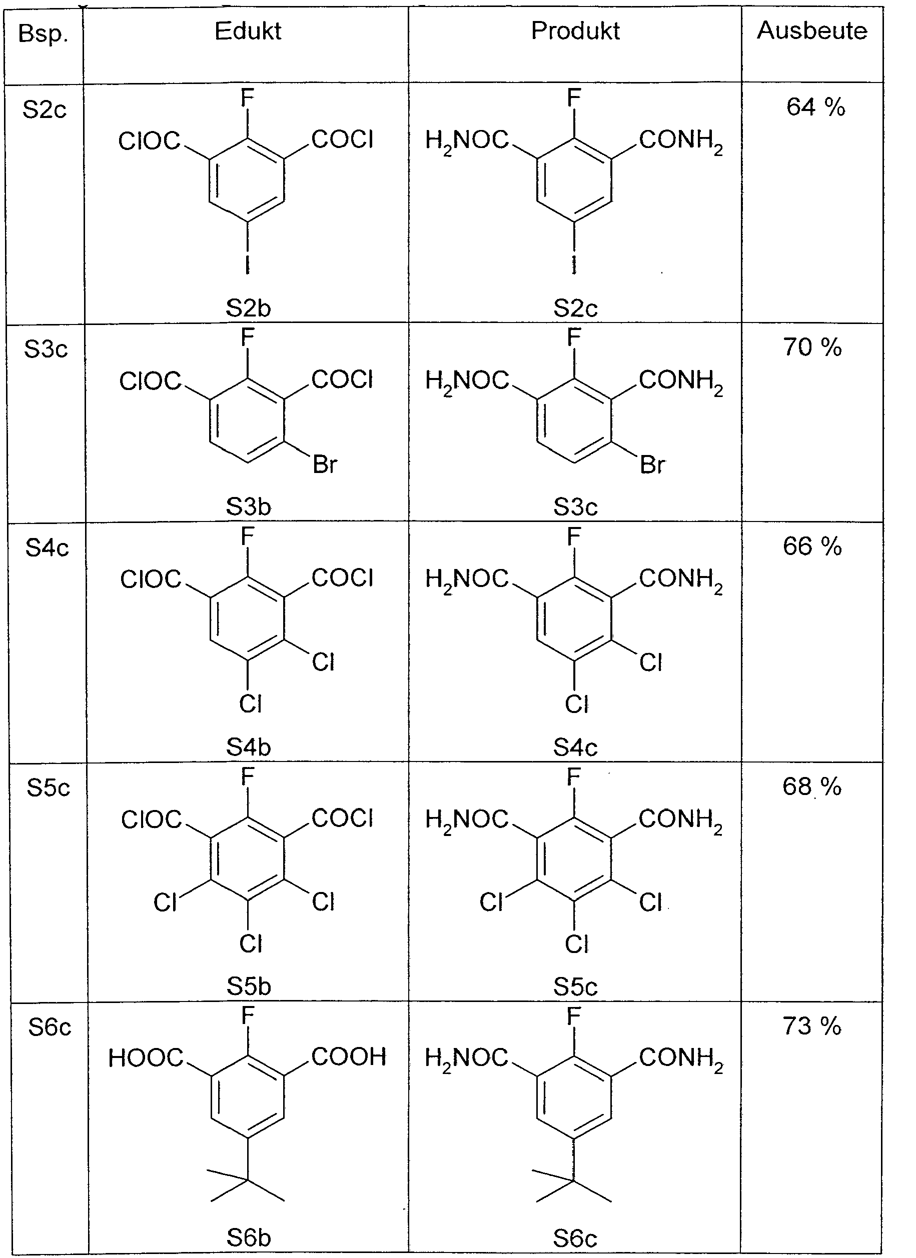

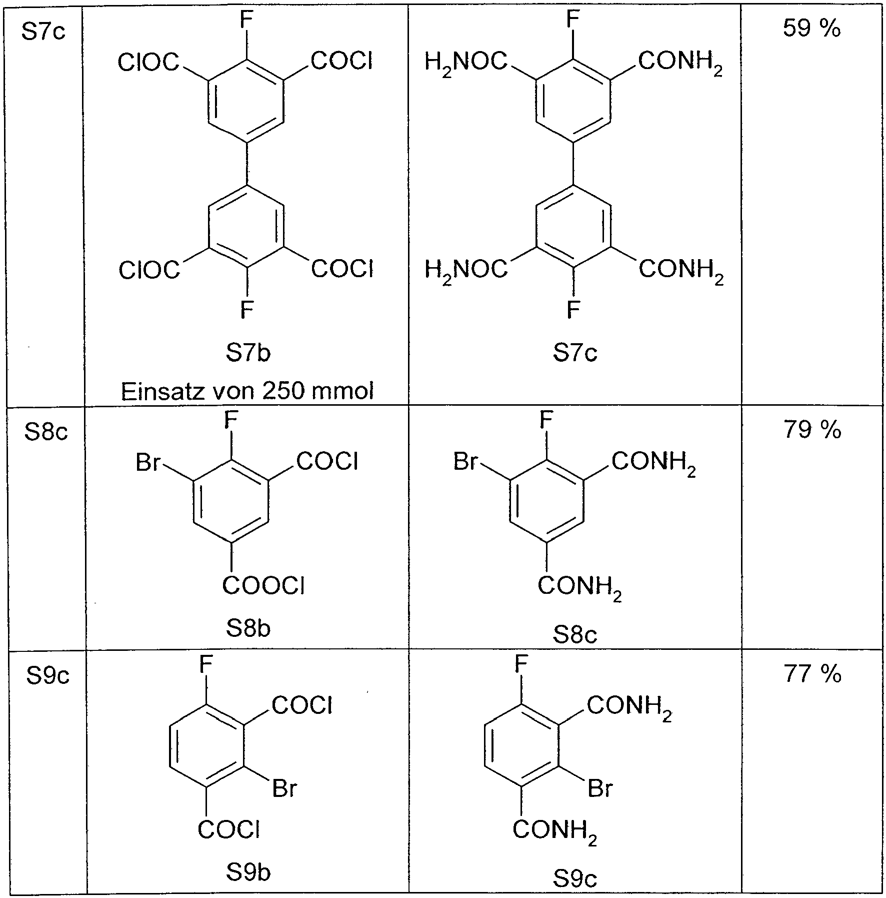

### d) S1: 5-Brom-2-fluor-isophthalsäurenitril, S1

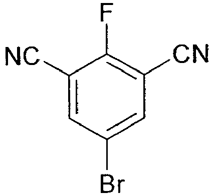

Durchführung analog W. P. Heilmann, J. Med. Chem., 1978, 21 (9), 906. Ein Gemisch aus 65.3 g (250 mmol) 5-Brom-2-fluor-isophthalsäureamid, S1c und 142 ml (1.5 mol) Phosphorylchlorid und 10 Tropfen DMF wird unter gutem Rühren langsam auf 90 °C erhitzt. Nach beendeter HCl-Entwicklung lässt man die Reaktionsmischung erkalten, gießt diese dann auf ein Gemisch aus 5 kg Eis und 1000 ml Wasser. Das ausgefallene Produkt wird abfiltriert, dreimal mit je 200 ml Wasser gewaschen und im Vakuum getrocknet. Die Reinigung erfolgt durch Umkristallsation aus Chlorbenzol. Ausbeute: 49.5 g (220 mmol) 88 %. Reinheit: ca. 98 % ig nach ¹H-NMR

Analog werden folgende Verbindungen dargestellt:

| Bsp. | Edukt | Produkt | Ausbeute |
|---|---|---|---|
| S2 | 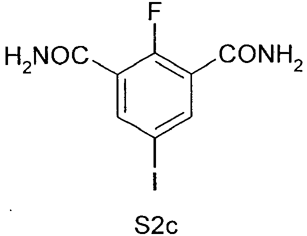 | 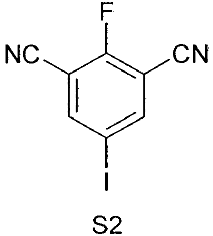 | 87 % |
| S3 | 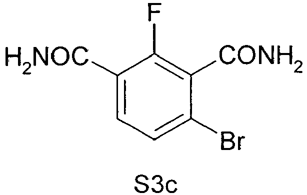 | 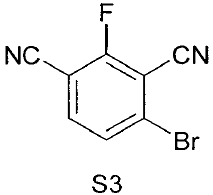 | 86 % |
| S4 | 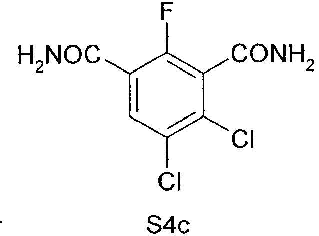 | 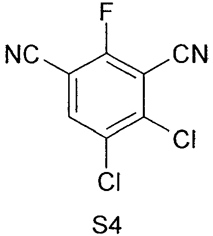 | 82 % |
| S5 | 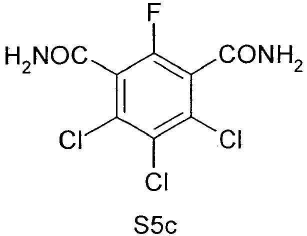 | 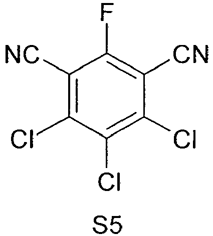 | 90 % |
| S6 | 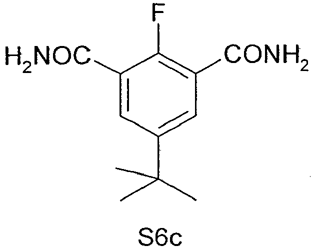 | 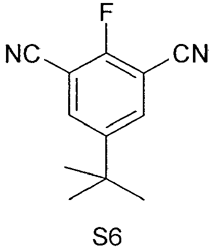 | 84 % |
| S7 | 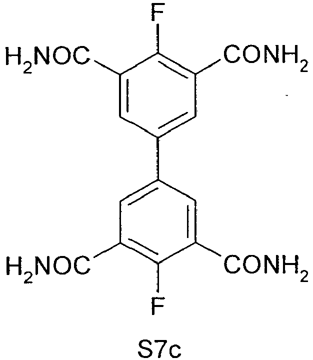 | 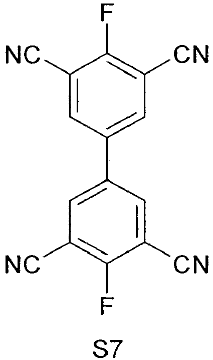 | 68 % |
| | Einsatz von 125 mmol | | |
| S8 | 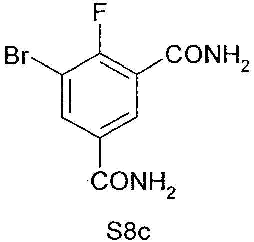 | 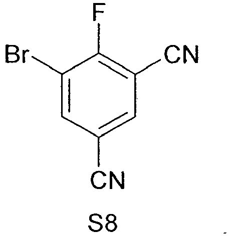 | 72 % |
| S9 | 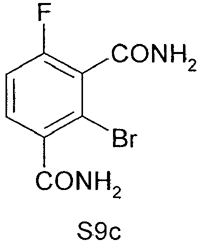 | 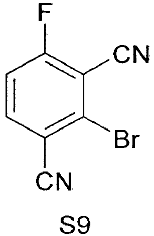 | 77 % |

### S10: 5-Phenyl-2-fluor-isophthalsäurenitril, S10

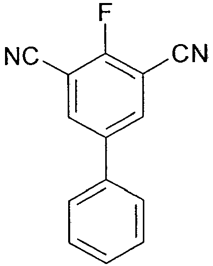

### Variante A:

Ein Gemisch aus 22.5 g (100 mmol) 5-Brom-2-fluor-isophthalsäurenitril, S1, 14.6 g (120 mmol) Phenylboronsäure [98-80-6], 42.5 g (200 mmol) Trikaliumphosphat, 1.8 g (6 mmol) Tri-o-tolylphosphin, 224 mg (1 mmol) Palladium(II)aceat, 200 ml Toluol, 50 ml Dioxan und 200 ml Wasser wird so lange unter Rückfluss erhitzt, bis das 5-Brom-2-fluor-isophthalsäurenitril, S1 verbraucht ist (ca. 6 h). Nach Erkalten trennt man die wässrige Phase ab, filtriert die organische Phase über ein Celite-Bett, um Palladium zu entfernen, wäscht das Filtrat dreimal mit je 200 ml Wasser, einmal mit 200 ml gesättigter Kochsalzlösung und trocknet über Magnesiumsulfat. Der nach Entfernen des Lösungsmittels erhaltene Rückstand wird zweimal aus DMF umkristallisiert. Ausbeute: 15.1 g (68 mmol) 68 %. Reinheit: ca. 99 %ig nach ¹H-NMR.

### Variante B:

Ein Gemisch aus 22.5 g (100 mmol) 5-Brom-2-fluor-isophthalsäurenitril, S1, 14.6 g (120 mmol) Phenylboronsäure, 17.5 g (300 mmol) Kaliumfluorid, wasserfrei, 263 mg (1.3 mmol) Tri-tert-butylphosphin, 224 mg (1 mmol) Palladium(II)acetat, 250 ml THF wird so lange unter Rückfluss erhitzt, bis das 5-Brom-2-fluor-isophthalsäurenitril, S1 verbraucht ist (ca. 2 h). Nach Erkalten filtriert man über ein Celite-Bett ab und wäscht mit 200 ml THF nach, um Salze und Palladium zu entfernen. Man engt das Filtrat zur Trockene ein, rührt den Rückstand mit 200 ml eines Gemischs aus Wasser/EtOH (1:1, vv) aus, saugt den Feststoff ab, wäscht diesen dreimal mit je 100 ml Ethanol und trocknet im Vakuum. Der so erhaltene Feststoff wird zweimal aus DMF umkristallisiert. Ausbeute: 16.9 g (76 mmol) 76 %. Reinheit: ca. 99 % ig nach ¹H-NMR. Eine weitere Reinigung kann durch wiederholte Umkristallisation oder Chromatographie sowie durch fraktionierte Sublimation im Vakuum erfolgen.

Analog werden folgende Verbindungen dargestellt:

| Bsp | Edukt | Boronsäure | Produkt Variante | Ausbeute |
|---|---|---|---|---|
| S11 | 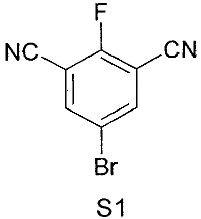 | 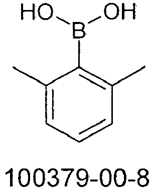 | 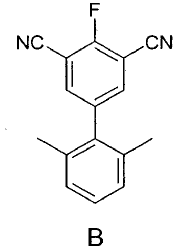 | 76 % |
| S12 | 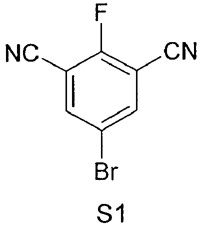 | 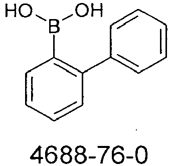 | 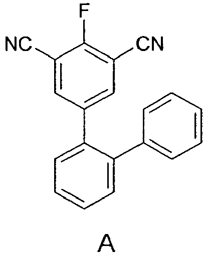 | 64 % |
| S13 | 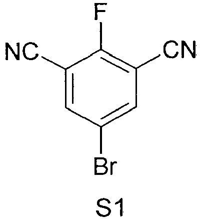 | 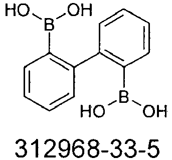 | 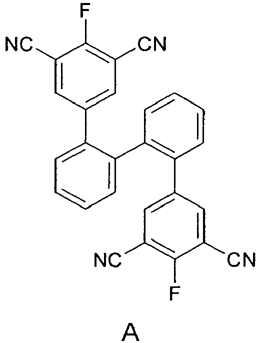 | 48 % |
| | | Einsatz von 45 mmol | | |
| S14 | 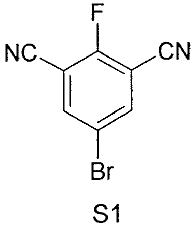 | 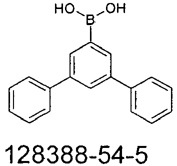 | 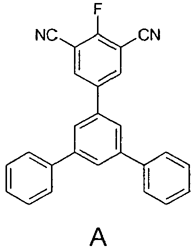 | 78 % |
| S15 | 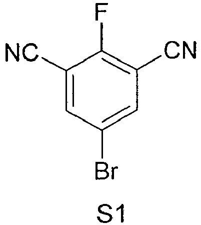 | 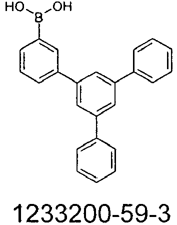 | 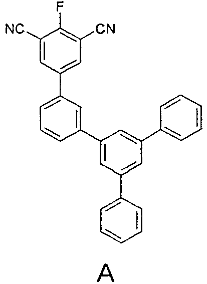 | 63 % |
| S16 | 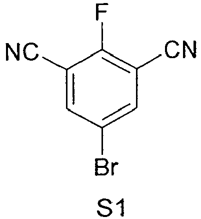 | 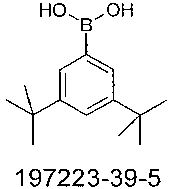 | 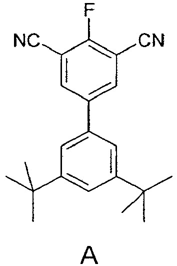 | 75 % |
| S17 | 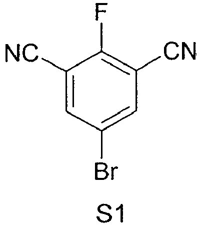 | 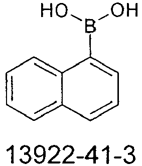 | 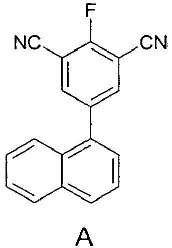 | 71 % |
| S18 | 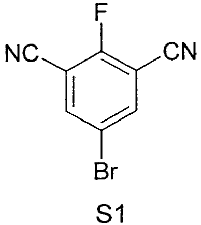 | 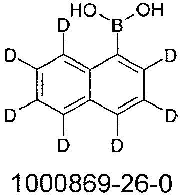 | 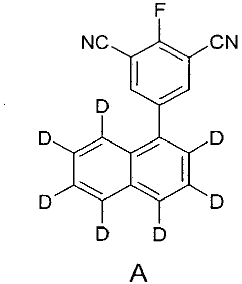 | 70 % |
| S19 | 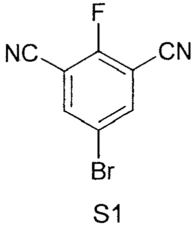 | 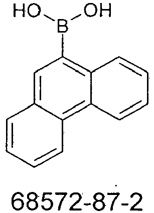 | 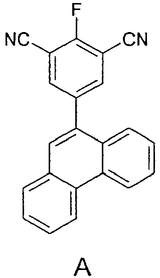 | 73 % |
| S20 | 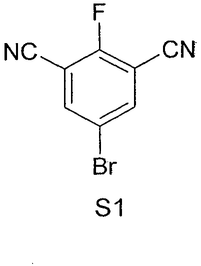 | 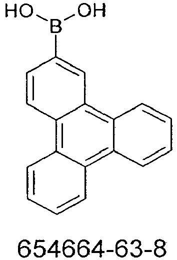 | 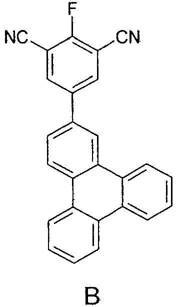 | 56 % |
| S21 | 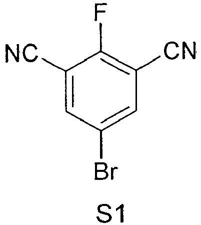 | 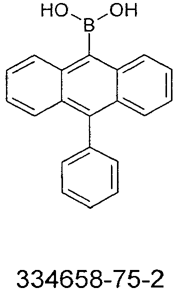 | 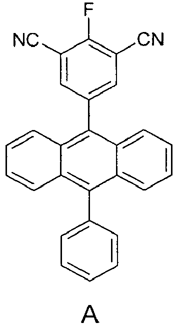 | 58 % |
| S22 | 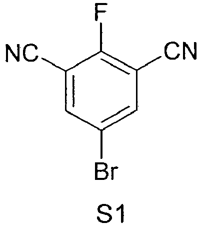 | 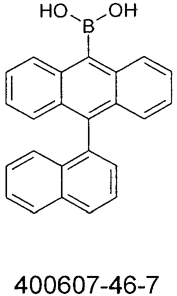 | 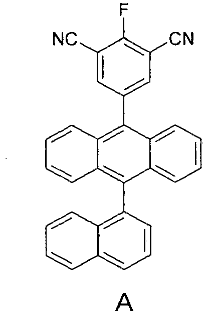 | 53 % |
| S23 | 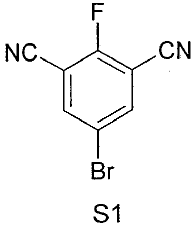 | 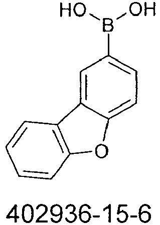 | 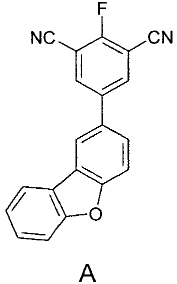 | 77 % |
| S24 | 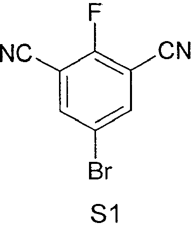 | 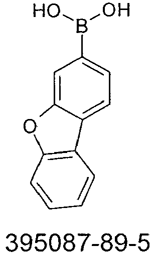 | 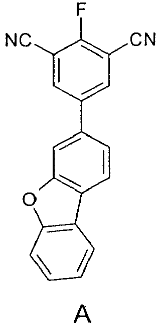 | 80 % |
| S25 | 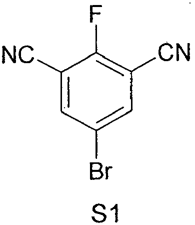 | 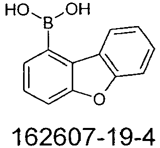 | 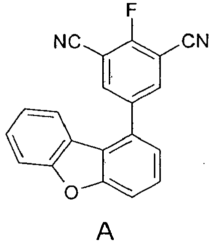 | 76 % |
| S26 | 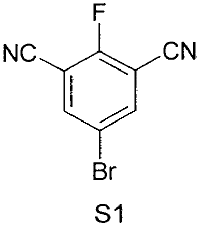 | 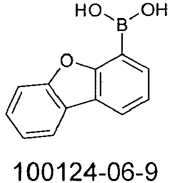 | 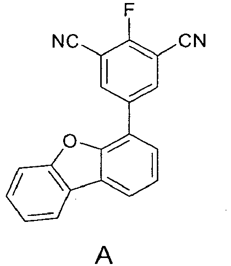 | 69 % |
| S27 | 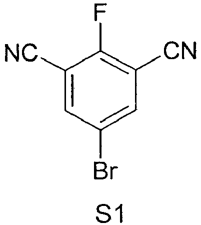 | 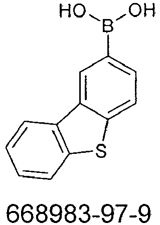 | 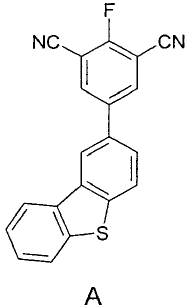 | 83 % |
| S28 | 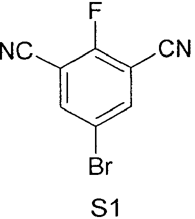 | 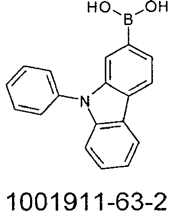 | 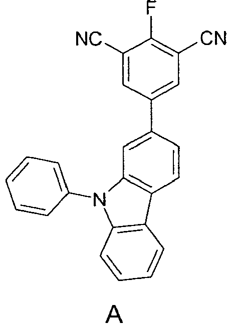 | 81 % |
| S29 | 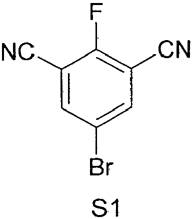 | 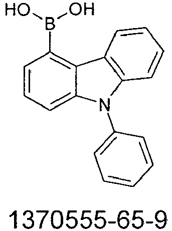 | 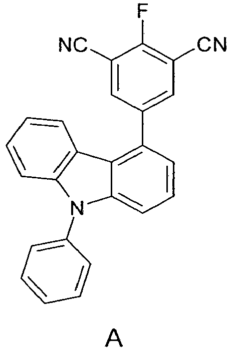 | 70 % |
| S30 | 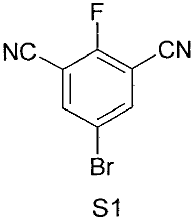 | 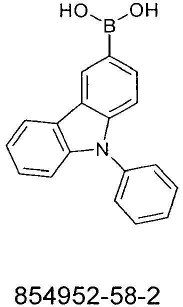 | 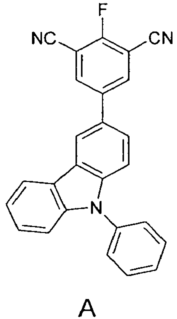 | 74 % |
| S31 | 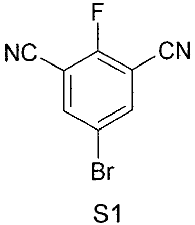 | 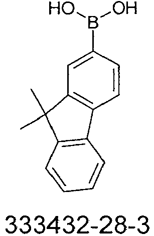 | 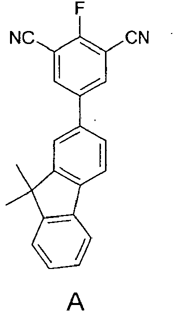 | 80 % |
| S32 | 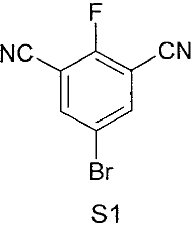 | 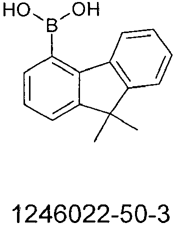 | 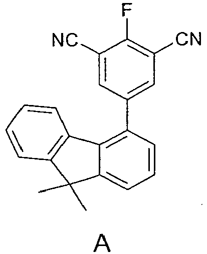 | 69 % |
| S33 | 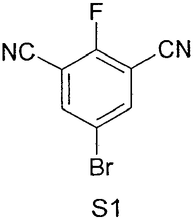 | 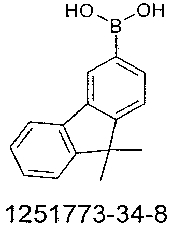 | 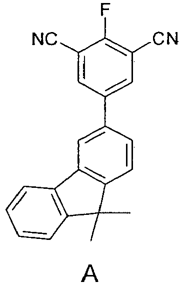 | 77 % |
| S34 | 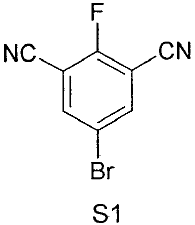 | 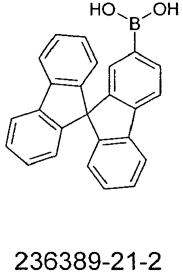 | 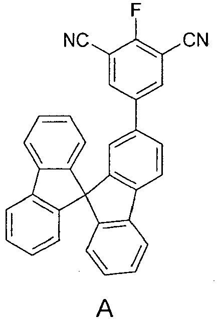 | 79 % |
| S35 | 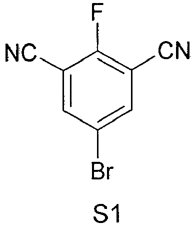 | 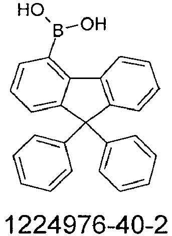 | 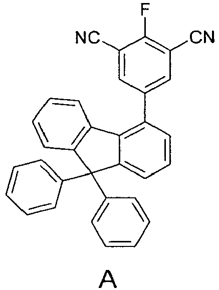 | 72 % |
| S36 | 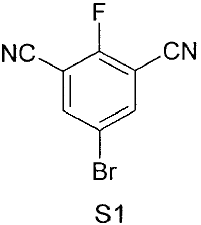 | 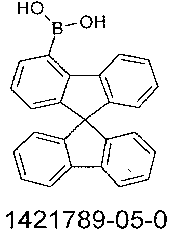 | 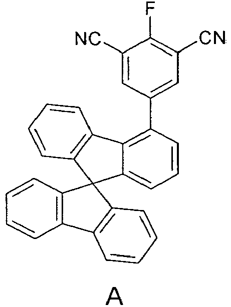 | 71 % |
| S37 | 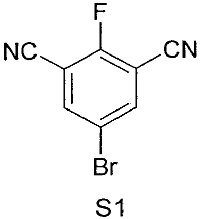 | 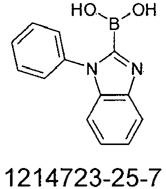 | 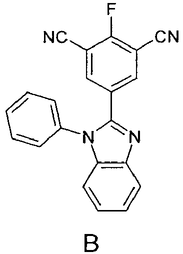 | 66 % |
| S38 | 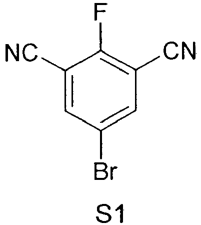 | 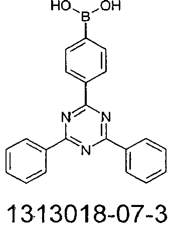 | 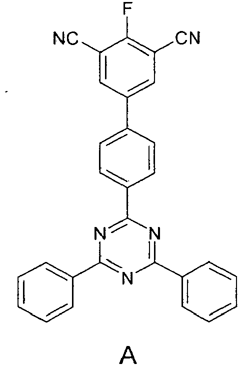 | 78 % |
| S39 | 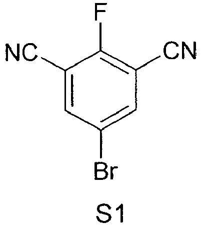 | 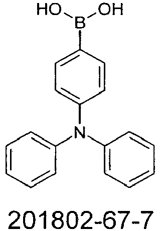 | 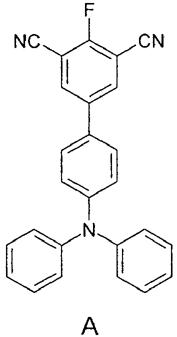 | 67 % |
| S40 | 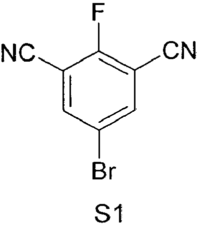 | 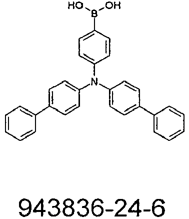 | 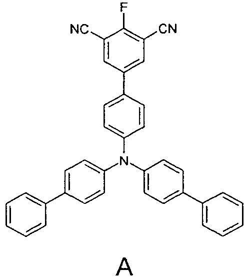 | 69 % |
| S41 | 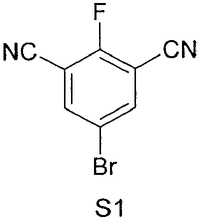 | 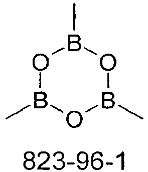 | 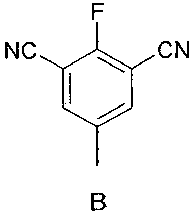 | 38 % |
| S42 | 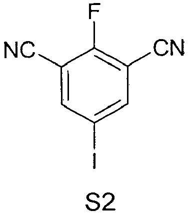 | 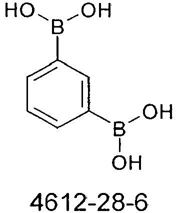 | 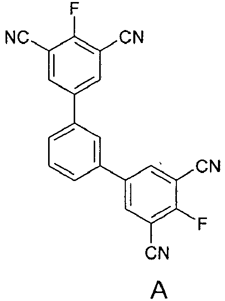 | 46 % |
| | | Einsatz von 45 mmol | | |
| S43 | 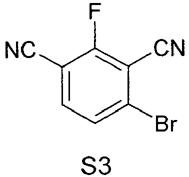 | 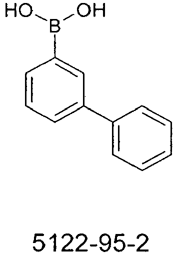 | 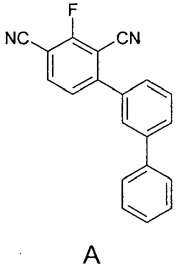 | 55 % |
| S44 | 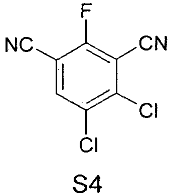 Einsatz von 50 mmol | 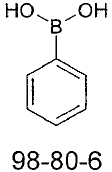 | 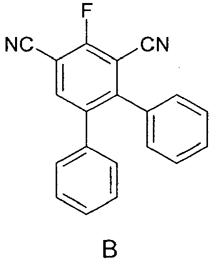 | 34 % |
| S45 | 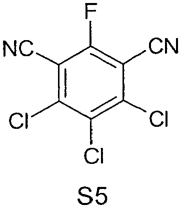 | 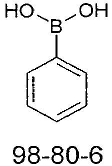 | 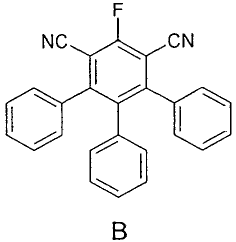 | 23 % |
| | Einsatz von 30 mmol | | | |
| S46 | 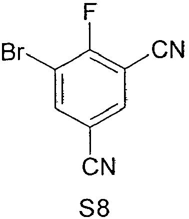 | 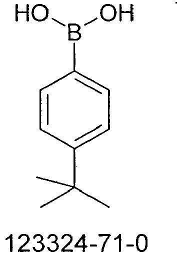 | 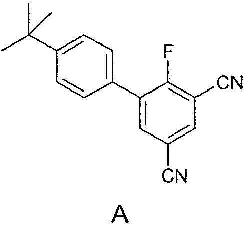 | 64 % |
| S47 | 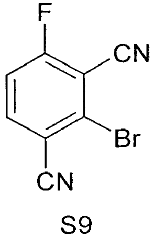 | 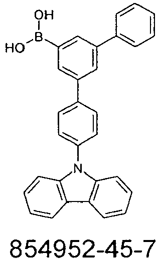 | 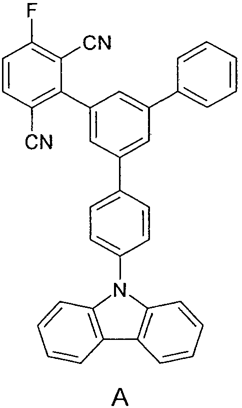 | 75 % |
| S48 | 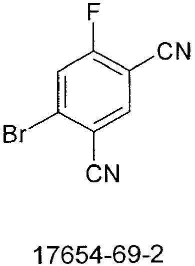 | 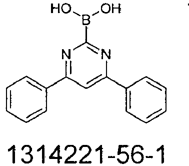 | 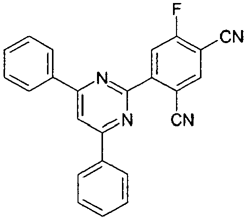 | 73 % |

### S49: 5-(N-Diphenylamino)-2-fluor-isophthalsäurenitril, S49

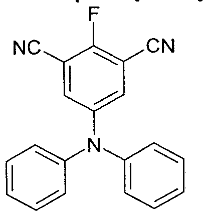

Ein Gemisch aus 22.5 g (100 mmol) 5-Brom-2-fluor-isophthalsäurenitril, S1, 20.3 g (120 mmol) Diphenylamin, 11.5 g (120 mmol) Natrium-tert-butanolat, 405 mg (2 mmol) Tri-tert-butylphosphin, 224 mg (1 mmol) Palladium(II)aceat und 300 ml Toluol wird so lange unter Rückfluss erhitzt, bis das 5-Brom-2-fluor-isophthalsäurenitril, S1 verbraucht ist (ca. 8 h). Nach Erkalten gibt man 100 ml Wasser zu, saugt den ausgefallenen Feststoff ab, wäscht diesen dreimal mit je 50 ml Ethanol und trocknet in Vakuum. Der so erhaltene Feststoff wird in 300 ml Dichlormethan gelöst, die Lösung wird über ein Celite-Bett filtriert, um Salze und Palladium zu entfernen. Der nach Einengen des Filtrats erhaltene Rückstand wird zweimal aus Dioxan / EtOH umkristallisiert. Ausbeute: 22.9 g (73 mmol) 73 %. Reinheit: ca. 99 % ig nach ¹H-NMR. Eine weitere Reinigung kann durch wiederholte Umkristallisation oder Chromatographie sowie durch fraktionierte Sublimation im Vakuum erfolgen.

Analog können folgende Verbindungen dargestellt werden:

| Bsp | Edukt | Amin | Produkt | Ausbeute |
|---|---|---|---|---|
| S50 | 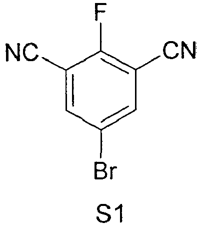 | 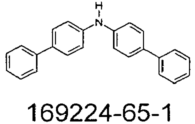 | 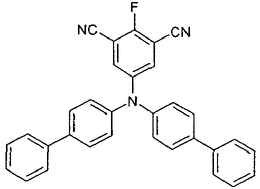 | 68 % |
| S51 | 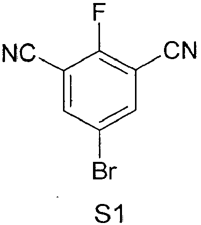 | 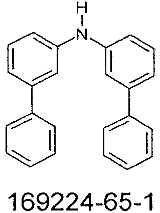 | 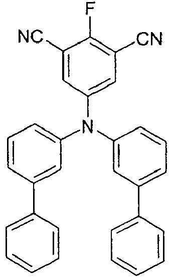 | 53 % |
| S52 | 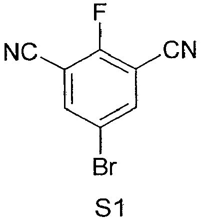 | 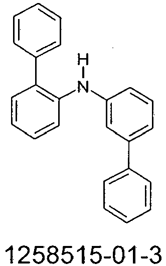 | 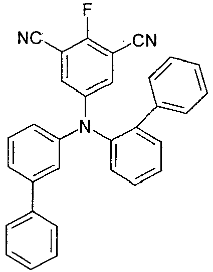 | 49 % |
| S53 | 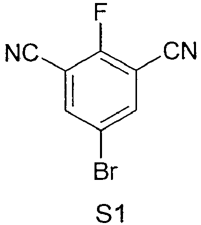 | 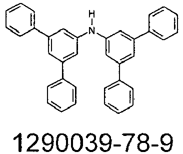 | 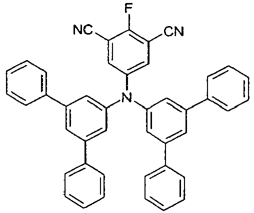 | 70 % |
| S54 | 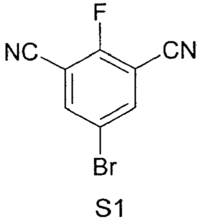 | 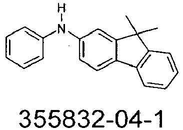 | 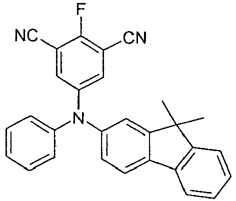 | 65 % |
| S55 | 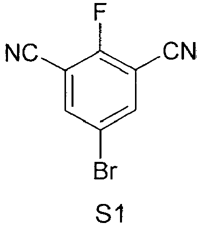 | 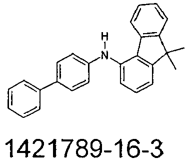 | 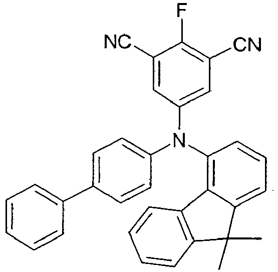 | 49 % |
| S56 | 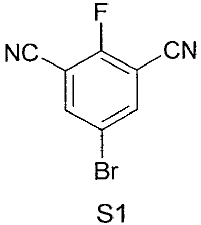 | 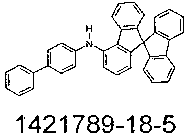 | 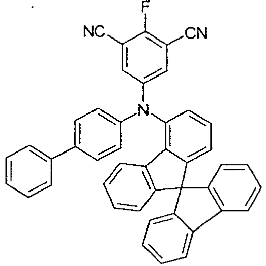 | 43 % |
| S57 | 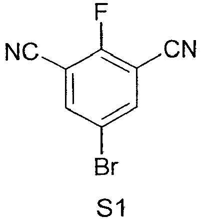 | 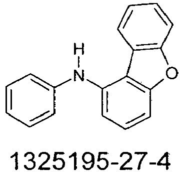 | 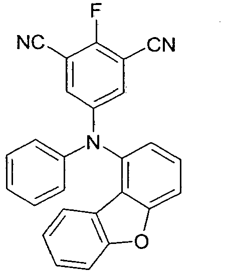 | 46 % |
| S58 | 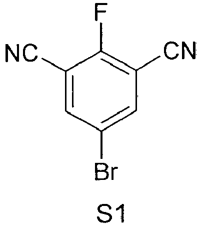 | 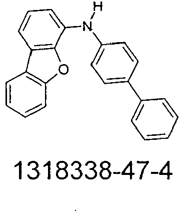 | 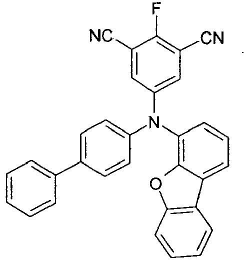 | 45 % |
| S59 | 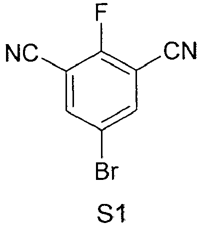 | 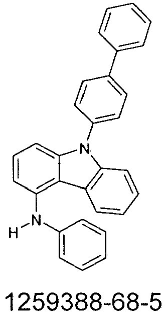 | 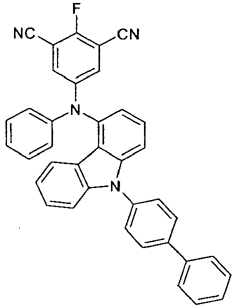 | 50 % |
| S60 | 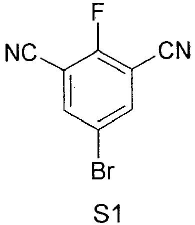 | 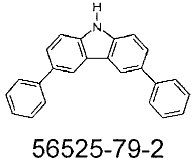 | 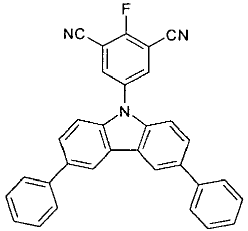 | 32 % |
| S61 | 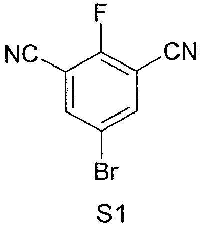 | 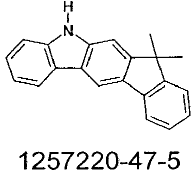 | 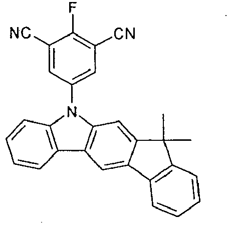 | 28 % |
| S62 | 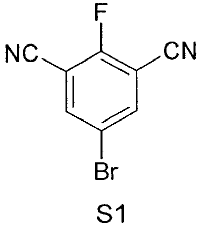 | 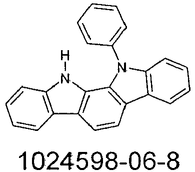 | 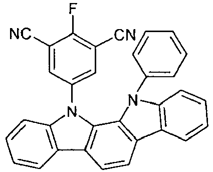 | 21 % |
| S63 | 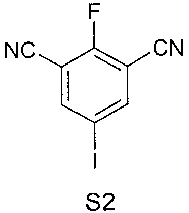 | 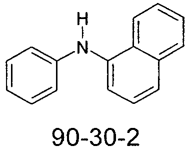 | 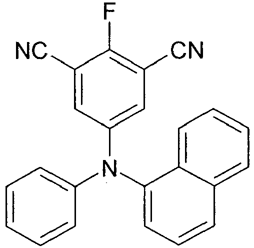 | 67 % |
| S64 | 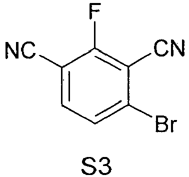 | 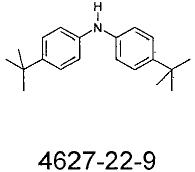 | 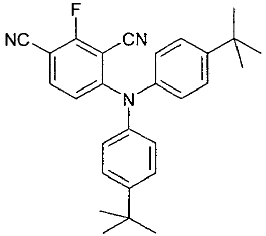 | 69 % |
| S65 | 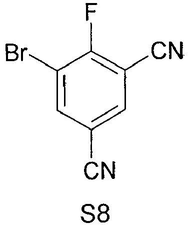 | 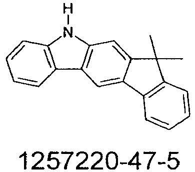 | 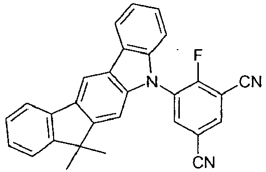 | |
| S66 | 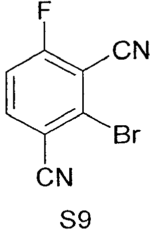 | 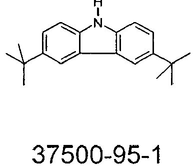 | 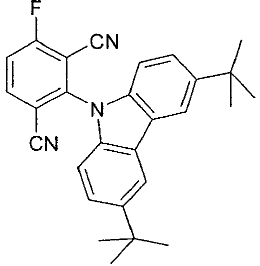 | |
| S67 | 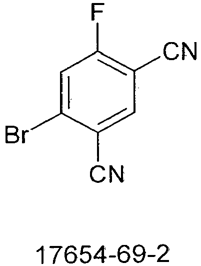 | 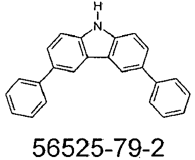 | 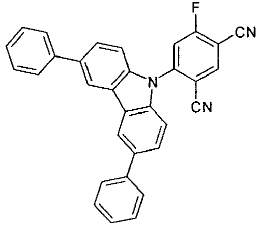 | |

### Beispiel S68: Umsetzung der Synthesebausteine in einer nukleophilen aromatischen Substitution

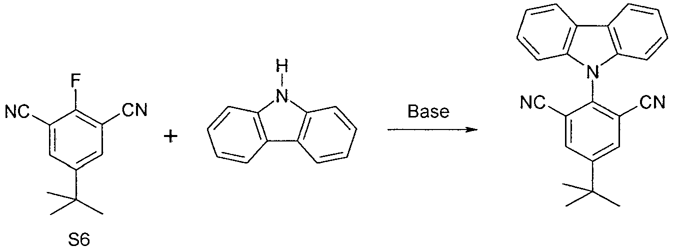

### Variante A:

Eine gut gerührte Suspension von 4.8 g (120 mmol) Natriumhydrid, 60 Gew.-% ige Dispersion in Mineralöl, in 200 ml THF wird unter Eiskühlung bei ca. + 10 °C portionsweise mit 20.1 g (120 mmol) Carbazol [51555-21-6] versetzt - Vorsicht Wasserstoffentwicklung! Schäumen! Nach beendeter Zugabe wird die Mischung 30 min. nachgerührt und dann portionsweise unter Eiskühlung so mit 20.2 g (100 mmol) mit 2-Fluor-5-tert-butyl-iso-phthalsäurenitril, S6 versetzt, dass die Temperatur + 20 °C nicht übersteigt. Nach beendeter Zugabe rührt man 2 h bei + 10 °C nach, entfernt dann das Kältebad, lässt auf 20 - 25 °C erwärmen rührt 2 h nach und erwärmt dann noch 12 h auf 40 °C. Nach Abkühlen auf Raumtemperatur quencht man durch Zutropfen von 30 ml MeOH und engt die Reaktionsmischung dann im Vakuum fast bis zur Trockene ein. Der Rückstand wird zweimal mit einem Gemisch aus 100 ml Methanol und 100 ml Wasser und dann einmal mit 200 ml Methanol heiß ausgerührt. Die Reinigung erfolgt durch fünfmalige Umkristallisation aus DMF und zweimalige fraktionierte Sublimation (p ca. 1 x 10⁻⁵ mbar, T ca. 180 °C). Ausbeute: 23.1 g (66 mmol) 66 %. Reinheit: 99.9 n. HPLC.

### Variante B:

Durchführung analog zu Variante A, jedoch wird das Carbazol in THF vorgelegt und dann tropfenweise mit 48 ml (120 mmol) n-BuLi, 2.5 molar in n-Hexan versetzt. Ausbeute: 20.6 g (59 mmol) 59 %. Reinheit: 99.9 n. HPLC.

### Variante C:

Eine gut gerührte Suspension von 20.1 g (120 mmol) Carbazol [51555-21-6], 20.2 g (100 mmol) mit 2-Fluor-5-tert-butyl-iso-phthalsäurenitril, S6, 31.8 g (150 mmol) Trikaliumphosphat, wasserfrei und 100 g Glaskugeln wird in 300 ml Dimethylacetamid 16 h bei 160 °C gerührt. Nach Erkalten gibt man 500 ml Wasser zu, saugt vom ausgefallenen Feststoff ab, wäscht diesen zweimal mit je 100 ml Wasser, zweimal mit je 100 ml Methanol und trocknet dann im Vakuum. Weitere Reinigung analoge Variante A. Ausbeute: 22.0 g (63 mmol) 63 %. Reinheit: 99.9 n. HPLC.

Analog können folgende Verbindungen dargestellt werden:

| Bsp | Edukt | Produkt Variante | Ausbeute |
|---|---|---|---|
| S69 | 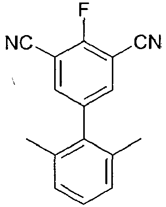 | 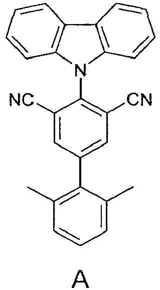 | 71 % |
| S70 | 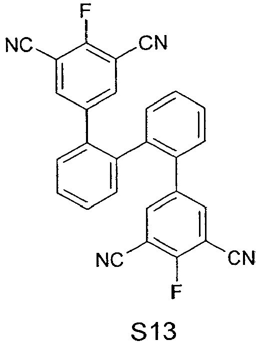 | 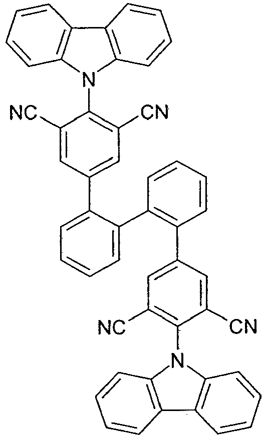 | 55 % |
| | Einsatz von 50 mmol | | |
| S71 | 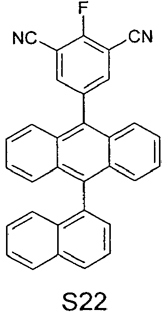 | 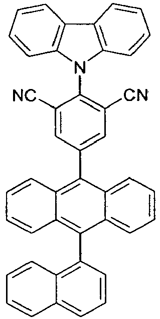 | 69 % |
| S72 | 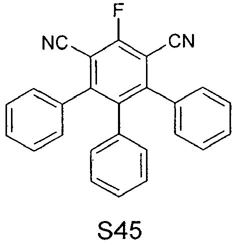 | 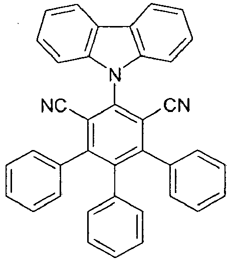 | 65 % |
| S73 | 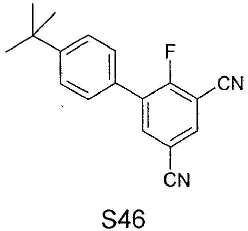 | 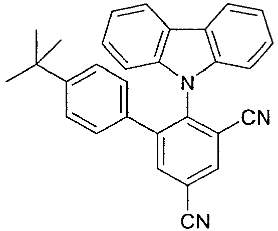 | 70 % |
| S74 | 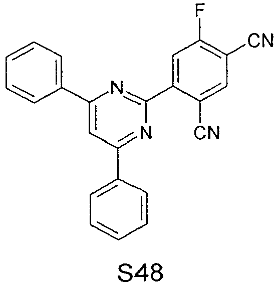 | 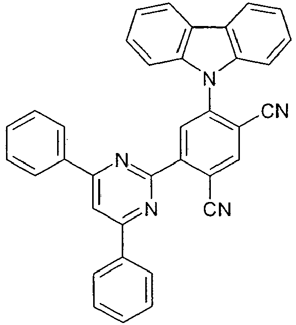 | 66 % |
| S75 | 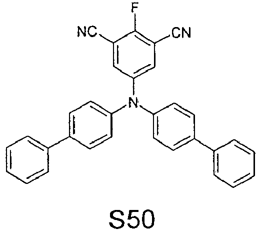 | 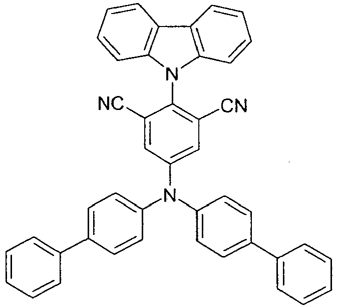 | 54 % |

### Beispiel S76: Palladium-katalysiere Cyanierung von Chlor-fluor-Aromaten

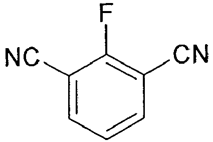

Durchführung analog M. Shevlin, Tetrahedron Letters, 2010, 51, 4833.

### Katalysator-Lösung:

Eine Lösung von 2.73 g (5.7 mmol) X-Phos und 643 mg (2.9 mmol) Palladium(II)acetat in 60 ml N,N-Dimethylacetamid wird mit 3 ml einer Mischung von 2.81 g konzentrierter Schwefelsäure in 28 ml N,N-Dimethylacetamid versetzt und 30 min. bei 80 °C gerührt, wobei eine dunkelbraune Lösung entsteht.

Eine gut gerührte Mischung aus 23.6 g (143 mmol) 1,3-Dichlor-2-fluorbenzol [2268-05-5], 20.2 g (172 mmol) Zink(II)cyanid, 750 mg (11.5 mmol) Zink-Staub und 280 ml N,N-Dimethylacetamid wird mit 1 ml der Katalysator-Lösung versetzt und 3 h bei 120 °C gerührt. Nach beendeter Reaktion wird das Lösungsmittel am Vakuum weitgehend entfernt, der Rückstand wird in 500 ml Ethylacetat aufgenommen, verbliebebne Salze werden abfiltiriert, das Filtrat wird dreimal mit 300 ml Wasser und einmal mit 300 ml Kochsalzlösung gewaschen und über Magnesiumsulfat getrocknet. Der nach Entfernen des Lösungsmittels verbliebene Rückstand wird zweimal aus Cyclohexan umkristallisiert. Ausbeute: 15.8 g (109 mmol) 76 %. Reinheit: ca. 97 % ig n. ¹H-NMR.

Analog kann die folgende Verbindung dargestellt werden:

| Bsp | Edukt | Produkt | Ausbeute |
|---|---|---|---|
| S77 | 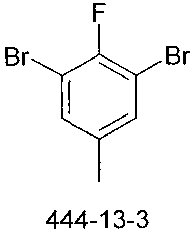 | 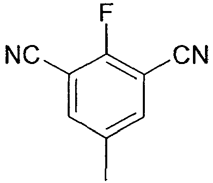 | 68 % |

## Patentansprüche

1. Verbindung gemäß Formel (1), Formel (2) oder Formel (3), 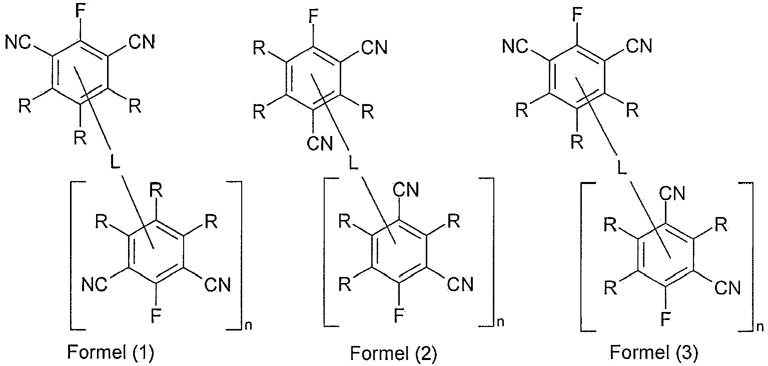 wobei für die verwendeten Symbole und Indizes gilt:
L ist eine Einfachbindung oder eine bi-, tri-, tetra-, penta- oder hexavalente Gruppe;
R ist bei jedem Auftreten gleich oder verschieden ausgewählt aus der Gruppe bestehend aus H, D, Cl, Br, I, NAr₂, N(R¹)₂, wobei R¹ ungleich H ist, C(=O)Ar, C(=O)R¹, BR¹, P(=O)Ar₂, PAr₂, OAr, S(=O)Ar, S(=O)₂Ar, Si(R¹)₃, einer geradkettigen Alkyl- oder Alkoxygruppe mit 1 bis 40 C-Atomen oder einer verzweigten oder cyclischen Alkyl- oder Alkoxygruppe mit 3 bis 40 C-Atomen oder einer Alkenyl- oder Alkinylgruppe mit 2 bis 40 C-Atomen, die jeweils mit einem oder mehreren Resten R¹ substituiert sein kann, wobei jeweils eine oder mehrere nicht-benachbarte CH₂-Gruppen durch R¹C=CR¹, C≡C, Si(R¹)₂, C=NR¹, P(=O)(R¹), NR¹, O, S oder CONR¹ ersetzt sein können und wobei ein oder mehrere H-Atome durch D, F, Cl, Br oder I ersetzt sein können, oder einem aromatischen oder heteroaromatischen Ringsystem mit 5 bis 60 aromatischen Ring-atomen, das jeweils mit einem oder mehreren Resten R¹ substituiert sein kann; dabei können optional zwei oder mehr benachbarte Substituenten R ein monocyclisches oder polycyclisches, aliphatisches, aromatisches oder heteroaromatisches Ringsystem bilden, das mit einem oder mehreren Resten R¹ substituiert sein kann;
Ar ist bei jedem Auftreten gleich oder verschieden ein aromatisches oder heteroaromatisches Ringsystem mit 5-30 aromatischen Ring-atomen, das mit einem oder mehreren Resten R¹ substituiert sein kann; dabei können zwei Reste Ar, welche an dasselbe N-Atom oder P-Atom binden, auch durch eine Einfachbindung oder eine Brücke, ausgewählt aus N(R¹), C(R¹)₂, O, S oder BR¹, miteinander verbrückt sein;
R¹ ist bei jedem Auftreten gleich oder verschieden ausgewählt aus der Gruppe bestehend aus H, D, F, Cl, Br, I, CN, NO₂, N(R²)₂, P(=O)(R²)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C≡C, Si(R²)₂, C=NR², P(=O)(R²), SO, SO₂, NR², O, S oder CONR² ersetzt sein können und wobei ein oder mehrere H-Atome durch D, F, Cl, Br, I oder CN ersetzt sein können, einem aromatischen oder heteroaromatischen Ringsystem mit 5 bis 60 aromatischen Ringatomen, das jeweils mit einem oder mehreren Resten R² substituiert sein kann; dabei können optional zwei oder mehr benachbarte Substituenten R¹ ein monocyclisches oder polycyclisches, aliphatisches Ringsystem bilden, das mit einem oder mehreren Resten R² substituiert sein kann;
R² ist bei jedem Auftreten gleich oder verschieden ausgewählt aus der Gruppe bestehend aus H, D, F, CN, einem aliphatischen Kohlenwasserstoffrest mit 1 bis 20 C-Atomen, einem aromatischen oder heteroaromatischen Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R² miteinander ein mono- oder polycyclisches, aliphatisches Ringsystem bilden können;
n ist 1, 2, 3, 4 oder 5, mit der Maßgabe, dass L jeweils anstelle eines Restes R an den Benzolgrundkörper bindet und die entsprechende Gruppe R somit nicht vorhanden ist.

2. Verbindung nach Anspruch 1, **dadurch gekennzeichnet, dass** n = 1, 2 oder 3 ist, bevorzugt 1 oder 2.

3. Verbindung nach Anspruch 1 oder 2, **dadurch gekennzeichnet, dass** L für eine Einfachbindung, NR, BR, P(=O)R, eine geradkettige Alkylen- oder Alkylidengruppe mit 1 bis 10 C-Atomen oder eine verzweigte oder cyclische Alkylen- oder Alkylidengruppe mit 3 bis 10 C-Atomen, die mit jeweils einem oder mehreren Resten R substituiert sein kann, wobei eine oder mehrere nicht benachbarte CH₂-Gruppen durch -RC=CR-, -C≡C-, Si(R)₂, C=O, -O-, -S- oder -CONR- ersetzt sein können und wobei ein oder mehrere H-Atome durch D oder F ersetzt sein können, oder ein aromatisches oder heteroaromatisches Ringsystem mit 5 bis 24 aromatischen Ringatomen, welches mit einem oder mehreren Resten R substituiert sein kann, steht.

4. Verbindung nach einem oder mehreren der Ansprüche 1 bis 3, ausgewählt aus den Verbindungen der Formeln (1f) bis (1h), (2h) bis (2l) und (3a) bis (3f), 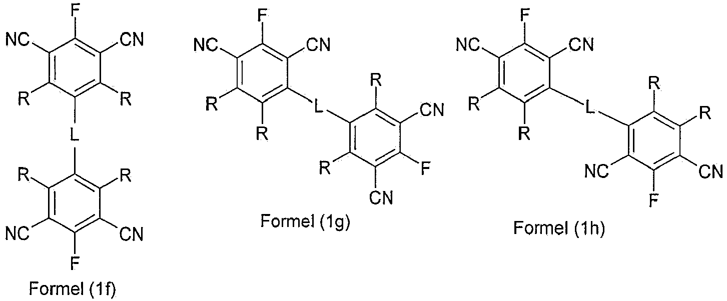 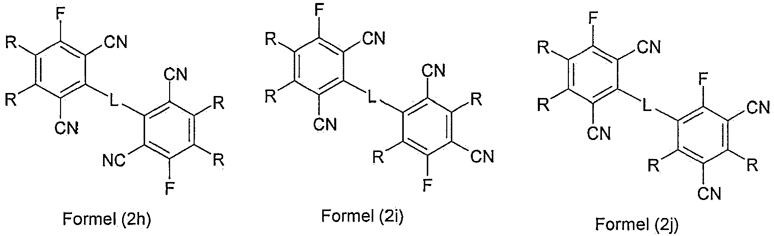 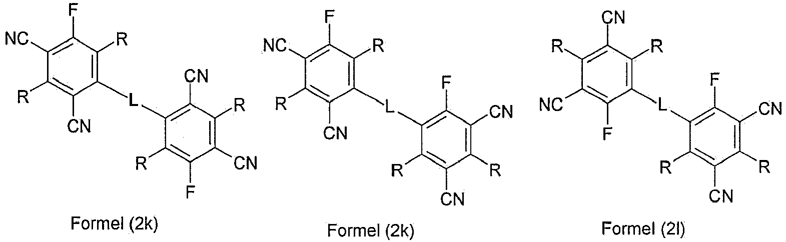 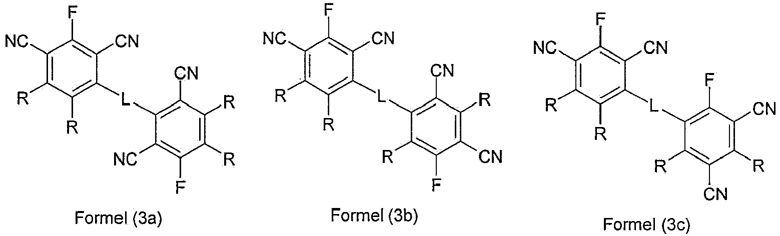 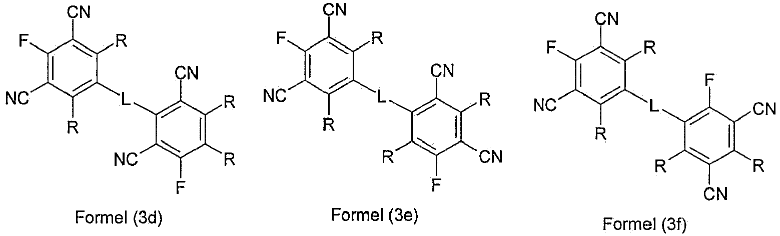 wobei die verwendeten Symbole die in Anspruch 1 genannten Bedeutungen aufweist.

5. Verbindung nach einem oder mehreren der Ansprüche 1 bis 4, **dadurch gekennzeichnet, dass** zwei benachbarte Reste R miteinander einen Ring der Formel (4) bilden, 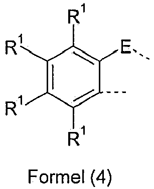 wobei R¹ die oben genannten Bedeutungen aufweist, die gestrichelten Bindungen die Verknüpfung der Gruppe mit dem Benzolgrundkörper andeuten und weiterhin gilt:
E ist ausgewählt aus der Gruppe bestehend aus C(R¹)₂, NR¹, O, S, BR¹ oder Si(R¹)₂.

6. Verbindung nach einem oder mehreren der Ansprüche 1 bis 5, **dadurch gekennzeichnet, dass** R bei jedem Auftreten gleich oder verschieden ausgewählt ist aus der Gruppe bestehend aus H, NAr₂, C(=O)Ar, C(=O)R¹, P(=O)Ar₂, PAr₂, Si(R¹)₃, einer geradkettigen Alkylgruppe mit 1 bis 10 C-Atomen oder einer verzweigten oder cyclischen Alkylgruppe mit 3 bis 10 C-Atomen oder einer Alkenylgruppe mit 2 bis 10 C-Atomen, die jeweils mit einem oder mehreren Resten R¹ substituiert sein kann, wobei jeweils eine oder mehrere nicht-benachbarte CH₂-Gruppen durch R¹C=CR¹ oder O ersetzt sein können und wobei ein oder mehrere H-Atome durch F ersetzt sein können, einem aromatischen oder heteroaromatischen Ringsystem mit 5 bis 40 aromatischen Ringatomen, das jeweils mit einem oder mehreren Resten R¹ substituiert sein kann; dabei können optional zwei oder mehr benachbarte Substituenten R ein monocyclisches oder polycyclisches, aliphatisches, aromatisches oder heteroaromatisches Ringsystem bilden, das mit einem oder mehreren Resten R¹ substituiert sein kann.

7. Verbindung nach einem oder mehreren der Ansprüche 1 bis 6, **dadurch gekennzeichnet, dass** keiner der Reste R, R¹ oder R² kondensierte Aryl- oder Heteroarylgruppen enthält, in denen Sechsringe direkt aneinander ankondensiert sind.

8. Verbindung nach einem oder mehreren der Ansprüche 1 bis 7, **dadurch gekennzeichnet, dass** mindestens ein Rest R ausgewählt ist aus der Gruppe bestehend aus einem aromatischen oder heteroaromatischen Ringsystem mit 5 bis 24 aromatischen Ringatome, welches durch einen oder mehrere Reste R¹ substituiert sein kann, insbesondere ausgewählt aus Benzol, Biphenyl, insbesondere ortho-, meta- oder para-Biphenyl, Terphenyl, insbesondere ortho-, meta-, para- oder verzweigtes Terphenyl, Quaterphenyl, insbesondere ortho-, meta- para- oder verzweigtes Quaterphenyl, 1-, 2-, 3- oder 4-Spirobifluoren, 1-, 2-, 3- oder 4-Fluoren, 1- oder 2-Naphthyl, Pyrrol, Furan, Thiophen, Indol, Benzofuran, Benzothiophen, Carbazol, Azacarbazol, Dibenzofuran, Dibenzothiophen, Pyridin, Pyrimidin, Pyrazin, Pyridazin, Triazin, Imidazol, Benzimidazol, Pyrazol, Thiazol, Oxazol, Oxadiazol, Triazol, Phenanthren, Triphenylen oder Kombinationen aus zwei oder drei dieser Gruppen, welche jeweils mit einem oder mehreren Resten R¹ substituiert sein können, oder dass mindestens ein Substituent R ausgewählt ist aus einer der Gruppen der Formeln (7) bis (18) 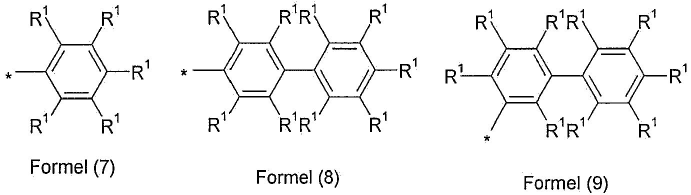 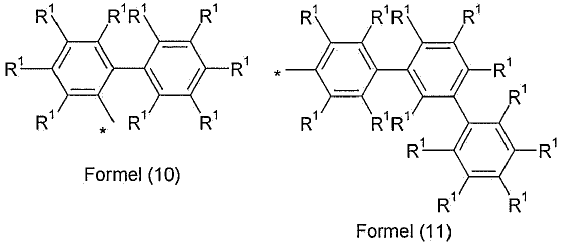 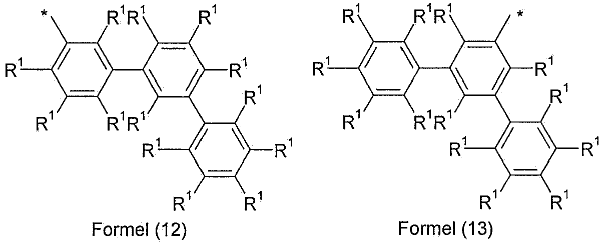 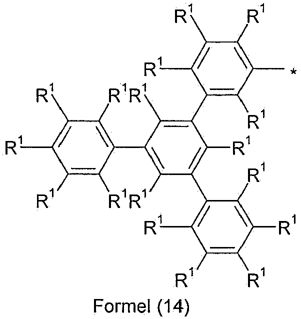 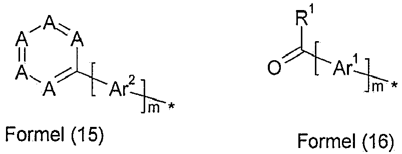 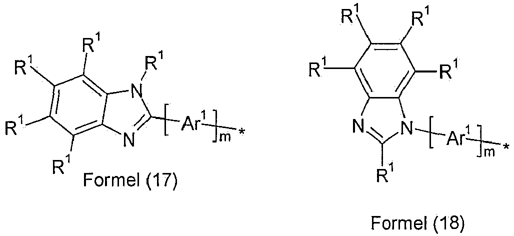 wobei die verwendeten Symbole die in Anspruch 1 genannten Bedeutungen haben, * die Position der Bindung der Gruppe andeutet und weiterhin gilt:
A ist bei jedem Auftreten gleich oder verschieden CR¹ oder N, mit der Maßgabe, dass eine, zwei oder drei Gruppen A für N stehen;
Ar¹ ist gleich oder verschieden bei jedem Auftreten ein bivalentes aromatisches oder heteroaromatisches Ringsystem mit 5 bis 16 aromatischen Ringatomen, welches durch einen oder mehrere Reste R¹ substituiert sein kann;
m ist 0 oder 1.

9. Verbindung nach einem oder mehreren der Ansprüche 1 bis 8, **dadurch gekennzeichnet, dass** mindestens ein Rest R ausgewählt ist aus der Gruppe bestehend aus Triarylaminderivaten, Carbazolderivaten, Indenocarbazolderivaten, Indolocarbazolderivaten, Azacarbazolderivaten, Indolderivaten, Furanderivaten, Benzofuranderivaten, Dibenzofuranderivaten, Thiophenderivaten, Benzothiophenderivaten oder Dibenzothiophenderivaten, welche jeweils durch einen oder mehrere Reste R¹ substituiert sein können, oder mindestens ein Substituent R steht für -NAr₂, wobei die beiden Gruppen Ar auch durch eine Gruppe ausgewählt aus NR¹, O, S, C(R¹)₂, Si(R¹)₂ oder BR¹ miteinander verbrückt sein können, insbesondere ausgewählt aus den Gruppen der Formeln (19) bis (33), 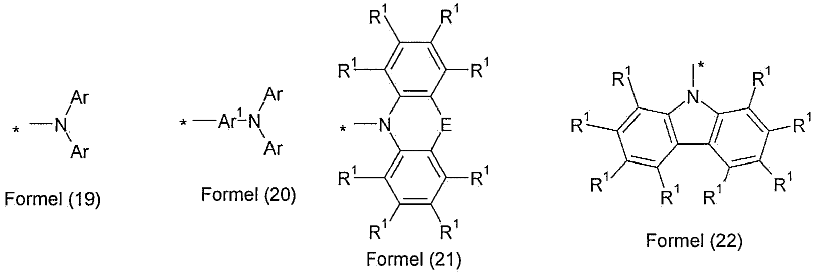 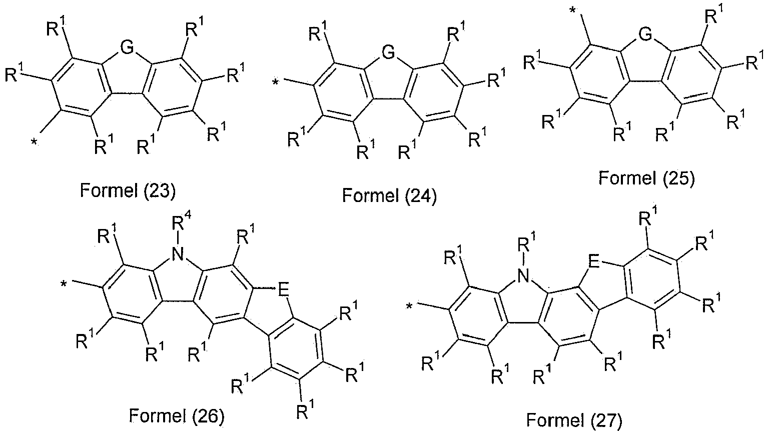 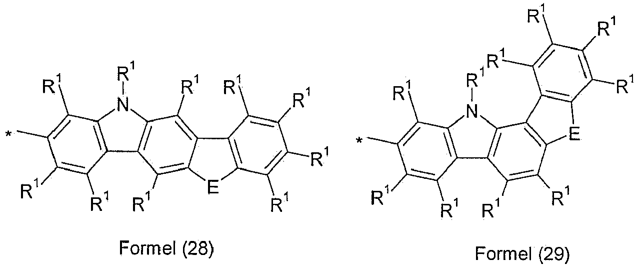 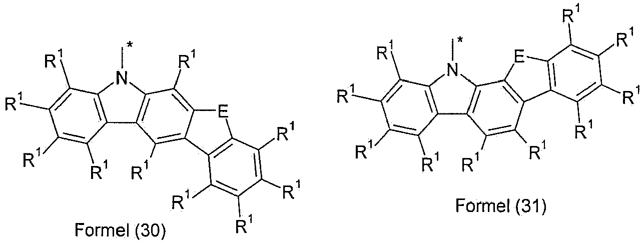 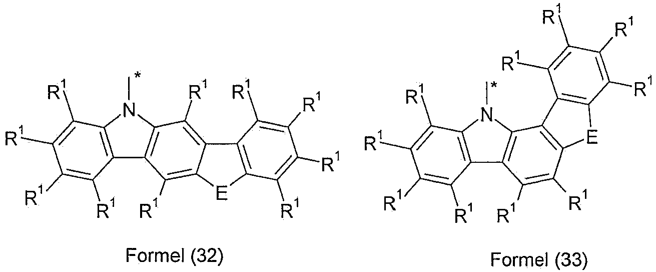 wobei die verwendeten Symbole die in den Ansprüchen 1 und 5 genannten Bedeutungen aufweisen und weiterhin gilt:
G ist ausgewählt aus der Gruppe bestehend aus NR¹, O oder S.
wobei jeweils eine oder zwei Gruppen CR¹ durch N ersetzt sein können.

10. Verfahren zur Herstellung einer Verbindung nach einem oder mehreren der Ansprüche 1 bis 9, umfassend die Reaktionsschritte:
a) Oxidation eines Fluor-meta-Xylolderivats zur Carbonsäure; und
b) Umsetzung der Carbonsäurefunktionalitäten zu Nitrilgruppen.

11. Verfahren zur Herstellung einer Verbindung nach einem oder mehreren der Ansprüche 1 bis 9 durch Umsetzung eines Fluor-metadihalogenbenzol-Derivats mit Zn(CN)₂ und Zn in Gegenwart eines Katalysators.

12. Verwendung einer Verbindung nach einem oder mehreren der Ansprüche 1 bis 9 als Edukt in einer nukleophilen aromatischen Substitutionsreaktion.

13. Verfahren zur Herstellung einer Verbindung gemäß Formel (34) bzw. Formel (35) bzw. Formel (36), 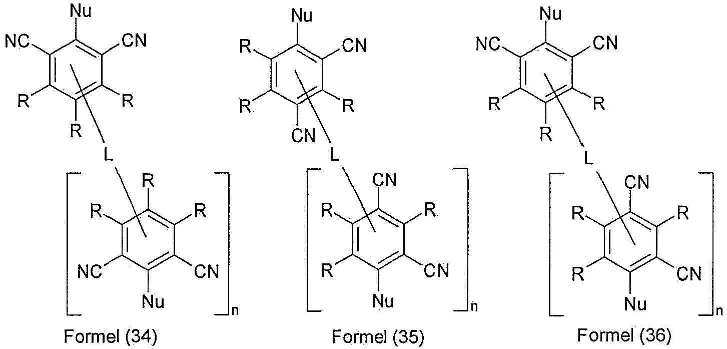 wobei Nu für eine nukleophile Gruppe steht und die anderen verwendeten Symbole die in Anspruch 1 genannten Bedeutungen aufweisen,
durch Umsetzung einer Verbindung nach einem oder mehreren der Ansprüche 1 bis 9 mit einem Nukleophil.

## Claims

1. Compound of the formula (1), formula (2) or formula (3), 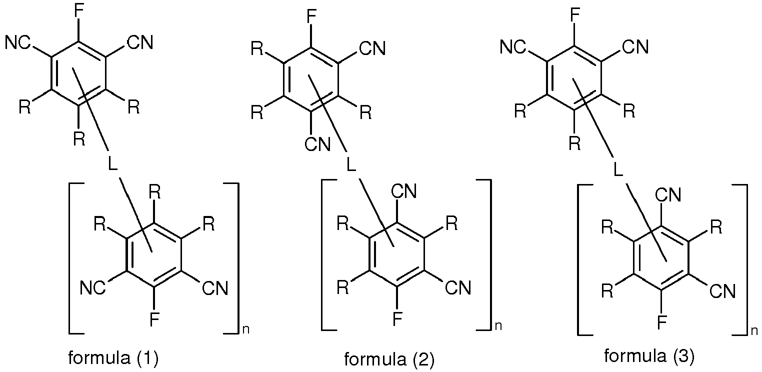 where the following applies to the symbols and indices used:
L is a single bond or a di-, tri-, tetra-, penta- or hexavalent group;
R is selected on each occurrence, identically or differently, from the group consisting of H, D, Cl, Br, I, NAr₂, N(R¹)₂, where R¹ is not equal to H, C(=O)Ar, C(=O)R¹, BR¹, P(=O)Ar₂, PAr₂, OAr, S(=O)Ar, S(=O)₂Ar, Si(R¹)₃, a straight-chain alkyl or alkoxy group having 1 to 40 C atoms or a branched or cyclic alkyl or alkoxy group having 3 to 40 C atoms or an alkenyl or alkynyl group having 2 to 40 C atoms, which may in each case be substituted by one or more radicals R¹, where in each case one or more non-adjacent CH₂ groups may be replaced by R¹C=CR¹, C≡C, Si(R¹)₂, C=NR¹, P(=O)(R¹), NR¹, O, S or CONR¹ and where one or more H atoms may be replaced by D, F, Cl, Br or I, or an aromatic or hetero-aromatic ring system having 5 to 60 aromatic ring atoms, which may in each case be substituted by one or more radicals R¹; two or more adjacent substituents R may optionally form a monocyclic or polycyclic, aliphatic, aromatic or heteroaromatic ring system, which may be substituted by one or more radicals R¹;
Ar is on each occurrence, identically or differently, an aromatic or heteroaromatic ring system having 5-30 aromatic ring atoms, which may be substituted by one or more radicals R¹; two radicals Ar which are bonded to the same N atom or P atom may also be bridged to one another by a single bond or a bridge selected from N(R¹), C(R¹)₂, O, S or BR¹;
R¹ is selected on each occurrence, identically or differently, from the group consisting of H, D, F, Cl, Br, I, CN, NO₂, N(R²)₂, P(=O)(R²)₂, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals R², where one or more non-adjacent CH₂ groups may be replaced by R²C=CR², C≡C, Si(R²)₂, C=NR², P(=O)(R²), SO, SO₂, NR², O, S or CONR² and where one or more H atoms may be replaced by D, F, Cl, Br, I or CN, an aromatic or heteroaromatic ring system having 5 to 60 aromatic ring atoms, which may in each case be substituted by one or more radicals R²; two or more adjacent substituents R¹ may optionally form a monocyclic or polycyclic, aliphatic ring system, which may be substituted by one or more radicals R²;
R² is selected on each occurrence, identically or differently, from the group consisting of H, D, F, CN, an aliphatic hydrocarbon radical having 1 to 20 C atoms, an aromatic or heteroaromatic ring system having 5 to 30 aromatic ring atoms, in which one or more H atoms may be replaced by D, F, Cl, Br, I or CN, where two or more adjacent substituents R² may form a mono- or polycyclic, aliphatic ring system with one another;
n is 0, 1, 2, 3, 4 or 5, with the proviso that L is in each case bonded to the benzene skeleton instead of a radical R and the corresponding group R is thus not present.

2. Compound according to Claim 1, **characterised in that** n = 1, 2 or 3, preferably 1 or 2.

3. Compound according to Claim 1 or 2, **characterised in that** L stands for a single bond, NR, BR, P(=O)R, a straight-chain alkylene or alkylidene group having 1 to 10 C atoms or a branched or cyclic alkylene or alkylidene group having 3 to 10 C atoms, which may be substituted by in each case one or more radicals R, where one or more non-adjacent CH₂ groups may be replaced by -RC=CR-, -C≡C-, Si(R)₂, C=O, -O-, -S- or -CONR- and where one or more H atoms may be replaced by D or F, or an aromatic or heteroaromatic ring system having 5 to 24 aromatic ring atoms, which may be substituted by one or more radicals R.

4. Compound according to one or more of Claims 1 to 3, selected from the compounds of the formulae (1f) to (1h), (2h) to (2l) and (3a) to (3f), 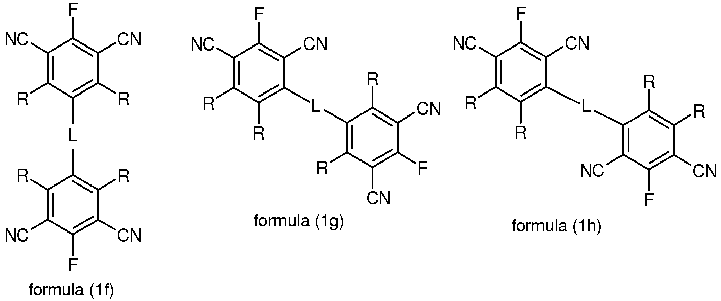 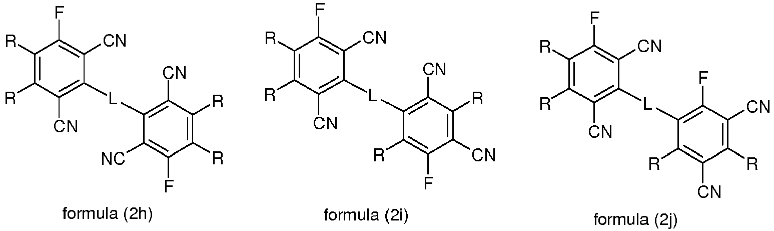 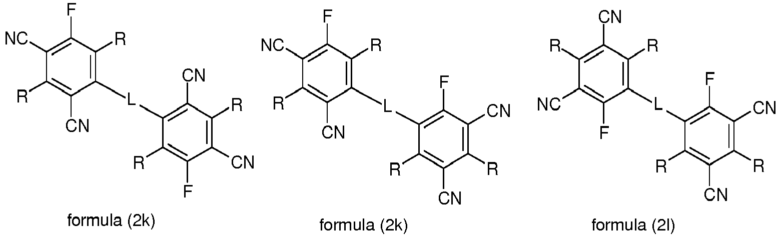 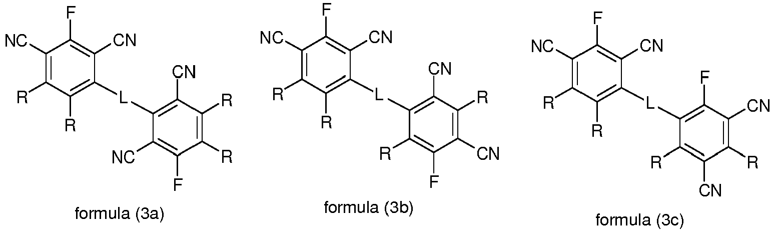 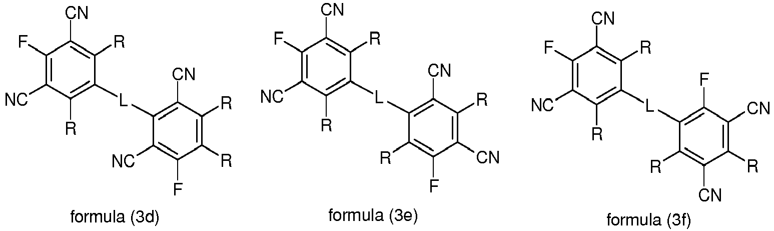 where the symbols used have the meanings given in Claim 1.

5. Compound according to one or more of Claims 1 to 4, **characterised in that** two adjacent radicals R form a ring of the formula (4) with one another, 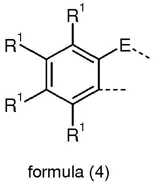 where R¹ has the meanings given above, the dashed bonds indicate the linking of the group to the benzene skeleton and furthermore:
E is selected from the group consisting of C(R¹)₂, NR¹, O, S, BR¹ or Si(R¹)₂.

6. Compound according to one or more of Claims 1 to 5, **characterised in that** R is selected on each occurrence, identically or differently, from the group consisting of H, NAr₂, C(=O)Ar, C(=O)R¹, P(=O)Ar₂, PAr₂, Si(R¹)₃, a straight-chain alkyl group having 1 to 10 C atoms or a branched or cyclic alkyl group having 3 to 10 C atoms or an alkenyl group having 2 to 10 C atoms, which may in each case be substituted by one or more radicals R¹, where in each case one or more non-adjacent CH₂ groups may be replaced by R¹C=CR¹ or O and where one or more H atoms may be replaced by F, an aromatic or heteroaromatic ring system having 5 to 40 aromatic ring atoms, which may in each case be substituted by one or more radicals R¹; two or more adjacent substituents R may optionally form a monocyclic or polycyclic, aliphatic, aromatic or heteroaromatic ring system, which may be substituted by one or more radicals R¹.

7. Compound according to one or more of Claims 1 to 6, **characterised in that** none of the radicals R, R¹ or R² contains condensed aryl or heteroaryl groups in which six-membered rings are condensed directly onto one another.

8. Compound according to one or more of Claims 1 to 7, **characterised in that** at least one radical R is selected from the group consisting of an aromatic or heteroaromatic ring system having 5 to 24 aromatic ring atoms, which may be substituted by one or more radicals R¹, in particular selected from benzene, biphenyl, in particular ortho-, meta- or para-biphenyl, terphenyl, in particular ortho-, meta-, para- or branched terphenyl, quaterphenyl, in particular ortho-, meta-, para- or branched quaterphenyl, 1-, 2-, 3- or 4-spirobifluorene, 1-, 2-, 3- or 4-fluorene, 1- or 2-naphthyl, pyrrole, furan, thiophene, indole, benzofuran, benzothiophene, carbazole, azacarbazole, dibenzofuran, dibenzothiophene, pyridine, pyrimidine, pyrazine, pyridazine, triazine, imidazole, benzimidazole, pyrazole, thiazole, oxazole, oxadiazole, triazole, phenanthrene, triphenylene or combinations of two or three of these groups, which may in each case be substituted by one or more radicals R¹, or **in that** at least one substituent R is selected from one of the groups of the formulae (7) to (18) 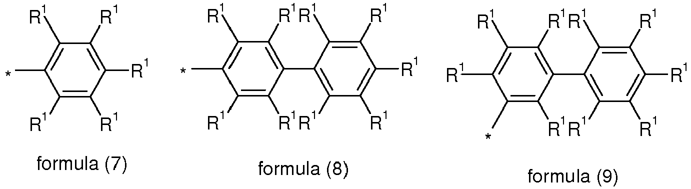 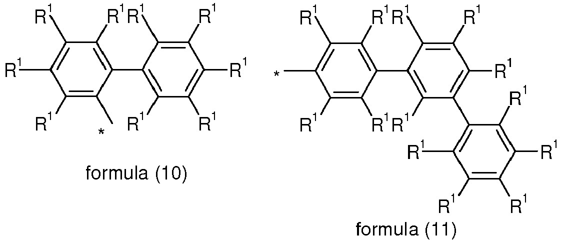 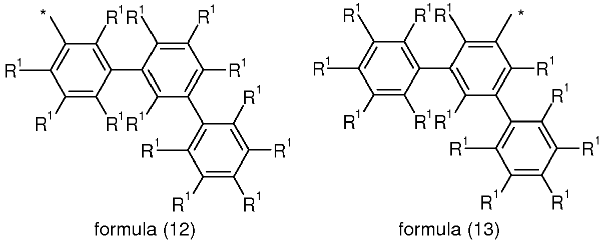 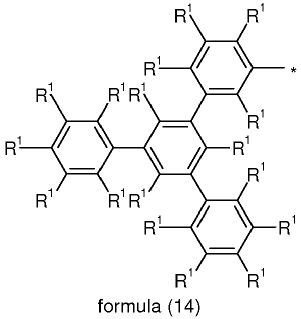 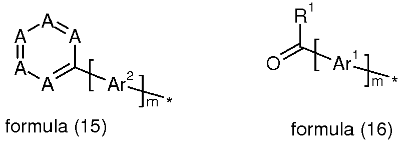 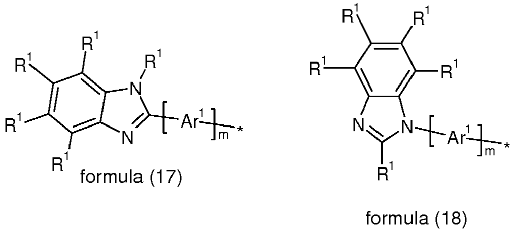 where the symbols used have the meanings given in Claim 1, * indicates the position of the bonding of the group and furthermore:
A is on each occurrence, identically or differently, CR¹ or N, with the proviso that one, two or three groups A stand for N;
Ar¹ is, identically or differently on each occurrence, a divalent aromatic or heteroaromatic ring system having 5 to 16 aromatic ring atoms, which may be substituted by one or more radicals R¹;
m is 0 or 1.

9. Compound according to one or more of Claims 1 to 8, **characterised in that** at least one radical R is selected from the group consisting of triarylamine derivatives, carbazole derivatives, indenocarbazole derivatives, indolocarbazole derivatives, azacarbazole derivatives, indole derivatives, furan derivatives, benzofuran derivatives, dibenzofuran derivatives, thiophene derivatives, benzothiophene derivatives or dibenzothiophene derivatives, which may in each case be substituted by one or more radicals R¹, or at least one substituent R stands for -NAr₂, where the two groups Ar may also be bridged to one another by a group selected from NR¹, O, S, C(R¹)₂, Si(R¹)₂ or BR¹, in particular selected from the groups of the formulae (19) to (33), 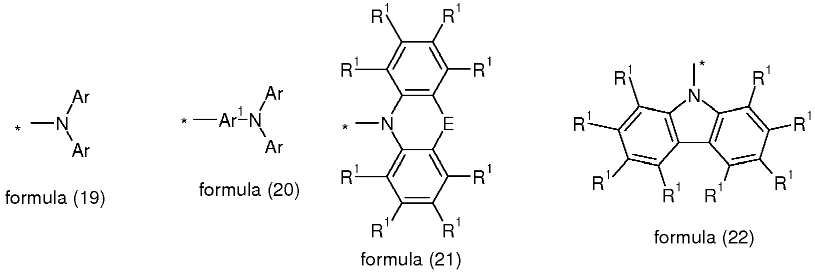 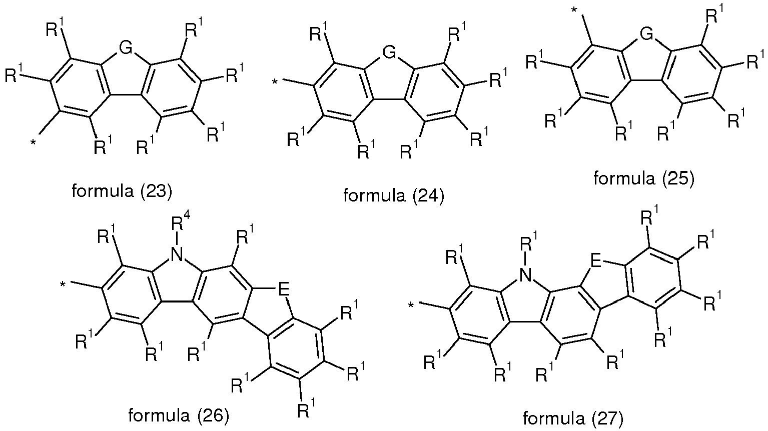 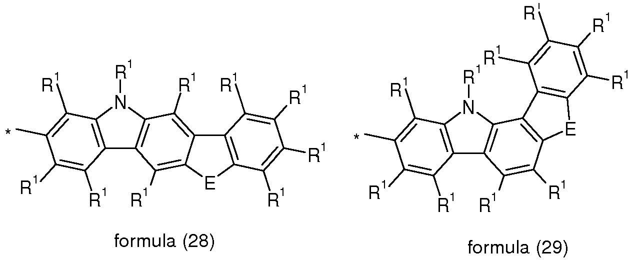 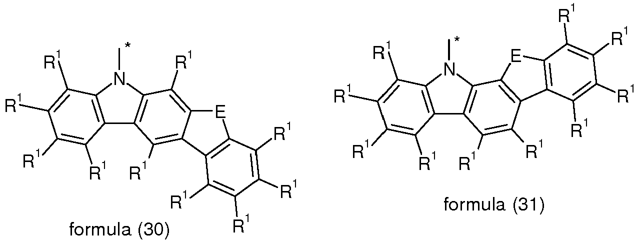 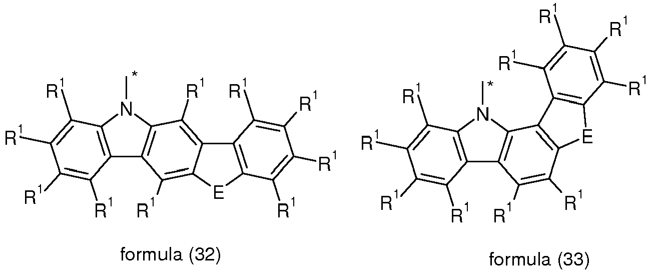 where the symbols used have the meanings given in Claims 1 and 5 and furthermore:
G is selected from the group consisting of NR¹, O or S,
where in each case one or two groups CR¹ may be replaced by N.

10. Process for the preparation of a compound according to one or more of Claims 1 to 9, comprising the reaction steps:
a) oxidation of a fluoro-meta-xylene derivative to give the carboxylic acid; and
b) conversion of the carboxylic acid functionalities into nitrile groups.

11. Process for the preparation of a compound according to one or more of Claims 1 to 9 by reaction of a fluoro-meta-dihalobenzene derivative with Zn(CN)₂ and Zn in the presence of a catalyst.

12. Use of a compound according to one or more of Claims 1 to 9 as starting material in a nucleophilic aromatic substitution reaction.

13. Process for the preparation of a compound of the formula (34) or formula (35) or formula (36), 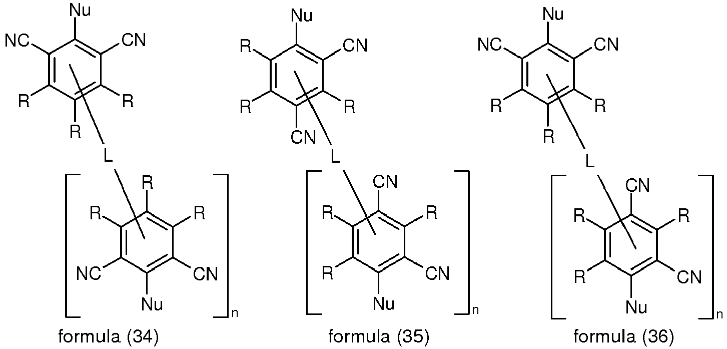 where Nu stands for a nucleophilic group and the other symbols used have the meanings given in Claim 1,
by reaction of a compound according to one or more of Claims 1 to 9 with a nucleophile.

## Revendications

1. Composé de la formule (1), de la formule (2) ou de la formule (3), 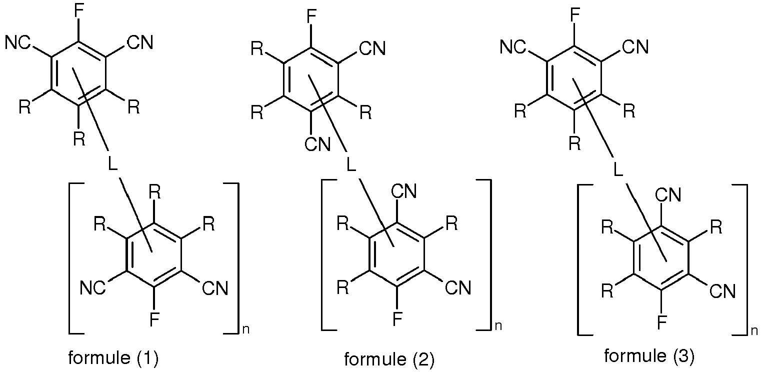 dans lesquelles ce qui suit s'applique aux symboles et aux indices qui sont utilisés :
L est une liaison simple ou un groupe di-, tri-, tétra-, penta- ou hexavalent ;
R est sélectionné pour chaque occurrence, de manière identique ou différente, parmi le groupe qui est constitué par H, D, Cl, Br, I, NAr₂, N(R¹)₂, où R¹ n'est pas égal à H, C(=O)Ar, C(=O)R¹, BR¹, P(=O)Ar₂, PAr₂, OAr, S(=O)Ar, S(=O)₂Ar, Si(R¹)₃, un groupe alkyle ou alcoxy en chaîne droite qui comporte de 1 à 40 atome(s) de C ou un groupe alkyle ou alcoxy ramifié ou cyclique qui comporte de 3 à 40 atomes de C ou un groupe alkényle ou alkynyle qui comporte de 2 à 40 atome(s) de C, lequel peut dans chaque cas être substitué par un radical ou par plusieurs radicaux R¹, où dans chaque cas, un ou plusieurs groupe(s) CH₂ non adjacents peut/ peuvent être remplacé(s) par R¹C=CR¹, C≡C, Si(R¹)₂, C=NR¹, P(=O)(R¹), NR¹, O, S ou CONR¹ et où un ou plusieurs atome(s) de H peut/peuvent être remplacé(s) par D, F, Cl, Br ou I, ou un système de cycle aromatique ou hétéroaromatique qui comporte de 5 à 60 atomes de cycle aromatique, lequel peut dans chaque cas être substitué par un radical ou par plusieurs radicaux R¹ ; deux substituants R adjacents ou plus peuvent en option former un système de cycle aliphatique, aromatique ou hétéroaromatique monocyclique ou polycyclique, lequel peut être substitué par un radical ou par plusieurs radicaux R¹;
Ar est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux R¹; deux radicaux Ar qui sont liés au même atome de N ou au même atome de P peuvent également être pontés l'un à l'autre par une liaison simple ou par un pont qui est sélectionné parmi N(R¹), C(R¹)₂, O, S ou BR¹;
R¹ est sélectionné pour chaque occurrence, de manière identique ou différente, parmi le groupe qui est constitué par H, D, F, Cl, Br, I, CN, NO₂, N(R²)₂, P(=O)(R²)₂, un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C ou un groupe alkényle ou alkynyle qui comporte de 2 à 40 atomes de C, lequel peut dans chaque cas être substitué par un radical ou par plusieurs radicaux R², où un ou plusieurs groupe(s) CH₂ non adjacents peut/peuvent être remplacé(s) par R²C=CR², C≡C, Si(R²)₂, C=NR², P(=O)(R²), SO, SO₂, NR², O, S ou CONR² et où un ou plusieurs atome(s) de H peut/ peuvent être remplacé(s) par D, F, Cl, Br, I ou CN, un système de cycle aromatique ou hétéroaromatique qui comporte de 5 à 60 atomes de cycle aromatique, lequel peut dans chaque cas être substitué par un radical ou par plusieurs radicaux R²; deux substituants R¹ adjacents ou plus peuvent en option former un système de cycle aliphatique monocyclique ou polycyclique, lequel peut être substitué par un radical ou par plusieurs radicaux R²;
R² est sélectionné pour chaque occurrence, de manière identique ou différente, parmi le groupe qui est constitué par H, D, F, CN, un radical hydrocarbone aliphatique qui comporte de 1 à 20 atome(s) de C, un système de cycle aromatique ou hétéroaromatique qui comporte de 5 à 30 atomes de cycle aromatique, où un ou plusieurs atome(s) de H peut/peuvent être remplacé(s) par D, F, Cl, Br, I ou CN, où deux substituants R² adjacents ou plus peuvent former un système de cycle aliphatique mono- ou polycyclique l'un avec l'autre ou les uns avec les autres ;
n est 0, 1, 2, 3, 4 ou 5, étant entendu que L est dans chaque cas lié au squelette benzène en lieu et place d'un radical R et le groupe R correspondant est de ce fait non présent.

2. Composé selon la revendication 1, **caractérisé en ce que** n = 1, 2 ou 3, de préférence 1 ou 2.

3. Composé selon la revendication 1 ou 2, **caractérisé en ce que** L représente une liaison simple, NR, BR, P(=O)R, un groupe alkylène ou alkylidène en chaîne droite qui comporte de 1 à 10 atome(s) de C ou un groupe alkylène ou alkylidène ramifié ou cyclique qui comporte de 3 à 10 atomes de C, lequel peut être substitué par, dans chaque cas, un radical ou plusieurs radicaux R, où un ou plusieurs groupe(s) CH₂ non adjacents peut/peuvent être remplacé(s) par -RC=CR-, -C≡C-, Si(R)₂, C=O, -O-, -S- ou -CONR- et où un ou plusieurs atome(s) de H peut/ peuvent être remplacé(s) par D ou F, ou un système de cycle aromatique ou hétéroaromatique qui comporte de 5 à 24 atomes de cycle aromatique, lequel peut être substitué par un radical ou par plusieurs radicaux R.

4. Composé selon une ou plusieurs des revendications 1 à 3, sélectionné parmi les composés des formules (1f) à (1h), (2h) à (2l) et (3a) à (3f), 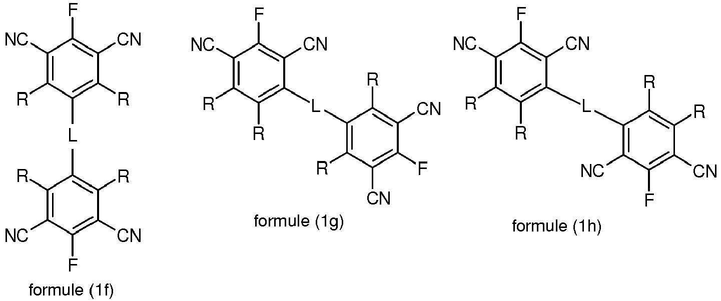 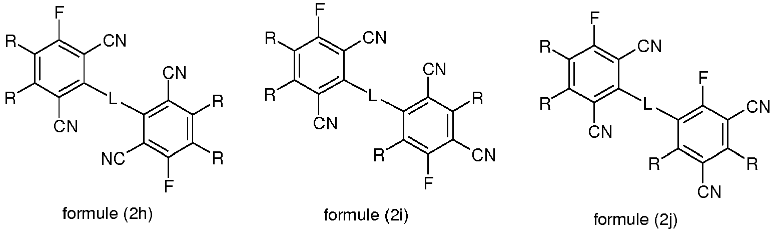 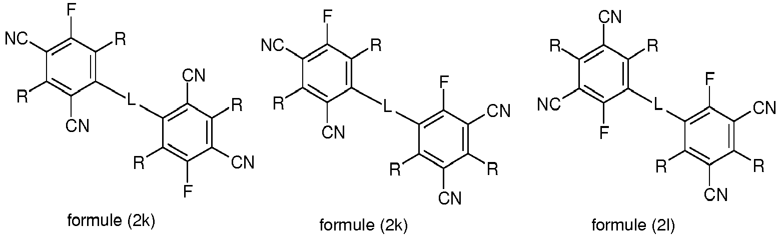 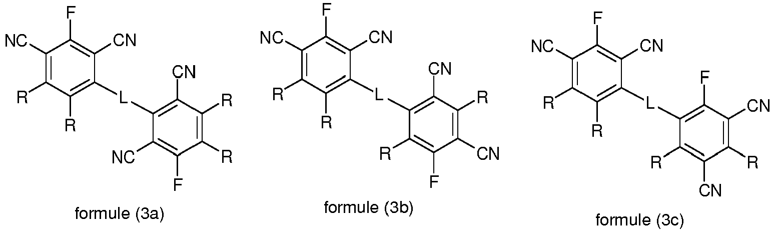 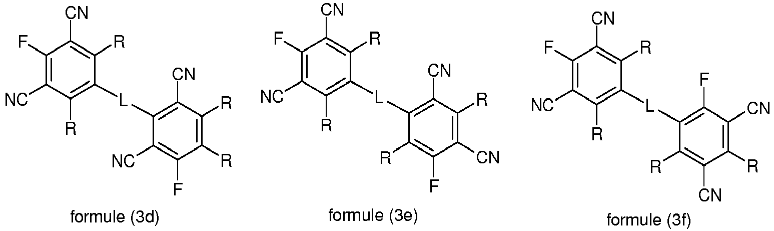 dans lesquelles les symboles qui sont utilisés présentent les significations qui ont été données selon la revendication 1.

5. Composé selon une ou plusieurs des revendications 1 à 4, **caractérisé en ce que** deux radicaux R adjacents forment un cycle de la formule (4) l'un avec l'autre, 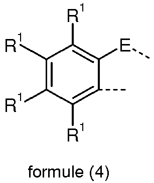 dans laquelle R¹ présente les significations qui ont été données ci-avant, les liens en pointillés indiquent la liaison du groupe sur le squelette benzène et en outre :
E est sélectionné parmi le groupe qui est constitué par C(R¹)₂, NR¹, O, S, BR¹ ou Si(R¹)₂.

6. Composé selon une ou plusieurs des revendications 1 à 5, **caractérisé en ce que** R est sélectionné pour chaque occurrence, de manière identique ou différente, parmi le groupe qui est constitué par H, NAr₂, C(=O)Ar, C(=O)R¹, P(=O)Ar₂, PAr₂, Si(R¹)₃, un groupe alkyle en chaîne droite qui comporte de 1 à 10 atome(s) de C ou un groupe alkyle ramifié ou cyclique qui comporte de 3 à 10 atomes de C ou un groupe alkényle qui comporte de 2 à 10 atomes de C, lequel peut dans chaque cas être substitué par un radical ou par plusieurs radicaux R¹, où, dans chaque cas, un ou plusieurs groupe(s) CH₂ non adjacents peut/peuvent être remplacé(s) par R¹C=CR¹ ou par O et où un ou plusieurs atome(s) de H peut/peuvent être remplacé(s) par F, un système de cycle aromatique ou hétéroaromatique qui comporte de 5 à 40 atomes de cycle aromatique, lequel peut dans chaque cas être substitué par un radical ou par plusieurs radicaux R¹; deux substituants R adjacents ou plus peuvent en option former un système de cycle aliphatique, aromatique ou hétéroaromatique monocyclique ou polycyclique, lequel peut être substitué par un radical ou par plusieurs radicaux R¹.

7. Composé selon une ou plusieurs des revendications 1 à 6, **caractérisé en ce qu'**aucun des radicaux R, R¹ ou R² ne contient des groupes aryle ou hétéroaryle condensés dans lesquels des cycles à six éléments sont condensés directement les uns sur les autres.

8. Composé selon une ou plusieurs des revendications 1 à 7, **caractérisé en ce qu'**au moins un radical R est sélectionné parmi le groupe qui est constitué par un système de cycle aromatique ou hétéroaromatique qui comporte de 5 à 24 atomes de cycle aromatique, lequel peut être substitué par un radical ou par plusieurs radicaux R¹, en particulier sélectionné parmi benzène, biphényle, en particulier ortho-, méta- ou para-biphényle, terphényle, en particulier ortho-, méta-, para- terphényle, quaterphényle ou les mêmes ramifiés, en particulier ortho-, méta-, para- quaterphényle, 1-, 2-, 3- ou 4-spirobifluorène, 1-, 2-, 3- ou 4-fluorène, 1- ou 2-naphtyle, pyrrole, furane, thiophène, indole, benzofurane, benzothiophène, carbazole, azacarbazole, dibenzofurane, dibenzothiophène, pyridine, pyrimidine, pyrazine, pyridazine, triazine, imidazole, benzimidazole, pyrazole, thiazole, oxazole, oxadiazole, triazole, phénanthrène, triphénylène ou les mêmes ramifiés ou des combinaisons de deux ou trois de ces groupes, lesquels peuvent dans chaque cas être substitués par un radical ou par plusieurs radicaux R¹, ou **en ce qu'**au moins un substituant R est sélectionné parmi l'un des groupes des formules (7) à (18) 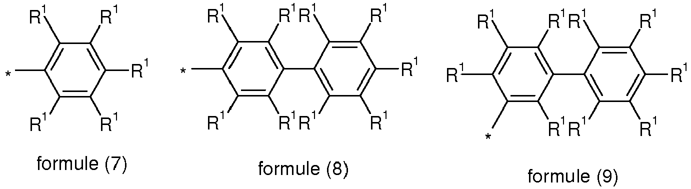 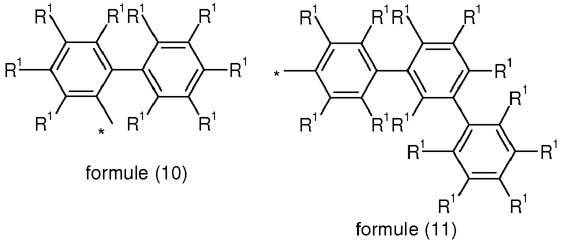 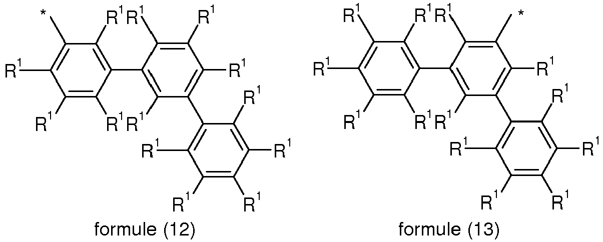 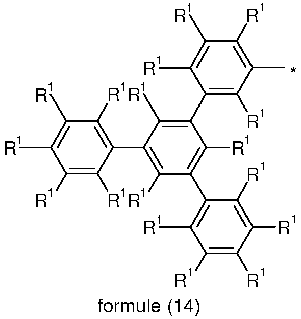 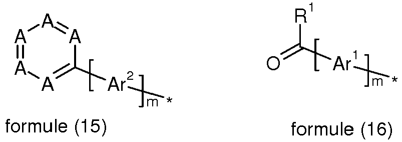 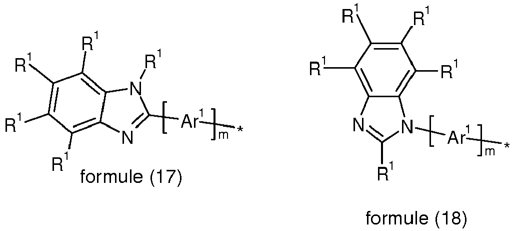 dans lesquelles les symboles qui sont utilisés présentent les significations qui ont été données selon la revendication 1, * indique la position de la liaison du groupe et en outre :
A est pour chaque occurrence, de manière identique ou différente, CR¹ ou N, étant entendu qu'un, deux ou trois groupe(s) A représente(nt) N ;
Ar¹ est, de manière identique ou différente pour chaque occurrence, un système de cycle aromatique ou hétéroaromatique divalent qui comporte de 5 à 16 atomes de cycle aromatique, lequel peut être substitué par un radical ou par plusieurs radicaux R¹;
m est 0 ou 1.

9. Composé selon une ou plusieurs des revendications 1 à 8, **caractérisé en ce qu'**au moins un radical R est sélectionné parmi le groupe qui est constitué par les dérivés de triarylamine, les dérivés de carbazole, les dérivés d'indénocarbazole, les dérivés d'indolocarbazole, les dérivés, d'azacarbazole, les dérivés d'indole, les dérivés de furane, les dérivés de benzofurane, les dérivés de dibenzofurane, les dérivés de thiophène, les dérivés de benzothiophène ou les dérivés de dibenzothiophène, lesquels peuvent dans chaque cas être substitués par un radical ou par plusieurs radicaux R¹, ou au moins un substituant R représente
-NAr₂, où les deux groupes Ar peuvent également être pontés l'un à l'autre par un groupe qui est sélectionné parmi NR¹, O, S, C(R¹)₂, Si(R¹)₂ ou BR¹, en particulier qui est sélectionné parmi les groupes des formules (19) à (33), 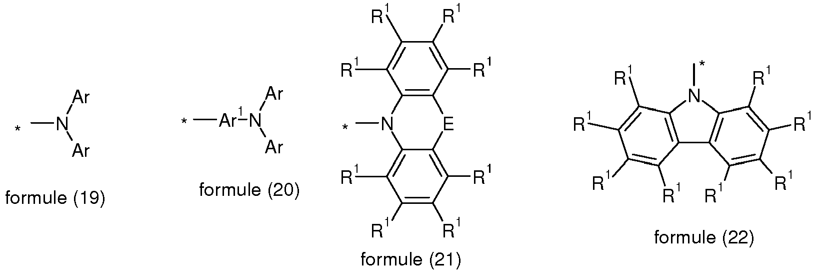 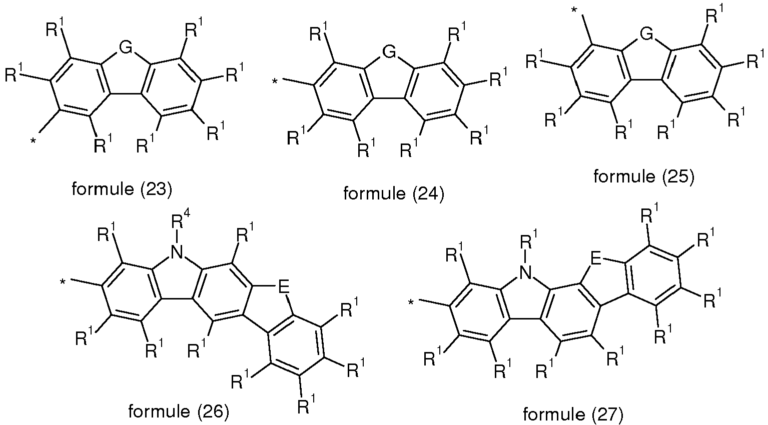 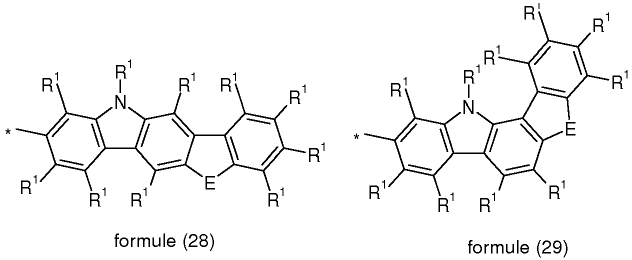 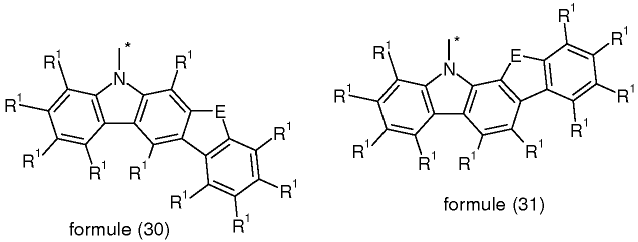 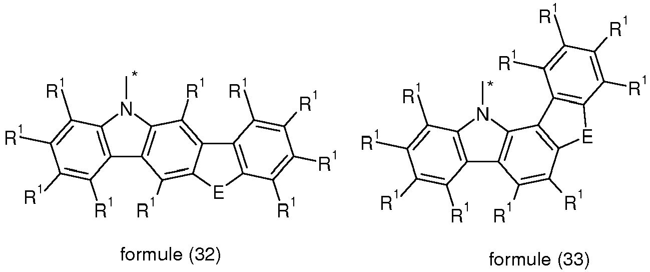 dans lesquelles les symboles qui sont utilisés présentent les significations qui ont été données selon les revendications 1 et 5 et en outre :
G est sélectionné parmi le groupe qui est constitué par NR¹, O ou S,
où dans chaque cas, un ou deux groupe(s) CR¹ peut/peuvent être remplacé(s) par N.

10. Procédé pour la préparation d'un composé selon une ou plusieurs des revendications 1 à 9, comprenant les étapes de réaction qui suivent :
a) l'oxydation d'un dérivé de fluoro-méta-xylène de manière à obtenir de l'acide carboxylique ; et
b) la conversion des fonctionnalités de l'acide carboxylique selon des groupes nitrile.

11. Procédé pour la préparation d'un composé selon une ou plusieurs des revendications 1 à 9 par réaction d'un dérivé de fluoro-méta-dihalo-benzène avec du Zn(CN)₂ et du Zn en présence d'un catalyseur.

12. Utilisation d'un composé selon une ou plusieurs des revendications 1 à 9 en tant que matériau de départ dans une réaction de substitution aromatique nucléophile aromatique.

13. Procédé pour la préparation d'un composé de la formule (34) ou de la formule (35) ou de la formule (36), 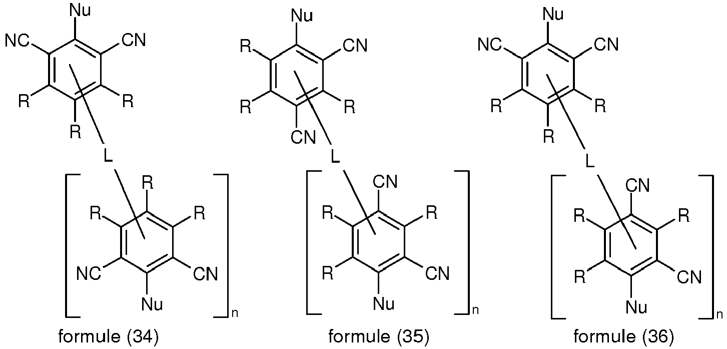 dans lesquelles Nu représente un groupe nucléophile et les autres symboles qui sont utilisés présentent les significations qui ont été données selon la revendication 1,
par réaction d'un composé selon une ou plusieurs des revendications 1 à 9 avec un nucléophile.
